(19) Europäisches Patentamt
European Patent Office
Office européen des brevets

(11) EP 4 234 564 A1

(12) **EUROPEAN PATENT APPLICATION**
published in accordance with Art. 153(4) EPC

(43) Date of publication:
**30.08.2023 Bulletin 2023/35**

(21) Application number: **21883317.6**

(22) Date of filing: **22.10.2021**

(51) International Patent Classification (IPC):
***C07F 15/06*** *(2006.01)* ***C09K 3/00*** *(2006.01)*

(52) Cooperative Patent Classification (CPC):
**C07F 11/005; C07F 15/06; C07F 15/065; C09K 3/00**

(86) International application number:
**PCT/KR2021/014871**

(87) International publication number:
**WO 2022/086250 (28.04.2022 Gazette 2022/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.10.2020 KR 20200137608**

(71) Applicant: **Lg Chem, Ltd.**
**Seoul 07336 (KR)**

(72) Inventors:
- **SON, Seonkyoung**
  **Daejeon 34122 (KR)**
- **LE, Duy Hieu**
  **Daejeon 34122 (KR)**
- **AN, Junhyun**
  **Daejeon 34122 (KR)**
- **LEE, Hoyong**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **COORDINATION COMPOUND AND LIGHT ABSORBER COMPRISING SAME**

(57) The present disclosure relates to a coordination compound, and a light absorber including the same.

【FIG. 1】

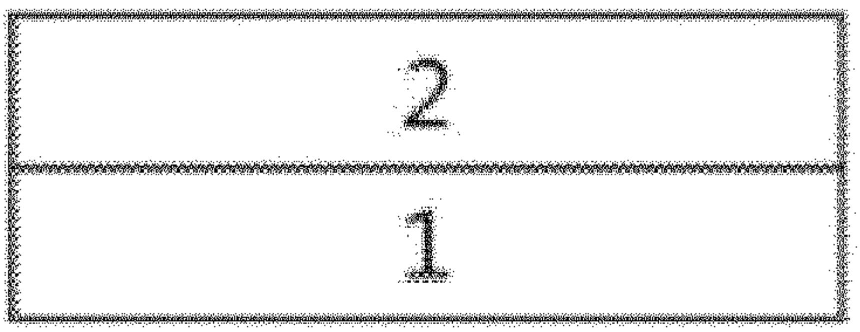


EP 4 234 564 A1

## Description

[Technical Field]

The present disclosure claims priority to and the benefits of Korean Patent Application No. 10-2020-0137608, filed with the Korean Intellectual Property Office on October 22, 2020, the entire contents of which are incorporated herein by reference.

The present disclosure relates to a coordination compound, and a light absorber including the same.

[Background Art]

A display apparatus included in an electronic device normally expresses an image using a combination of light having specific wavelengths of R (red), G (green) and B (blue). At this time, in the case of indoors, visibility of the display apparatus is favorable since there is no external light having high energy.

However, in the case of outdoors, strong light reflection occurs on the apparatus surface, an electrode in the apparatus or the like due to external light having high energy such as sunlight, and the amount of light emitting in the display decreases, which causes problems of significantly reducing contrast ratio and visibility of the display.

In order to resolve the problems of reducing contrast ratio and visibility of the display caused by the external light reflection, attempts have been made to reduce reflected light by designing to include a region absorbing external light inside the display. For example, an organic light emitting device (OLED) includes a polarizing plate in the device in order to reduce reflected light caused by the external light. The polarizing plate included in the electronic device improves surface reflection, electrode reflection and image emission brightness by controlling the amount of absorbed external light.

However, using a polarizing plate in an electronic device to control the amount of external light has problems of not flexibly adjusting color tones of the emitted color, increasing material costs, and not flexibly designing a structure of the device.

(Patent Document 1) Publication of Japanese Patent Application Laid-Open No. 2012-211305

[Disclosure]

[Technical Problem]

The present disclosure provides a novel coordination compound.

The present disclosure provides a light absorber including the coordination compound.

The present disclosure provides an adhesive film including the light absorber.

The present disclosure provides an optical film including the light absorber.

The present disclosure provides an electronic device including the light absorber.

[Technical Solution]

One embodiment of the present disclosure provides a coordination compound, wherein, in a compound represented by $A^{n-}$, A is represented by the following Chemical Formula A, and n is 1 or 2.

[Chemical Formula A]

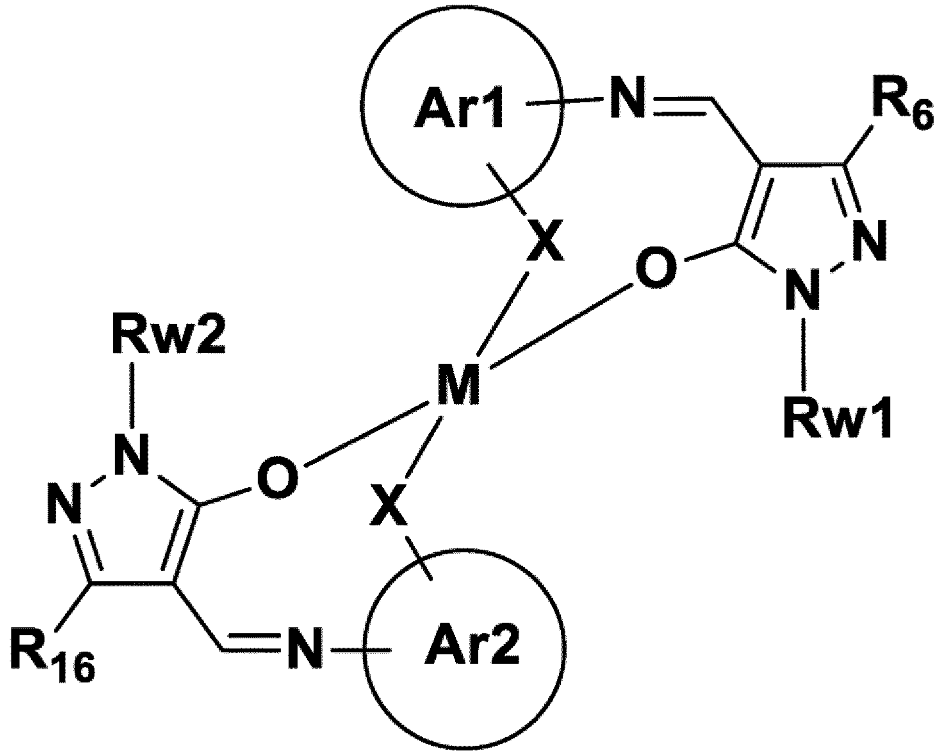

In Chemical Formula A,

M is a Cr ion or a Co ion,
X is O or O-C=O,
Rw1 and Rw2 are the same as or different from each other, and each independently a substituted or unsubstituted alkyl group; a substituted or unsubstituted alkenyl group; a substituted or unsubstituted cycloalkyl group; a substituted or unsubstituted aryl group; or a substituted or unsubstituted heteroaryl group,
Ar1 and Ar2 are the same as or different from each other, and each independently a substituted or unsubstituted arylene group; or a substituted or unsubstituted heteroarylene group,
$R_6$ and $R_{16}$ are the same as or different from each other, and each independently hydrogen; deuterium; a nitrile group; a nitro group; a hydroxyl group; -COOH; a halogen group; an imide group; a substituted or unsubstituted alkyl group; a substituted or unsubstituted cycloalkyl group; a substituted or unsubstituted heterocyclic group; a substituted or unsubstituted aryl group; a substituted or unsubstituted heteroaryl group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted arylphosphine group; a substituted or unsubstituted phosphine oxide group; a substituted or unsubstituted styryl group; $-OR_{100}$; $-CO_2R_{101}$; $-COR_{102}$; $-OCOR_{103}$; - $CONR_{104}R_{105}$; or $-SO_2R_{106}$, and
$R_{100}$ to $R_{106}$ are the same as or different from each other, and each independently hydrogen; a substituted or unsubstituted alkyl group; a substituted or unsubstituted cycloalkyl group; a substituted or unsubstituted aryl group; a substituted or unsubstituted heteroaryl group; or a substituted or unsubstituted amine group.

Another embodiment of the present disclosure provides a light absorber including the coordination compound.
Still another embodiment of the present disclosure provides an adhesive film including the light absorber.
Still another embodiment of the present disclosure provides an optical film including the light absorber.
Still another embodiment of the present disclosure provides an electronic device including the light absorber.

[Advantageous Effects]

A coordination compound according to one embodiment of the present disclosure is used as a light absorber.
A coordination compound according to one embodiment of the present disclosure or a light absorber including the same is used in a film to enhance blue luminance transmittance.

A coordination compound according to one embodiment of the present disclosure or a light absorber including the same improves reflected color.

[Description of Drawings]

FIG. 1 illustrates an adhesive film according to one embodiment of the present disclosure.
FIG. 2 illustrates an optical film according to one embodiment of the present disclosure.

[Reference Numeral]

1: Base
2: Adhesive Film
3: Binder Resin Film
10: Optical Film

[Mode for Disclosure]

Hereinafter, the present disclosure will be described in more detail.

In the present disclosure, a description of a certain part "including" certain constituents means capable of further including other constituents, and does not exclude other constituents unless particularly stated on the contrary.

In the present disclosure, a description of a certain member (layer) being placed "on" another member (layer) includes not only a case of the certain member (layer) being in contact with the another member but a case of still another member (layer) being present between the two members (layers).

In the present disclosure, the "layer" has a meaning compatible with a 'film' mainly used in the art, and means coating that covers a target area. The size of the "layer" is not limited, and each "layer" may have the same or a different size. According to one embodiment, the size of the "layer" may be the same as the whole device, may correspond to the size of a specific functional area, or may be as small as a single subpixel.

Unless defined otherwise in the present disclosure, all technological and scientific terms used in the present disclosure have the same meanings as terms commonly understood by those skilled in the art. Although methods and materials similar or equivalent to those described in the present disclosure may be used in implementing or experimenting embodiments of the present disclosure, suitable methods and materials are described later. All publications, patent applications, patents and other reference documents mentioned in the present disclosure are incorporated by reference in the present disclosure as a whole, and when conflicting, the present disclosure including definitions has priority unless specific passage is mentioned. Furthermore, materials, methods and examples are for illustrative purposes only, and not to limit the present disclosure.

One embodiment of the present disclosure provides a coordination compound, wherein, in a compound represented by $A^{n-}$, A is represented by the following Chemical Formula A, and n is 1 or 2.

[Chemical Formula A]

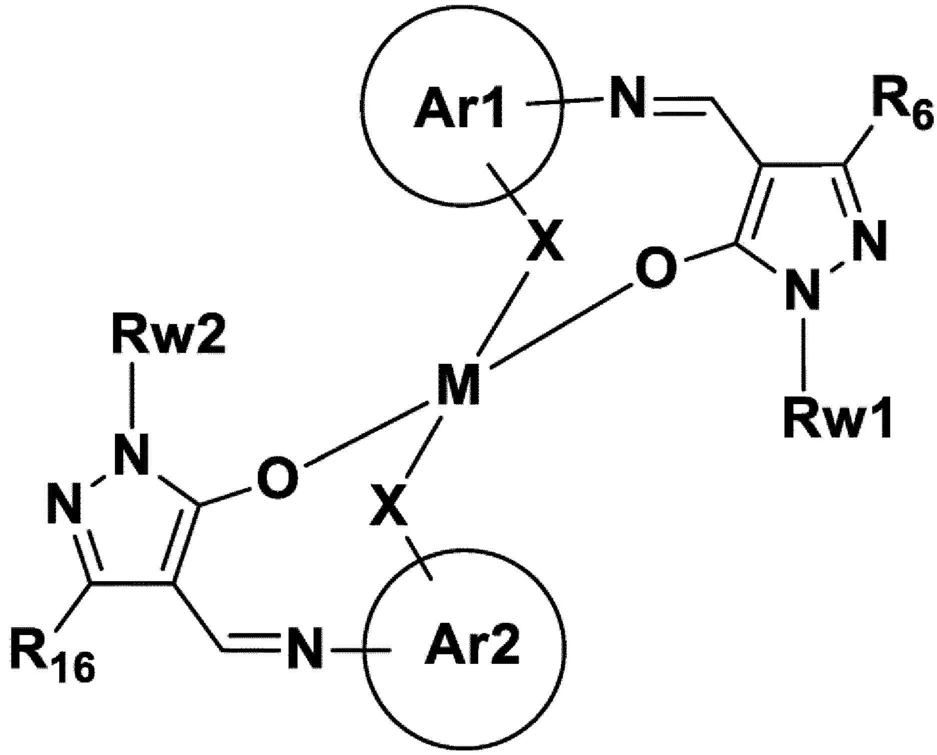

In Chemical Formula A,

M is a Cr ion or a Co ion,
X is O or O-C=O,
Rw1 and Rw2 are the same as or different from each other, and each independently a substituted or unsubstituted alkyl group; a substituted or unsubstituted alkenyl group; a substituted or unsubstituted cycloalkyl group; a substituted or unsubstituted aryl group; or a substituted or unsubstituted heteroaryl group,
Ar1 and Ar2 are the same as or different from each other, and each independently a substituted or unsubstituted arylene group; or a substituted or unsubstituted heteroarylene group,
$R_6$ and $R_{16}$ are the same as or different from each other, and each independently hydrogen; deuterium; a nitrile group; a nitro group; a hydroxyl group; -COOH; a halogen group; an imide group; a substituted or unsubstituted alkyl group; a substituted or unsubstituted cycloalkyl group; a substituted or unsubstituted heterocyclic group; a substituted or unsubstituted aryl group; a substituted or unsubstituted heteroaryl group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted arylphosphine group; a substituted or unsubstituted phosphine oxide group; a substituted or unsubstituted styryl group; $-OR_{100}$; $-CO_2R_{101}$; $-COR_{102}$; $-OCOR_{103}$; - $CONR_{104}R_{105}$; or $-SO_2R_{106}$, and
$R_{100}$ to $R_{106}$ are the same as or different from each other, and each independently hydrogen; a substituted or unsubstituted alkyl group; a substituted or unsubstituted cycloalkyl group; a substituted or unsubstituted aryl group; a substituted or unsubstituted heteroaryl group; or a substituted or unsubstituted amine group.

An organic light emitting device (OLED) panel generally has high reflectivity due to its characteristic that a reflective electrode is included as an essential constitution. One way to reduce reflectivity of an OLED panel is using a light absorber absorbing visible light in an optical film. The coordination compound according to the present disclosure absorbs visible light and is used as a light absorber. In addition, a light absorber including the coordination compound according to the present disclosure is used in a film to achieve an advantage of enhancing blue luminance transmittance or improving reflected color.

Specifically, when using the coordination compound described above or the light absorber described above according to one embodiment of the present disclosure in an OLED, external light reflection may be efficiently suppressed without using a circular polarizing plate. Not using a circular polarizing plate in an OLED has advantages of saving material costs and properly maintaining flexibility of the OLED.

In addition, by the structure represented by Chemical Formula A including an imine bond (C=N) instead of an azo bond (N=N), a maximum absorption wavelength that is present in a region of 450 nm or greater may be shortened, and adjusted to have a target maximum absorption wavelength between 380 nm and 450 nm.

When M of the structure represented by Chemical Formula A bonds to N and O instead of bonding to O or O-C=O of X, and particularly when M is a Co ion, a maximum absorption wavelength is expressed in a short wavelength region of 320 nm to 340 nm, and when M is a Cu ion, a maximum absorption wavelength is expressed between 500 nm to 560 nm with a very wide shape, and an actual effect as a blue-cut dye is insignificant.

In the present disclosure, a "complex" is also referred to as a coordination compound or a complex compound, and means a compound formed through a coordination bond of ligand lone pair electrons to a central metal ion having many empty orbitals.

Examples of substituents in the present disclosure are described below, however, the substituents are not limited thereto.

In the present disclosure, the term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present disclosure, the term "substituted or unsubstituted" means being substituted with one, two or more substituents selected from the group consisting of deuterium; a halogen group; a nitrile group (-CN); a nitro group ($-NO_2$); a hydroxyl group; -COOH; an imide group; an amide group; an alkylthioxy group; an arylthioxy group; an alkylsulfoxy group; an arylsulfoxy group; an alkenyl group; a silyl group; a boron group; an arylphosphine group; a phosphine oxide group; an alkoxy group; an aryloxy group; $-CO_2R$; -COR; -OCOR; $-SO_2R$; an alkyl group; a cycloalkyl group; an aryl group; a heterocyclic group; an amine group; a styryl group; and a heteroaryl group, or being substituted with a substituent linking two or more substituents among the substituents illustrated above, or having no substituents. Herein, R is hydrogen; an alkyl group; a cycloalkyl group; an aryl group; or a heteroaryl group.

Examples of the substituents are described below, however, the substituents are not limited thereto.

In the present disclosure, "deuterium" refers to a stable isotope of hydrogen having a mass approximately twice that of a most common isotope, that is, a mass of approximately 2 atomic mass units.

In the present disclosure, examples of the halogen group may include fluorine, chlorine, bromine or iodine.

In the present disclosure, the alkyl group may be linear or branched, and although not particularly limited thereto, the number of carbon atoms is preferably from 1 to 30; 1 to 20; 1 to 10; or 1 to 5. Specific examples thereof may include methyl, ethyl, propyl, n-propyl, isopropyl, butyl, n-butyl, isobutyl, t-butyl, sec-butyl, 1-methylbutyl, 1-ethylbutyl, pentyl, n-pentyl, isopentyl, neopentyl, t-pentyl, hexyl, n-hexyl, 1-methylpentyl, 2-methylpentyl, 3,3-dimethylbutyl, 2-ethylbutyl, heptyl, n-heptyl, 1-methylhexyl, cyclopentylmethyl, cyclohexylmethyl, octyl, n-octyl, t-octyl, 1-methylheptyl, 2-ethylhexyl, 2-propylpentyl, n-nonyl, 2,2-dimethylheptyl, 1-ethylpropyl, 1,1-dimethylpropyl, isohexyl, 4-methylhexyl, 5-methylhexyl and the like, but are not limited thereto. For reference, in the present disclosure, the propyl group includes an isopropyl group. In addition, in the present disclosure, the butyl group includes a tert-butyl group.

In the present disclosure, the descriptions on the alkyl group provided above are applied to the alkylene group except that the alkylene group is a divalent group.

In the present disclosure, the cycloalkyl group is not particularly limited, and although not particularly limited thereto, the number of carbon atoms is preferably from 3 to 60; 3 to 30; or 3 to 20. Specific examples of the cycloalkyl group may include a cyclopropyl group, a cyclobutyl group, a cyclopentyl group, a cyclohexyl group, a cycloheptyl group, a cyclooctyl group and the like, but are not limited thereto.

In the present disclosure, the descriptions on the cycloalkyl group provided above are applied to the cycloalkylene group except that the cycloalkylene group is a divalent group.

In the present disclosure, the aryl group means a monovalent aromatic hydrocarbon or a monovalent group of an aromatic hydrocarbon derivative. In the present disclosure, the aromatic hydrocarbon means a compound including a planar ring in which pi electrons are fully conjugated, and the group derived from aromatic hydrocarbon means a structure in which aromatic hydrocarbon or cyclic aliphatic hydrocarbon is fused to aromatic hydrocarbon. In addition, in the present disclosure, the aryl group includes a monovalent group in which two or more aromatic hydrocarbons or aromatic hydrocarbon derivatives are linked to each other. The aryl group is not particularly limited, but preferably has 6 to 60; 6 to 50; 6 to 30; 6 to 25; 6 to 20; 6 to 18; 6 to 15; 6 to 13; or 6 to 12 carbon atoms, and may be a monocyclic aryl group or a polycyclic aryl group.

The monocyclic aryl group is not particularly limited, but preferably has 6 to 60; 6 to 54; 6 to 48; 6 to 42; 6 to 36; 6 to 30; 6 to 24; 6 to 18; or 6 to 12 carbon atoms. Specific examples thereof may include a phenyl group, a biphenyl group, a terphenyl group and the like, but are not limited thereto.

The polycyclic aryl group is not particularly limited, but preferably has 6 to 60 carbon atoms; 6 to 45 carbon atoms; 6 to 30 carbon atoms; 6 to 25; 6 to 22; 6 to 20; 6 to 18; 6 to 16; 6 to 15; 6 to 14; 6 to 13; 6 to 12; or 6 to 10 carbon atoms. Examples thereof may include a naphthyl group, an anthracenyl group, a phenanthrenyl group, a pyrenyl group,

a perylenyl group, a triphenylenyl group, a chrysenyl group, a fluorenyl group and the like, but are not limited thereto.

In the present disclosure, the heteroaryl group means a monovalent aromatic heteroring. Herein, the aromatic heteroring means a monovalent group of an aromatic ring or aromatic ring derivative, and means a group including one or more selected from the group consisting of N, O, P, S, Si and Se as a heteroatom. The aromatic ring derivative includes all structures in which an aromatic ring or aliphatic ring is fused to an aromatic ring. In addition, in the present disclosure, the heteroaryl group includes a monovalent group in which two or more aromatic rings including a heteroatom or aromatic ring derivatives including a heteroatom are linked to each other. The heteroaryl group preferably has 2 to 60; 2 to 50; 2 to 30; 2 to 20; 2 to 18; or 2 to 13 carbon atoms. Examples of the heteroaryl group may include a thiophene group, a furanyl group, a pyrrole group, an imidazole group, a thiazole group, an oxazole group, a pyridine group, a pyrimidine group, a triazine group, a triazole group, an acridine group, a pyridazine group, a pyrazine group, a quinoline group, a quinazoline group, a quinoxaline group, an isoquinoline group, an indole group, a carbazole group, a benzoxazole group, a benzimidazole group, a benzothiazole group, a benzocarbazole group, a benzothiophene group, a dibenzothiophene group, a benzofuran group, a phenanthrolinyl group, a dibenzofuran group and the like, but are not limited thereto.

In the present disclosure, the heteroaryl group may be monocyclic or polycyclic, and may be aromatic, aliphatic or a fused ring of aromatic and aliphatic.

In the present disclosure, the heterocyclic group is a monovalent group of an aliphatic ring or an aliphatic ring derivative, and means a group including one or more selected form the group consisting of N, O, P, S, Si and Se as a heteroatom.

In the present disclosure, the aliphatic ring is a hydrocarbon ring that is not aromatic or an aliphatic heteroring, and as examples of the hydrocarbon ring, the examples of the cycloalkyl group described above may be included, and as examples of the aliphatic heteroring, morpholine may be included, however, the hydrocarbon ring and the aliphatic heteroring are not limited thereto.

In the present disclosure, the descriptions on the aryl group or the heteroaryl group may be applied to the aromatic ring.

In the present disclosure, the imide group means a structure of $-C(O)NR^xC(O)R^y$. Specifically, $R^x$ and $R^y$ may be each independently hydrogen, or the substituted or unsubstituted alkyl group, cycloalkyl group, alkenyl group, heterocyclic group, aryl group or heteroaryl group as defined in the present disclosure. Specifically, the number of carbon atoms of the imide group is not particularly limited, but is preferably from 1 to 25.

In the present disclosure, the amide group may be represented by -CONRoRp, and as Ro and Rp, the descriptions on the hydrogen; the alkyl group; the cycloalkyl group; the aryl group; or the heteroaryl group provided above are each independently applied.

In the present disclosure, the alkylthioxy group may be represented by -SRs1, and the descriptions on the alkyl group provided above may be applied to Rs1.

In the present disclosure, the arylthioxy group may be represented by -SRs2, and the descriptions on the aryl group provided above may be applied to Rs2.

In the present disclosure, the alkoxy group may be represented by -ORx, and the descriptions on the alkyl group provided above may be applied to Rx.

In the present disclosure, the aryloxy group may be represented by -ORy, and the descriptions on the aryl group provided above may be applied to Ry.

In the present disclosure, the alkylsulfoxy group may be represented by -SORs3, and the descriptions on the alkyl group provided above may be applied to Rs3.

In the present disclosure, the arylsulfoxy group may be represented by -SORs4, and the descriptions on the aryl group provided above may be applied to Rs4.

In the present disclosure, the alkenyl group refers to linear or branched-type unsaturated hydrocarbon including one or more double bonds. Specifically, the alkenyl group may be linear or branched, and although not particularly limited thereto, the number of carbon atoms is preferably from 2 to 40. According to one embodiment, the number of carbon atoms of the alkenyl group is from 2 to 20. According to another embodiment, the number of carbon atoms of the alkenyl group is from 2 to 10. According to another embodiment, the number of carbon atoms of the alkenyl group is from 2 to 6. Specific examples thereof may include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group and the like, but are not limited thereto.

In the present disclosure, the silyl group may be represented by $-Si(Ri)_3$, and as Ri, the descriptions on the alkyl group; the alkenyl group; or the aryl group provided above may be applied. Examples thereof may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present disclosure, the boron group may be represented by $-B(Rb)_3$, and as Rb, the descriptions on the alkyl group or the aryl group provided above may be applied. Specific examples thereof may include a trimethylboron

group, a triethylboron group, a t-butyldimethylboron group, a triphenylboron group, a phenylboron group and the like, but are not limited thereto.

In the present disclosure, the arylphosphine group means a structure of $-PR^aR^bR^c$. $R^a$, $R^b$ and $R^c$ may be each independently hydrogen, or the alkyl group, the cycloalkyl group, the alkenyl group, the heterocyclic group, the aryl group or the heteroaryl group as defined in the present disclosure.

In the present disclosure, the phosphine oxide group means a structure of $-P(=0)\ R^aR^bR^c$. Specifically, $R^a$, $R^b$ and $R^c$ may be each independently hydrogen, or the alkyl group, the cycloalkyl group, the alkenyl group, the heterocyclic group, the aryl group or the heteroaryl group as defined in the present disclosure.

In the present disclosure, the amine group means a structure of $-N(Rn)_2$. Rn may be hydrogen; an alkyl group; a cycloalkyl group; an aryl group; or a heteroaryl group, and as the alkyl group, the cycloalkyl group, the aryl group and the heteroaryl group, the descriptions provided above in the present disclosure may be applied.

In the present disclosure, the styryl group means that any one or more hydrogens of styrene ($CHCH=CH_2$) function as a bonding position.

In the present disclosure, an "adjacent group" may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

In the present disclosure, "adjacent substituents bonding to each other to form an aromatic ring" may mean adjacent substituents bonding to each other to form the aryl group or the heteroaryl group described above.

In the present disclosure, "adjacent substituents bonding to each other to form an aliphatic ring" may mean 2 adjacent substituents bonding to each other to form the cycloalkyl group or the heterocyclic group described above.

In one embodiment of the present disclosure, Rw1 and Rw2 are the same as or different from each other, and each independently a substituted or unsubstituted alkyl group having 1 to 30 carbon atoms; a substituted or unsubstituted alkenyl group having 2 to 30 carbon atoms; a substituted or unsubstituted cycloalkyl group having 3 to 30 carbon atoms; a substituted or unsubstituted aryl group having 6 to 30 carbon atoms; or a substituted or unsubstituted heteroaryl group having 3 to 30 carbon atoms.

In one embodiment of the present disclosure, Rw1 and Rw2 are the same as or different from each other, and each independently a substituted or unsubstituted alkyl group having 1 to 20 carbon atoms; a substituted or unsubstituted alkenyl group having 2 to 20 carbon atoms; a substituted or unsubstituted cycloalkyl group having 3 to 20 carbon atoms; a substituted or unsubstituted aryl group having 6 to 20 carbon atoms; or a substituted or unsubstituted heteroaryl group having 3 to 20 carbon atoms.

In one embodiment of the present disclosure, Rw1 and Rw2 are the same as or different from each other, and each independently a substituted or unsubstituted alkyl group having 1 to 10 carbon atoms; a substituted or unsubstituted alkenyl group having 2 to 10 carbon atoms; a substituted or unsubstituted cycloalkyl group having 3 to 10 carbon atoms; a substituted or unsubstituted aryl group having 6 to 10 carbon atoms; or a substituted or unsubstituted heteroaryl group having 3 to 10 carbon atoms.

In one embodiment of the present disclosure, Rw1 and Rw2 are the same as or different from each other, and each independently a substituted or unsubstituted phenyl group.

In one embodiment of the present disclosure, Rw1 and Rw2 are the same as or different from each other, and each independently a phenyl group unsubstituted or substituted with a nitro group, a nitrile group, a sulfonic acid group, -COOH, chlorine, fluorine, a methyl group, a trifluoromethyl group, an ethyl group, an isopropyl group, a tert-butyl group, a methoxy group, an ethoxy group, $-OCF_3$, $-COOCH_2CH_3$, $-COOCH_3$, a cyclohexyl group, a phenyl group, a phenyl group substituted with an n-propyl group, a pyridine group or a thiazole group.

In one embodiment of the present disclosure, Ar1 and Ar2 are the same as or different from each other, and each independently a substituted or unsubstituted arylene group having 6 to 30 carbon atoms; or a substituted or unsubstituted heteroarylene group having 2 to 30 carbon atoms.

In one embodiment of the present disclosure, Ar1 and Ar2 are the same as or different from each other, and each independently a substituted or unsubstituted arylene group having 6 to 20 carbon atoms; or a substituted or unsubstituted heteroarylene group having 2 to 20 carbon atoms.

In one embodiment of the present disclosure, Ar1 and Ar2 are the same as or different from each other, and each independently a substituted or unsubstituted arylene group having 6 to 10 carbon atoms; or a substituted or unsubstituted heteroarylene group having 2 to 10 carbon atoms.

In one embodiment of the present disclosure, Ar1 and Ar2 are the same as or different from each other, and each independently a substituted or unsubstituted phenylene group; or a substituted or unsubstituted divalent pyridine group.

In one embodiment of the present disclosure, Ar1 and Ar2 are the same as or different from each other, and each independently a phenylene group substituted with fluorine, chlorine, bromine, a nitro group, a nitrile group, a sulfonic

acid group, a methyl group, a trifluoromethyl group, a tert-butyl group, a methoxy group, a cyclohexyl group, a phenyl group, a phenyl group substituted with a methyl group, a phenyl group substituted with a methoxy group, a thiophene group, a pyridine group, $-COCH_3$, $-COOCH_3$ or $-SO_2CH_3$, a phenylene group having adjacent substituents bonding to each other to form a benzene ring; an unsubstituted phenylene group; or an unsubstituted divalent pyridine group.

In one embodiment of the present disclosure, M is $Co^{2+}$; $Co^{3+}$; $Cr^{2+}$; or $Cr^{3+}$.

In one embodiment of the present disclosure, M is $Co^{2+}$.

In one embodiment of the present disclosure, M is $Co^{3+}$.

In one embodiment of the present disclosure, M is $Cr^{2+}$.

In one embodiment of the present disclosure, M is $Cr^{3+}$.

In one embodiment of the present disclosure, X is O.

In one embodiment of the present disclosure, X is O-C=O.

In one embodiment of the present disclosure, when X is O-C=O, bonding occurs in the order of M-O-(C=O)-Ar1 or M-O-(C=O)-Ar2 based on M in Chemical Formula A.

According to one embodiment of the present disclosure, Chemical Formula A is represented by the following Chemical Formula A-1.

[Chemical Formula A-1]

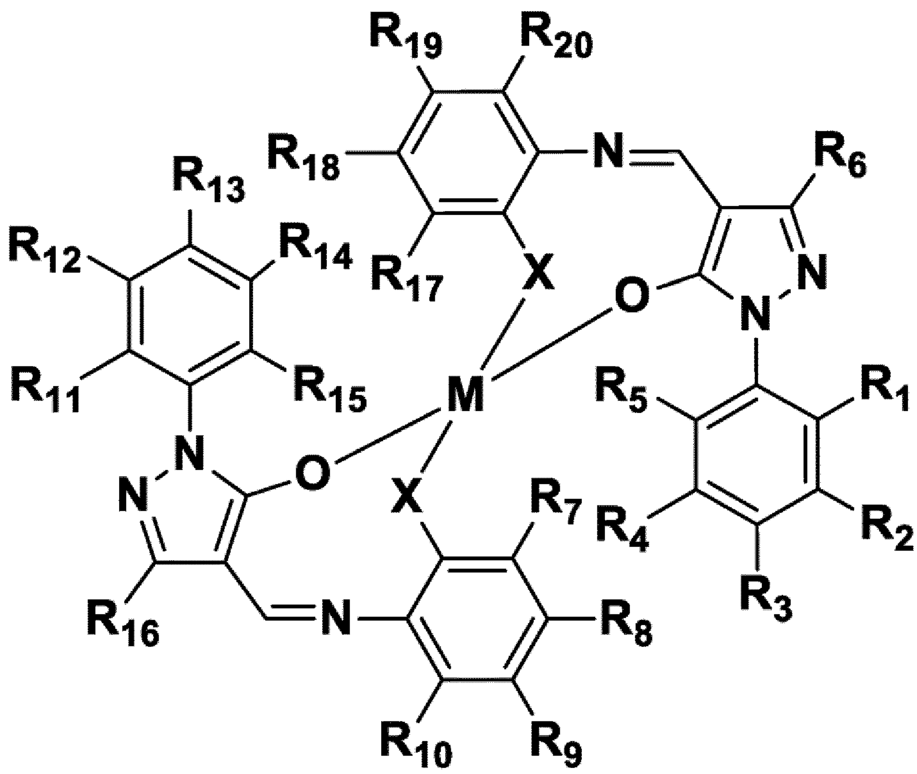

In Chemical Formula A-1,

M, X, $R_6$ and $R_{16}$ have the same definitions as in Chemical Formula A,

$R_1$ to $R_5$ $R_7$ to $R_{15}$ and $R_{17}$ to $R_{20}$ are the same as or different from each other, and each independently hydrogen; deuterium; a nitrile group; a nitro group; a hydroxyl group; - COOH; a halogen group; an imide group; a substituted or unsubstituted alkyl group; a substituted or unsubstituted cycloalkyl group; a substituted or unsubstituted heterocyclic group; a substituted or unsubstituted aryl group; a substituted or unsubstituted heteroaryl group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted arylphosphine group; a substituted or unsubstituted phosphine oxide group; $-OR_{100}$; - $CO_2R_{101}$; $—COR_{102}$; $-OCOR_{103}$; $-CONR_{104}R_{105}$; or $-SO_2R_{106}$, or adjacent substituents bond to each other to form a substituted or unsubstituted aromatic ring or a substituted or unsubstituted aliphatic ring, and

$R_{100}$ to $R_{106}$ are the same as or different from each other, and each independently hydrogen; a substituted or unsubstituted alkyl group; a substituted or unsubstituted cycloalkyl group; a substituted or unsubstituted aryl group; a substituted or unsubstituted heteroaryl group; or a substituted or unsubstituted amine group.

In one embodiment of the present disclosure, $R_1$ to $R_{20}$ are the same as or different from each other, and each independently hydrogen; deuterium; a nitrile group; a nitro group; a hydroxyl group; -COOH; a halogen group; an imide group; a substituted or unsubstituted alkyl group having 1 to 30 carbon atoms; a substituted or unsubstituted cycloalkyl group having 3 to 30 carbon atoms; a substituted or unsubstituted heterocyclic group having 2 to 30 carbon atoms; a substituted or unsubstituted aryl group having 6 to 30 carbon atoms; a substituted or unsubstituted heteroaryl group having 2 to 30 carbon atoms; a substituted or unsubstituted alkylthioxy group having 1 to 30 carbon atoms; a substituted or unsubstituted arylthioxy group having 6 to 30 carbon atoms; a substituted or unsubstituted alkylsulfoxy group having 1 to 30 carbon atoms; a substituted or unsubstituted arylsulfoxy group having 6 to 30 carbon atoms; a substituted or unsubstituted alkenyl group having 2 to 30 carbon atoms; a substituted or unsubstituted silyl group having 1 to 30 carbon atoms; a substituted or unsubstituted boron group having 1 to 30 carbon atoms; a substituted or unsubstituted arylphosphine group having 6 to 30 carbon atoms; a substituted or unsubstituted phosphine oxide group having 1 to 30 carbon atoms; $-OR_{100}$; $- CO_2R_{101}$; $—COR_{102}$; $-OCOR_{103}$; $-CONR_{104}R_{105}$; or $-SO_2R_{106}$, or adjacent substituents bond to each other to form a substituted or unsubstituted aromatic ring having 6 to 30 carbon atoms or a substituted or unsubstituted aliphatic ring having 2 to 30 carbon atoms, and $R_{100}$ to $R_{106}$ are the same as or different from each other and each independently hydrogen; a substituted or unsubstituted alkyl group having 1 to 30 carbon atoms; a substituted or unsubstituted cycloalkyl group having 3 to 30 carbon atoms; a substituted or unsubstituted aryl group having 6 to 30 carbon atoms; a substituted or unsubstituted heteroaryl group having 2 to 30 carbon atoms; or a substituted or unsubstituted amine group.

In one embodiment of the present disclosure, $R_1$ to $R_{20}$ are the same as or different from each other, and each independently hydrogen; deuterium; a nitrile group; a nitro group; a hydroxyl group; -COOH; a halogen group; an imide group; a substituted or unsubstituted alkyl group having 1 to 20 carbon atoms; a substituted or unsubstituted cycloalkyl group having 3 to 20 carbon atoms; a substituted or unsubstituted heterocyclic group having 2 to 20 carbon atoms; a substituted or unsubstituted aryl group having 6 to 20 carbon atoms; a substituted or unsubstituted heteroaryl group having 2 to 20 carbon atoms; a substituted or unsubstituted alkylthioxy group having 1 to 20 carbon atoms; a substituted or unsubstituted arylthioxy group having 6 to 20 carbon atoms; a substituted or unsubstituted alkylsulfoxy group having 1 to 20 carbon atoms; a substituted or unsubstituted arylsulfoxy group having 6 to 20 carbon atoms; a substituted or unsubstituted alkenyl group having 2 to 20 carbon atoms; a substituted or unsubstituted silyl group having 1 to 20 carbon atoms; a substituted or unsubstituted boron group having 1 to 20 carbon atoms; a substituted or unsubstituted arylphosphine group having 6 to 20 carbon atoms; a substituted or unsubstituted phosphine oxide group having 1 to 20 carbon atoms; $-OR_{100}$; $- CO_2R_{101}$; $—COR_{102}$; $-OCOR_{103}$; $-CONR_{104}R_{105}$; or $-SO_2R_{106}$, or adjacent substituents bond to each other to form a substituted or unsubstituted aromatic ring having 6 to 20 carbon atoms or a substituted or unsubstituted aliphatic ring having 2 to 20 carbon atoms, and $R_{100}$ to $R_{106}$ are the same as or different from each other and each independently hydrogen; a substituted or unsubstituted alkyl group having 1 to 20 carbon atoms; a substituted or unsubstituted cycloalkyl group having 3 to 20 carbon atoms; a substituted or unsubstituted aryl group having 6 to 20 carbon atoms; a substituted or unsubstituted heteroaryl group having 2 to 20 carbon atoms; or a substituted or unsubstituted amine group.

In one embodiment of the present disclosure, $R_1$ to $R_{20}$ are the same as or different from each other, and each independently hydrogen; deuterium; a nitrile group; a nitro group; a hydroxyl group; -COOH; a halogen group; an imide group; a substituted or unsubstituted alkyl group having 1 to 10 carbon atoms; a substituted or unsubstituted cycloalkyl group having 3 to 10 carbon atoms; a substituted or unsubstituted heterocyclic group having 2 to 10 carbon atoms; a substituted or unsubstituted aryl group having 6 to 10 carbon atoms; a substituted or unsubstituted heteroaryl group having 2 to 10 carbon atoms; a substituted or unsubstituted alkylthioxy group having 1 to 10 carbon atoms; a substituted or unsubstituted arylthioxy group having 6 to 10 carbon atoms; a substituted or unsubstituted alkylsulfoxy group having 1 to 10 carbon atoms; a substituted or unsubstituted arylsulfoxy group having 6 to 10 carbon atoms; a substituted or unsubstituted alkenyl group having 2 to 10 carbon atoms; a substituted or unsubstituted silyl group having 1 to 10 carbon atoms; a substituted or unsubstituted boron group having 1 to 10 carbon atoms; a substituted or unsubstituted arylphosphine group having 6 to 10 carbon atoms; a substituted or unsubstituted phosphine oxide group having 1 to 10 carbon atoms; $-OR_{100}$; $- CO_2R_{101}$; $—COR_{102}$; $-OCOR_{103}$; $-CONR_{104}R_{105}$; or $-SO_2R_{106}$, or adjacent substituents bond to each other to form a substituted or unsubstituted aromatic ring having 6 to 10 carbon atoms or a substituted or unsubstituted aliphatic ring having 2 to 10 carbon atoms, and $R_{100}$ to $R_{106}$ are the same as or different from each other and each independently hydrogen; a substituted or unsubstituted alkyl group having 1 to 10 carbon atoms; a substituted or unsubstituted cycloalkyl group having 3 to 10 carbon atoms; a substituted or unsubstituted aryl group having 6 to 10 carbon atoms; a substituted or unsubstituted heteroaryl group having 2 to 10 carbon atoms; or a substituted or unsubstituted amine group.

In one embodiment of the present disclosure, $R_1$ to $R_{20}$ are the same as or different from each other, and each independently hydrogen; deuterium; a nitrile group; a nitro group; a hydroxyl group; -COOH; a halogen group; a substituted or unsubstituted alkyl group; a substituted or unsubstituted cycloalkyl group; a substituted or unsubstituted alkenyl group; a substituted or unsubstituted aryl group; a substituted or unsubstituted heteroaryl group; $-OR_{100}$; $-CO_2R_{101}$; $-COR_{102}$; or - $SO_2R_{106}$, or adjacent substituents bond to each other to form a substituted or unsubstituted aromatic ring.

In one embodiment of the present disclosure, $R_1$ to $R_{20}$ are the same as or different from each other, and each independently hydrogen; deuterium; a nitrile group; a nitro group; a hydroxyl group; -COOH; a halogen group; a substituted or unsubstituted alkyl group having 1 to 30 carbon atoms; a substituted or unsubstituted cycloalkyl group having 3 to 30 carbon atoms; a substituted or unsubstituted alkenyl group having 2 to 30 carbon atoms; a substituted or unsubstituted aryl group having 6 to 30 carbon atoms; a substituted or unsubstituted heteroaryl group having 2 to 30 carbon atoms; -$OR_{100}$; $-CO_2R_{101}$; $-COR_{102}$; or $-SO_2R_{106}$, or adjacent substituents bond to each other to form a substituted or unsubstituted aromatic ring having 6 to 30 carbon atoms.

In one embodiment of the present disclosure, $R_1$ to $R_{20}$ are the same as or different from each other, and each independently hydrogen; deuterium; a nitrile group; a nitro group; a hydroxyl group; -COOH; a halogen group; a substituted or unsubstituted alkyl group having 1 to 20 carbon atoms; a substituted or unsubstituted cycloalkyl group having 3 to 20 carbon atoms; a substituted or unsubstituted alkenyl group having 2 to 20 carbon atoms; a substituted or unsubstituted aryl group having 6 to 20 carbon atoms; a substituted or unsubstituted heteroaryl group having 2 to 20 carbon atoms; -$OR_{100}$; $-CO_2R_{101}$; $-COR_{102}$; or $-SO_2R_{106}$, or adjacent substituents bond to each other to form a substituted or unsubstituted aromatic ring having 6 to 20 carbon atoms.

In one embodiment of the present disclosure, $R_1$ to $R_{20}$ are the same as or different from each other, and each independently hydrogen; deuterium; a nitrile group; a nitro group; a hydroxyl group; -COOH; a halogen group; a substituted or unsubstituted alkyl group having 1 to 10 carbon atoms; a substituted or unsubstituted cycloalkyl group having 3 to 10 carbon atoms; a substituted or unsubstituted alkenyl group having 2 to 10 carbon atoms; a substituted or unsubstituted aryl group having 6 to 10 carbon atoms; a substituted or unsubstituted heteroaryl group having 2 to 10 carbon atoms; -$OR_{100}$; $-CO_2R_{101}$; $-COR_{102}$; or $-SO_2R_{106}$, or adjacent substituents bond to each other to form a substituted or unsubstituted aromatic ring having 6 to 10 carbon atoms.

In one embodiment of the present disclosure, $R_1$ to $R_{20}$ are the same as or different from each other, and each independently hydrogen; deuterium; a nitrile group; a nitro group; a hydroxyl group; -COOH; chlorine; fluorine; bromine; a substituted or unsubstituted methyl group; a substituted or unsubstituted ethyl group; a substituted or unsubstituted propyl group; a substituted or unsubstituted butyl group; a substituted or unsubstituted cyclohexyl group; a substituted or unsubstituted ethenyl group; a substituted or unsubstituted phenyl group; a substituted or unsubstituted biphenyl group; a substituted or unsubstituted benzoxazole group; a substituted or unsubstituted pyridine group; a substituted or unsubstituted thiophene group; a substituted or unsubstituted furan group; a substituted or unsubstituted thiazole group; $-OR_{100}$; $-CO_2R_{101}$; - $COR_{102}$; or $-SO_2R_{106}$, or adjacent substituents bond to each other to form a substituted or unsubstituted benzene ring.

In one embodiment of the present disclosure, $R_1$ to $R_{20}$ are the same as or different from each other, and each independently hydrogen; deuterium; a nitrile group; a nitro group; a hydroxyl group; -COOH; chlorine; fluorine; bromine; a methyl group; a trifluoromethyl group; an ethyl group; an isopropyl group; a tert-butyl group; a cyclohexyl group; an ethenyl group substituted with a phenyl group substituted with a nitro group, an isopropyl group or a tert-butyl group; a phenyl group substituted with a nitro group, a nitrile group, - COOH, fluorine, a methoxy group, $-OCF_3$, $-COOCH_2CH_3$, a methyl group, an n-propyl group, a cyclohexyl group or a pyridine group; a biphenyl group; a benzoxazole group; a pyridine group; a thiophene group; a furan group; a thiazole group; $-OR_{100}$; - $CO_2R_{101}$; $-COR_{102}$; or $-SO_2R_{106}$, or adjacent substituents bond to each other to form a benzene ring.

In one embodiment of the present disclosure, $R_1$ to $R_{20}$ are the same as or different from each other, and each independently hydrogen; chlorine; fluorine; a nitro group; a methyl group; a trifluoromethyl group; an isopropyl group; a tert-butyl group; a phenyl group; $-OR_{100}$; $-CO_2R_{101}$; or $-SO_2R_{106}$, or adjacent substituents bond to each other to form a benzene ring.

In one embodiment of the present disclosure, $R_8$ and $R_9$, $R_{19}$ and $R_{18}$, $R_9$ and $R_{10}$ or $R_{19}$ and $R_{20}$ each bond to each other to form a substituted or unsubstituted benzene ring.

In one embodiment of the present disclosure, $R_8$ and $R_9$, $R_{19}$ and $R_{18}$, $R_9$ and $R_{10}$ or $R_{19}$ and $R_{20}$ each bond to each other to form a benzene ring.

In one embodiment of the present disclosure, $R_{100}$ to $R_{106}$ are the same as or different from each other, and each independently a substituted or unsubstituted alkyl group; or a substituted or unsubstituted amine group.

In one embodiment of the present disclosure, $R_{100}$ to $R_{106}$ are the same as or different from each other, and each independently a substituted or unsubstituted alkyl group having 1 to 30 carbon atoms; or a substituted or unsubstituted amine group.

In one embodiment of the present disclosure, $R_{100}$ to $R_{106}$ are the same as or different from each other, and each independently a substituted or unsubstituted alkyl group having 1 to 20 carbon atoms; or a substituted or unsubstituted amine group.

In one embodiment of the present disclosure, $R_{100}$ to $R_{106}$ are the same as or different from each other, and each independently a substituted or unsubstituted alkyl group having 1 to 10 carbon atoms; or a substituted or unsubstituted amine group.

In one embodiment of the present disclosure, $R_{100}$ to $R_{106}$ are the same as or different from each other, and each independently a substituted or unsubstituted methyl group; a substituted or unsubstituted ethyl group; or $-NH_2$.

In one embodiment of the present disclosure, $R_{100}$ to $R_{106}$ are the same as or different from each other, and each independently a methyl group; a fluoromethyl group; an ethyl group; or $-NH_2$.

In one embodiment of the present disclosure, $R_{100}$ is a methyl group; a trifluoromethyl group; or an ethyl group.

In one embodiment of the present disclosure, $R_{101}$ is a methyl group; or an ethyl group.

In one embodiment of the present disclosure, $R_{102}$ is a methyl group.

In one embodiment of the present disclosure, $R_{106}$ is a methyl group; or $-NH_2$.

According to one embodiment of the present disclosure, $R_1$ to $R_5$ and $R_{11}$ to $R_{15}$ are the same as or different from each other, and each independently hydrogen; deuterium; a nitrile group; a nitro group; a hydroxyl group; -COOH; a halogen group; an imide group; a substituted or unsubstituted alkyl group; a substituted or unsubstituted cycloalkyl group; a substituted or unsubstituted heterocyclic group; a substituted or unsubstituted aryl group; a substituted or unsubstituted heteroaryl group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted arylphosphine group; a substituted or unsubstituted phosphine oxide group; $-OR_{100}$; $-CO_2R_{101}$; $-COR_{102}$; $-OCOR_{103}$; $-CONR_{104}R_{105}$; or $-SO_2R_{106}$, or adjacent substituents bond to each other to form a substituted or unsubstituted aromatic ring or a substituted or unsubstituted aliphatic ring, and $R_{100}$ to $R_{106}$ are the same as or different from each other and each independently hydrogen; a substituted or unsubstituted alkyl group; a substituted or unsubstituted cycloalkyl group; a substituted or unsubstituted aryl group; a substituted or unsubstituted heteroaryl group; or a substituted or unsubstituted amine group.

According to one embodiment of the present disclosure, $R_1$ to $R_5$ and $R_{11}$ to $R_{15}$ are the same as or different from each other, and each independently hydrogen; deuterium; a halogen group; a nitrile group; a nitro group; -COOH; a substituted or unsubstituted alkyl group having 1 to 30 carbon atoms; a substituted or unsubstituted cycloalkyl group having 3 to 30 carbon atoms; an aryl group having 6 to 30 carbon atoms unsubstituted or substituted with an alkyl group; a substituted or unsubstituted heteroaryl group having 2 to 30 carbon atoms; $-OR_{100}$; or $-CO_2R_{101}$, and $R_{100}$ and $R_{101}$ are the same as or different from each other and each independently a substituted or unsubstituted alkyl group having 1 to 30 carbon atoms.

According to one embodiment of the present disclosure, $R_1$ to $R_5$ and $R_{11}$ to $R_{15}$ are the same as or different from each other, and each independently hydrogen; deuterium; a halogen group; a nitrile group; a nitro group; -COOH; a substituted or unsubstituted alkyl group having 1 to 20 carbon atoms; a substituted or unsubstituted cycloalkyl group having 3 to 20 carbon atoms; an aryl group having 6 to 20 carbon atoms unsubstituted or substituted with an alkyl group; a substituted or unsubstituted heteroaryl group having 2 to 20 carbon atoms; $-OR_{100}$; or $-CO_2R_{101}$, and $R_{100}$ and $R_{101}$ are the same as or different from each other and each independently a substituted or unsubstituted alkyl group having 1 to 20 carbon atoms.

According to one embodiment of the present disclosure, $R_1$ to $R_5$ and $R_{11}$ to $R_{15}$ are the same as or different from each other, and each independently hydrogen; deuterium; a halogen group; a nitrile group; a nitro group; -COOH; a substituted or unsubstituted alkyl group having 1 to 10 carbon atoms; a substituted or unsubstituted cycloalkyl group having 3 to 10 carbon atoms; an aryl group having 6 to 10 carbon atoms unsubstituted or substituted with an alkyl group; a substituted or unsubstituted heteroaryl group having 2 to 10 carbon atoms; $-OR_{100}$; or $-CO_2R_{101}$, and $R_{100}$ and $R_{101}$ are the same as or different from each other and each independently a substituted or unsubstituted alkyl group having 1 to 10 carbon atoms.

According to one embodiment of the present disclosure, $R_1$ to $R_5$ and $R_{11}$ to $R_{15}$ are the same as or different from each other, and each independently hydrogen; deuterium; a fluoro group; a chloro group; a nitrile group; a nitro group; -COOH; a substituted or unsubstituted methyl group; a substituted or unsubstituted ethyl group; a substituted or unsubstituted propyl group; a substituted or unsubstituted tert-butyl group; a substituted or unsubstituted cyclohexyl group; a substituted or unsubstituted phenyl group; a substituted or unsubstituted pyridine group; a substituted or unsubstituted thiazole group; $-OR_{100}$; or $-CO_2R_{101}$, and $R_{100}$ and $R_{101}$ are the same as or different from each other and each independently a substituted or unsubstituted methyl group; or a substituted or unsubstituted ethyl group.

According to one embodiment of the present disclosure, $R_1$ to $R_5$ and $R_{11}$ to $R_{15}$ are the same as or different from each other, and each independently hydrogen; deuterium; a fluoro group; a chloro group; a nitrile group; a nitro group; -COOH; a methyl group unsubstituted or substituted with a halogen group; an ethyl group; a propyl group; a tert-butyl group; a cyclohexyl group; a phenyl group unsubstituted or substituted with an alkyl group; a pyridine group; a thiazole group; $-OR_{100}$; or $-CO_2R_{101}$, and $R_{100}$ and $R_{101}$ are the same as or different from each other and each independently a methyl group unsubstituted or substituted with a halogen group; or an ethyl group.

According to one embodiment of the present disclosure, $R_1$ to $R_5$ and $R_{11}$ to $R_{15}$ are the same as or different from each other, and each independently hydrogen; deuterium; a fluoro group; a chloro group; a nitrile group; a nitro group; -COOH; a methyl group unsubstituted or substituted with a chloro group; an ethyl group; a propyl group; a tert-butyl group; a cyclohexyl group; a phenyl group unsubstituted or substituted with a propyl group; a pyridine group; a thiazole group; $-OR_{100}$; or $-CO_2R_{101}$, and $R_{100}$ and $R_{101}$ are the same as or different from each other and each independently a methyl group unsubstituted or substituted with a chloro group; or an ethyl group.

According to one embodiment of the present disclosure, $R_1$ to $R_5$ and $R_{11}$ to $R_{15}$ are the same as or different from each other, and each independently hydrogen; deuterium; a fluoro group; a chloro group; a nitrile group; a nitro group; -COOH; a methyl group unsubstituted or substituted with a chloro group; an ethyl group; an isopropyl group; a tert-butyl group; a cyclohexyl group; a phenyl group unsubstituted or substituted with a propyl group; a pyridine group; a thiazole group; $-OCH_3$; $-OCF_3$; $-OC_2H_5$; $-CO_2CH_3$; or $-CO_2C_2H_5$.

According to one embodiment of the present disclosure, $R_7$ to $R_{10}$ and $R_{17}$ to $R_{20}$ are the same as or different from each other, and each independently hydrogen; deuterium; a nitrile group; a nitro group; a hydroxyl group; -COOH; a halogen group; an imide group; a substituted or unsubstituted alkyl group; a substituted or unsubstituted cycloalkyl group; a substituted or unsubstituted heterocyclic group; a substituted or unsubstituted aryl group; a substituted or unsubstituted heteroaryl group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted arylphosphine group; a substituted or unsubstituted phosphine oxide group; $-OR_{100}$; $-CO_2R_{101}$; $-COR_{102}$; $-OCOR_{103}$; $-CONR_{104}R_{105}$; or $-SO_2R_{106}$, or adjacent substituents bond to each other to form a substituted or unsubstituted aromatic ring or a substituted or unsubstituted aliphatic ring, and $R_{100}$ to $R_{106}$ are the same as or different from each other and each independently hydrogen; a substituted or unsubstituted alkyl group; a substituted or unsubstituted cycloalkyl group; a substituted or unsubstituted aryl group; a substituted or unsubstituted heteroaryl group; or a substituted or unsubstituted amine group.

According to one embodiment of the present disclosure, $R_7$ to $R_{10}$ and $R_{17}$ to $R_{20}$ are the same as or different from each other, and each independently hydrogen; deuterium; a halogen group; a nitrile group; a nitro group; a substituted or unsubstituted alkyl group having 1 to 30 carbon atoms; a substituted or unsubstituted cycloalkyl group having 3 to 30 carbon atoms; an aryl group having 6 to 30 carbon atoms unsubstituted or substituted with $-OR_{100}$ or an alkyl group; a substituted or unsubstituted heteroaryl group having 2 to 30 carbon atoms; $-OR_{100}$; $-CO_2R_{101}$; $-COR_{102}$; or $-SO_2R_{106}$, or adjacent substituents bond to each other to form a substituted or unsubstituted aromatic ring, $R_{100}$, $R_{101}$ and $R_{102}$ are the same as or different from each other and each independently a substituted or unsubstituted alkyl group having 1 to 30 carbon atoms, and $R_{106}$ is a substituted or unsubstituted alkyl group having 1 to 30 carbon atoms; or $-NH_2$.

According to one embodiment of the present disclosure, $R_7$ to $R_{10}$ and $R_{17}$ to $R_{20}$ are the same as or different from each other, and each independently hydrogen; deuterium; a halogen group; a nitrile group; a nitro group; a substituted or unsubstituted alkyl group having 1 to 20 carbon atoms; a substituted or unsubstituted cycloalkyl group having 3 to 20 carbon atoms; an aryl group having 6 to 20 carbon atoms unsubstituted or substituted with $-OR_{100}$ or an alkyl group; a substituted or unsubstituted heteroaryl group having 2 to 20 carbon atoms; $-OR_{100}$; $-CO_2R_{101}$; $-COR_{102}$; or $-SO_2R_{106}$, or adjacent substituents bond to each other to form a substituted or unsubstituted aromatic ring, $R_{100}$, $R_{101}$ and $R_{102}$ are the same as or different from each other and each independently a substituted or unsubstituted alkyl group having 1 to 20 carbon atoms, and $R_{106}$ is a substituted or unsubstituted alkyl group having 1 to 20 carbon atoms; or $-NH_2$.

According to one embodiment of the present disclosure, $R_7$ to $R_{10}$ and $R_{17}$ to $R_{20}$ are the same as or different from each other, and each independently hydrogen; deuterium; a halogen group; a nitrile group; a nitro group; a substituted or unsubstituted alkyl group having 1 to 10 carbon atoms; a substituted or unsubstituted cycloalkyl group having 3 to 10 carbon atoms; an aryl group having 6 to 10 carbon atoms unsubstituted or substituted with $-OR_{100}$ or an alkyl group; a substituted or unsubstituted heteroaryl group having 2 to 10 carbon atoms; $-OR_{100}$; $-CO_2R_{101}$; $-COR_{102}$; or $-SO_2R_{106}$, or adjacent substituents bond to each other to form a substituted or unsubstituted aromatic ring, $R_{100}$, $R_{101}$ and $R_{102}$ are the same as or different from each other and each independently a substituted or unsubstituted alkyl group having 1 to 10 carbon atoms, and $R_{106}$ is a substituted or unsubstituted alkyl group having 1 to 10 carbon atoms; or $-NH_2$.

According to one embodiment of the present disclosure, $R_7$ to $R_{10}$ and $R_{17}$ to $R_{20}$ are the same as or different from each other, and each independently hydrogen; deuterium; a fluoro group; a chloro group; a bromo group; a nitrile group; a nitro group; a substituted or unsubstituted methyl group; a substituted or unsubstituted propyl group; a substituted or unsubstituted tert-butyl group; a substituted or unsubstituted cyclohexyl group; a substituted or unsubstituted phenyl group; a substituted or unsubstituted thiophene group; a substituted or unsubstituted pyridine group; $-OR_{100}$; $-CO_2R_{101}$; $-COR_{102}$; or $- SO_2R_{106}$, or adjacent substituents bond to each other to form a substituted or unsubstituted benzene ring, $R_{100}$, $R_{101}$ and $R_{102}$ are the same as or different from each other and each independently a substituted or unsubstituted methyl group; or a substituted or unsubstituted ethyl group, and $R_{106}$ is a substituted or unsubstituted methyl group; or $-NH_2$.

According to one embodiment of the present disclosure, $R_7$ to $R_{10}$ and $R_{17}$ to $R_{20}$ are the same as or different from each other, and each independently hydrogen; deuterium; a fluoro group; a chloro group; a bromo group; a nitrile group; a nitro group; a methyl group unsubstituted or substituted with a fluoro group; an isopropyl group; a tert-butyl group; a cyclohexyl group; a phenyl group unsubstituted or substituted with $-OCH_3$ or an alkyl group; a thiophene group; a pyridine group; $-OCH_3$; $-CO_2CH_3$; $-CO_2C_2H_5$; $-SO_2CH_3$ or $-SO_2NH_2$, or adjacent substituents bond to each other to form a benzene ring.

According to one embodiment of the present disclosure, $R_6$ and $R_{16}$ are the same as or different from each other, and each independently hydrogen; deuterium; a nitrile group; a nitro group; a hydroxyl group; -COOH; a halogen group; an imide group; a substituted or unsubstituted alkyl group; a substituted or unsubstituted cycloalkyl group; a substituted or unsubstituted heterocyclic group; a substituted or unsubstituted aryl group; a substituted or unsubstituted heteroaryl group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted arylphosphine group; a substituted or unsubstituted phosphine oxide group; a substituted or unsubstituted styryl group; $-OR_{100}$; $-CO_2R_{101}$; $-COR_{102}$; $-OCOR_{103}$; $-CONR_{104}R_{105}$; or $-SO_2R_{106}$, and $R_{100}$ to $R_{106}$ are the same as or different from each other and each independently hydrogen; a substituted or unsubstituted alkyl group; a substituted or unsubstituted cycloalkyl group; a substituted or unsubstituted aryl group; a substituted or unsubstituted heteroaryl group; or a substituted or unsubstituted amine group.

In one embodiment of the present disclosure, $R_6$ and $R_{16}$ are the same as or different from each other, and each independently a nitrile group; a substituted or unsubstituted alkyl group; a substituted or unsubstituted alkenyl group; a substituted or unsubstituted aryl group; a substituted or unsubstituted heteroaryl group; $-OR_{100}$; or $-CO_2R_{101}$.

In one embodiment of the present disclosure, $R_6$ and $R_{16}$ are the same as or different from each other, and each independently a nitrile group; a substituted or unsubstituted alkyl group having 1 to 30 carbon atoms; a substituted or unsubstituted alkenyl group having 2 to 30 carbon atoms; a substituted or unsubstituted aryl group having 6 to 30 carbon atoms; a substituted or unsubstituted heteroaryl group having 2 to 30 carbon atoms; $-OR_{100}$; or $-CO_2R_{101}$.

In one embodiment of the present disclosure, $R_6$ and $R_{16}$ are the same as or different from each other, and each independently a nitrile group; a substituted or unsubstituted alkyl group having 1 to 20 carbon atoms; a substituted or unsubstituted alkenyl group having 2 to 20 carbon atoms; a substituted or unsubstituted aryl group having 6 to 20 carbon atoms; a substituted or unsubstituted heteroaryl group having 2 to 20 carbon atoms; $-OR_{100}$; or $-CO_2R_{101}$.

In one embodiment of the present disclosure, $R_6$ and $R_{16}$ are the same as or different from each other, and each independently a nitrile group; a substituted or unsubstituted alkyl group having 1 to 10 carbon atoms; a substituted or unsubstituted alkenyl group having 2 to 10 carbon atoms; a substituted or unsubstituted aryl group having 6 to 10 carbon atoms; a substituted or unsubstituted heteroaryl group having 2 to 10 carbon atoms; $-OR_{100}$; or $-CO_2R_{101}$.

In one embodiment of the present disclosure, $R_6$ and $R_{16}$ are the same as or different from each other, and each independently a nitrile group; a substituted or unsubstituted methyl group; a substituted or unsubstituted ethyl group; a substituted or unsubstituted propyl group; a substituted or unsubstituted ethenyl group; a substituted or unsubstituted phenyl group; a substituted or unsubstituted pyridine group; a substituted or unsubstituted benzoxazole group; $-OR_{100}$; or $-CO_2R_{101}$ .

According to one embodiment of the present disclosure, $R_6$ and $R_{16}$ are the same as or different from each other, and each independently a nitrile group; a methyl group unsubstituted or substituted with a fluoro group; a substituted or unsubstituted ethyl group; a substituted or unsubstituted isopropyl group; a phenyl group unsubstituted or substituted with one or more substituents selected from the group consisting of a halogen group, a nitrile group, a nitro group, -COOH, an alkyl group, $-OR_{100}$ and $-CO_2R_{101}$; a substituted or unsubstituted benzoxazole group; a substituted or unsubstituted pyridine group; a styryl group unsubstituted or substituted with an alkyl group or a nitro group; $-OR_{100}$; or $-CO_2R_{101}$, and $R_{100}$ and $R_{101}$ are the same as or different from each other and each independently a substituted or unsubstituted methyl group; or a substituted or unsubstituted ethyl group.

In one embodiment of the present disclosure, $R_6$ and $R_{16}$ are the same as or different from each other, and each independently a nitrile group; a methyl group; an ethyl group; an isopropyl group; an ethenyl group substituted with a phenyl group substituted with a nitro group, an isopropyl group or a tert-butyl group; a phenyl group unsubstituted or substituted with a nitro group, fluorine, a nitrile group; -COOH, a methyl group, a methoxy group, $-OCF_3$ or $-COOCH_2CH_3$; a pyridine group; a benzoxazole group; $-OR_{100}$; or $-CO_2R_{101}$, and $R_{100}$ and $R_{101}$ are the same as or different from each other and each independently a substituted or unsubstituted methyl group; or a substituted or unsubstituted ethyl group.

In one embodiment of the present disclosure, Chemical Formula A is any one selected from the group consisting of the following materials.

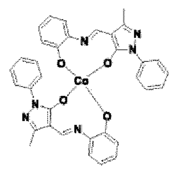
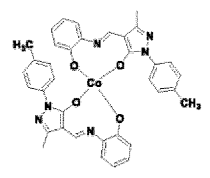
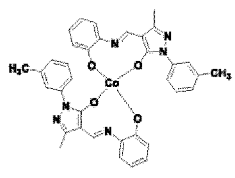
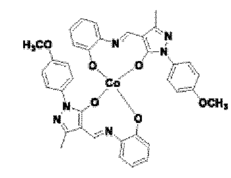
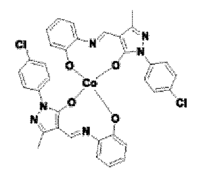

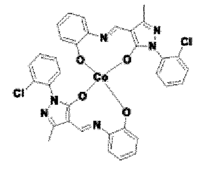
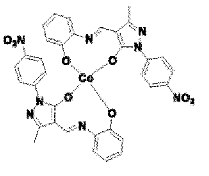
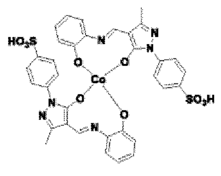
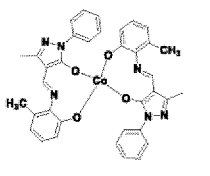
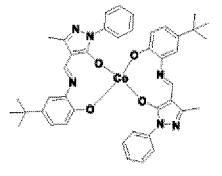

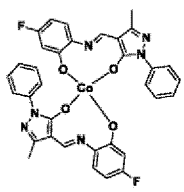
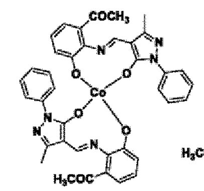
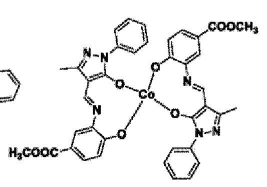
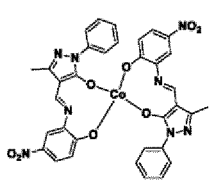
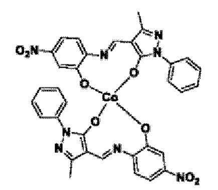

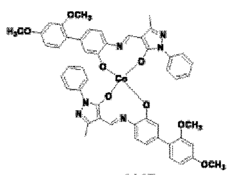
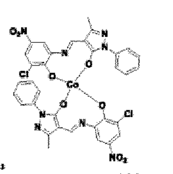
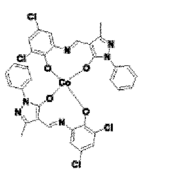
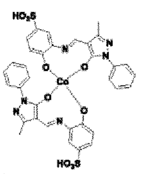
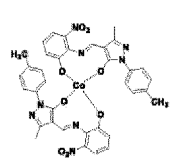
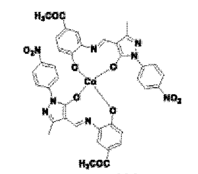

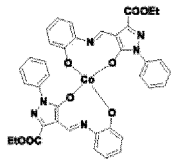
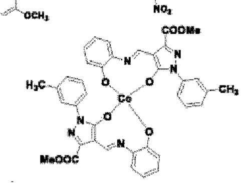
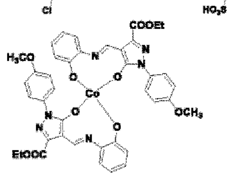
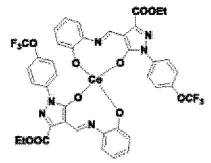
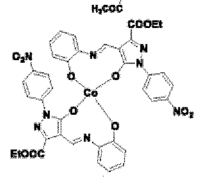

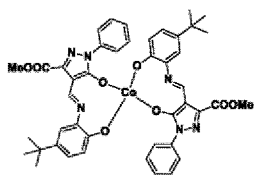
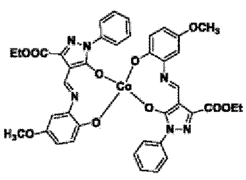
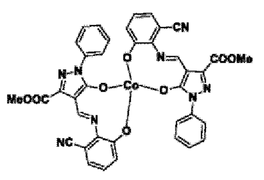
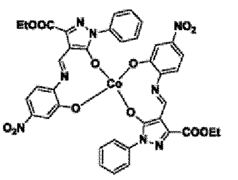

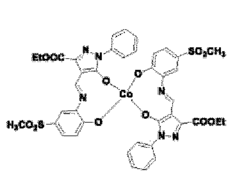
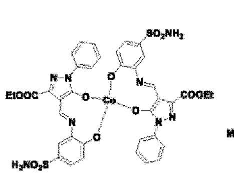
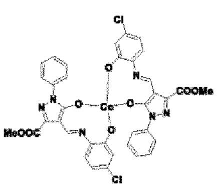
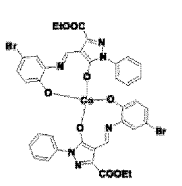
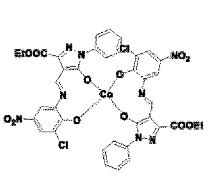

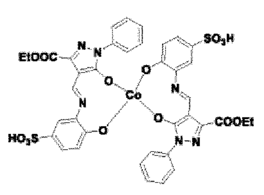
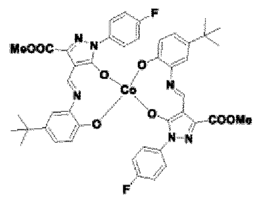
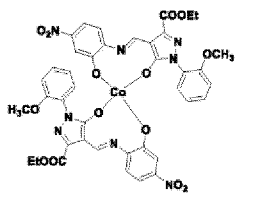
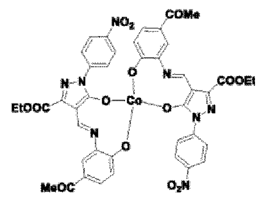

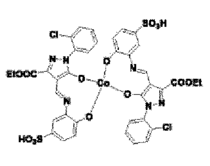
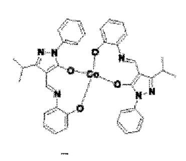
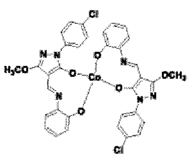
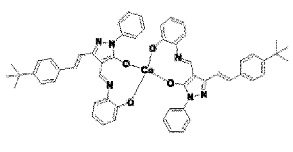
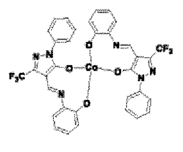

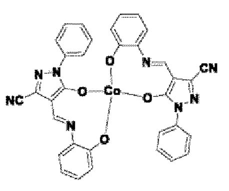
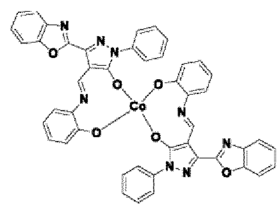
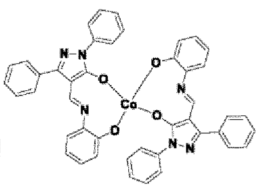
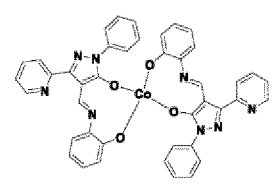

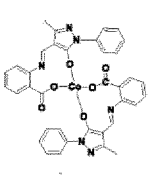
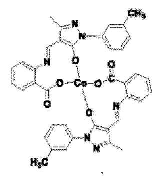
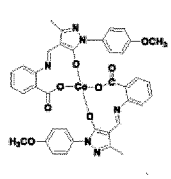
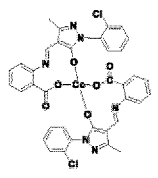
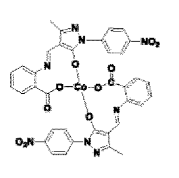
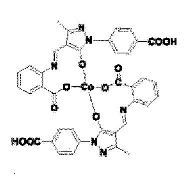

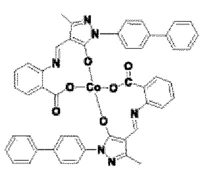
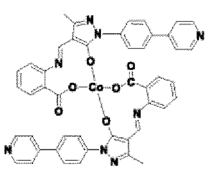
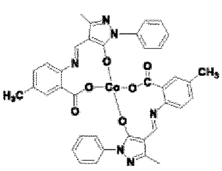
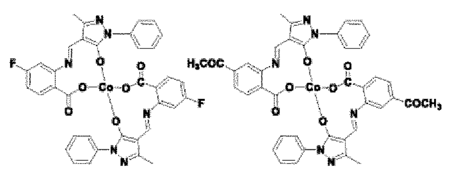

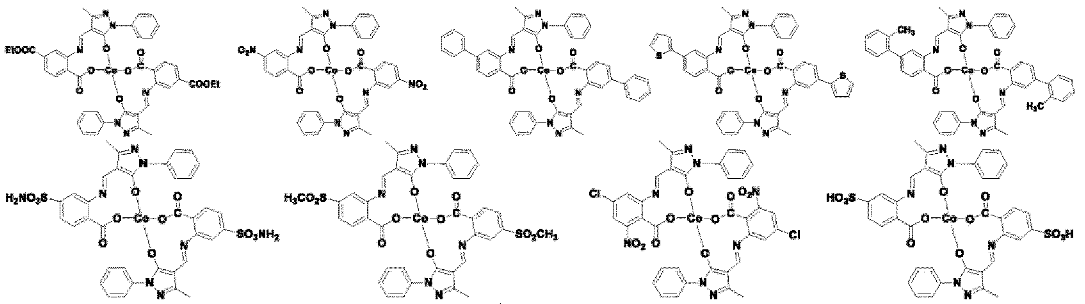

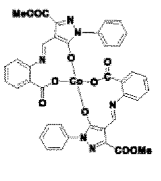
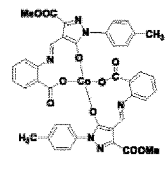
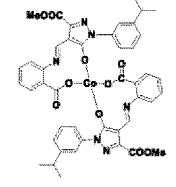
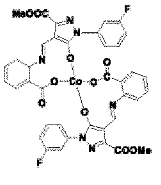
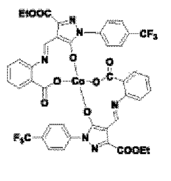
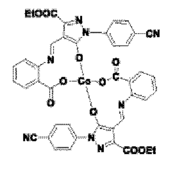

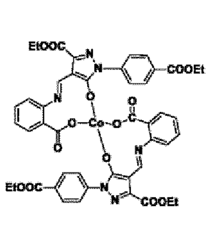
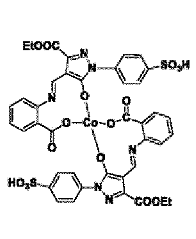
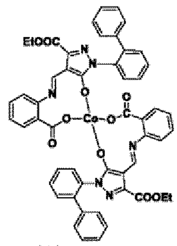
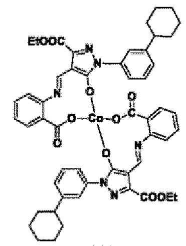
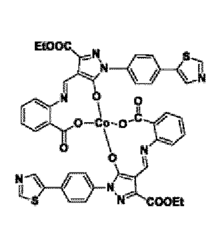
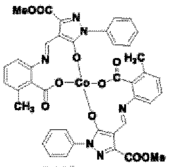
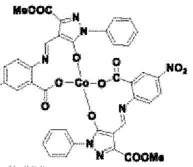
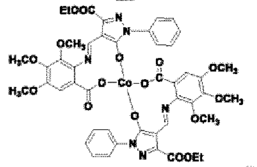
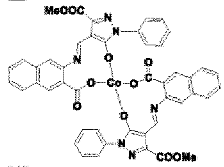
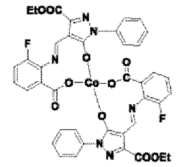
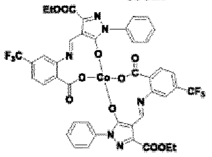
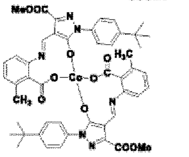
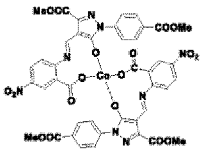
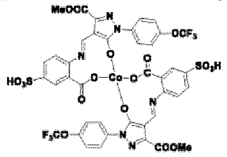
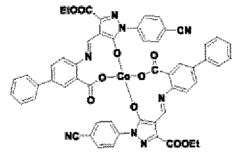
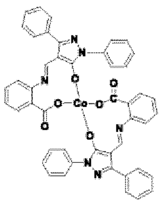
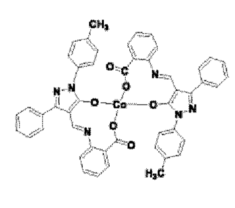
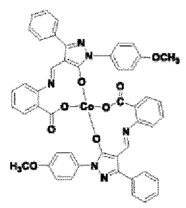
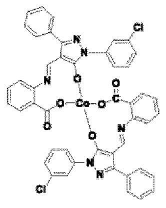
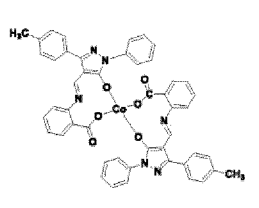
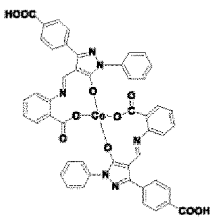
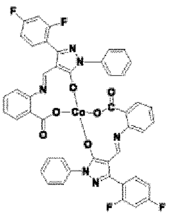
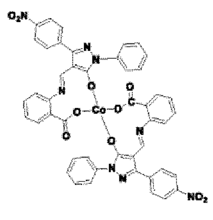
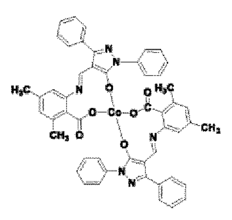
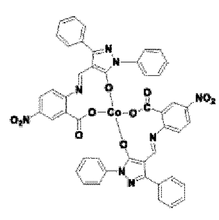
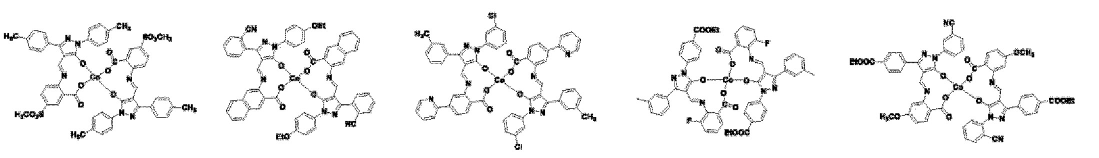
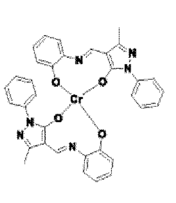
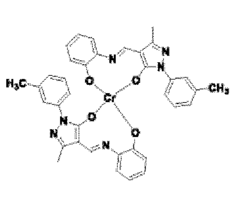
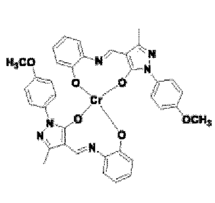
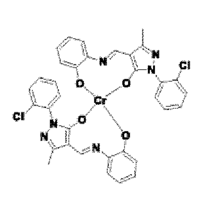
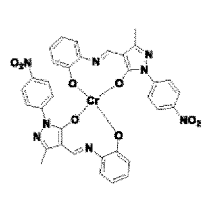
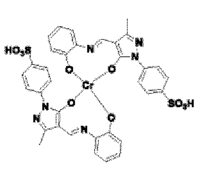
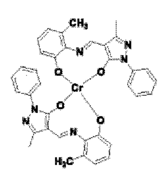
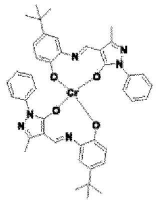
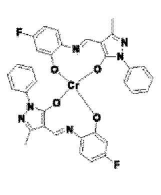
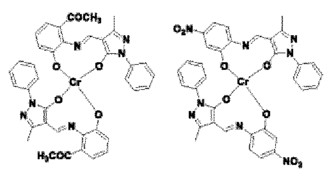

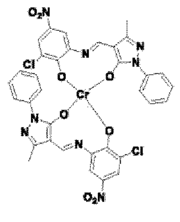 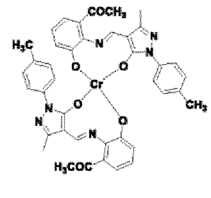 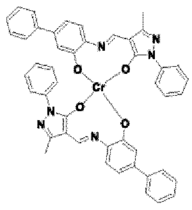 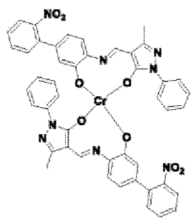 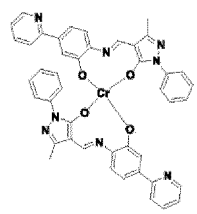

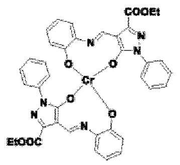 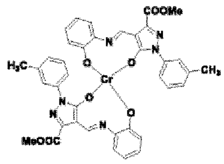 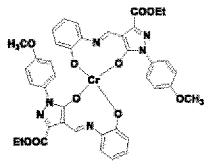 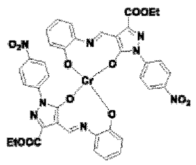 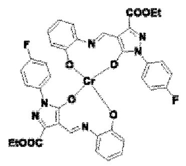

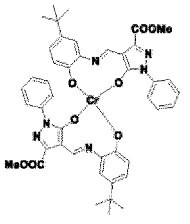 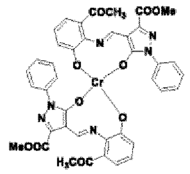 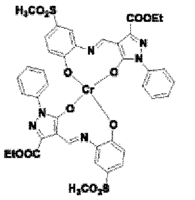 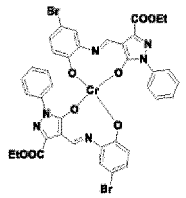 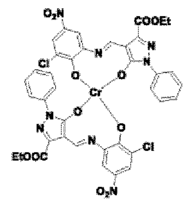

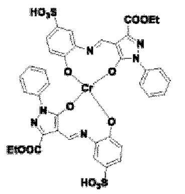 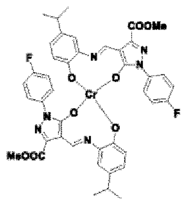 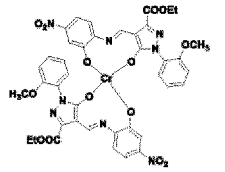 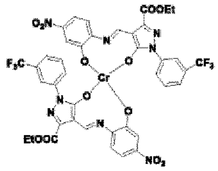 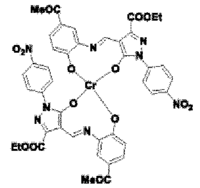

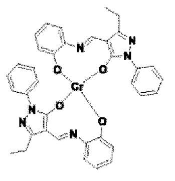 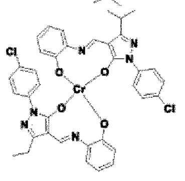 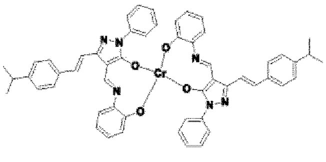 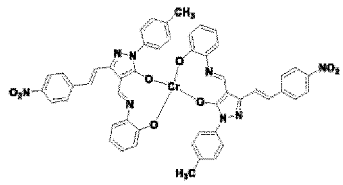

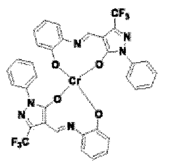 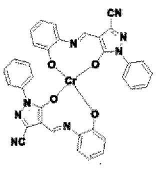 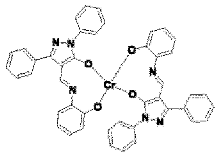 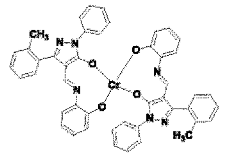 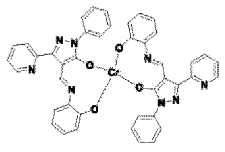

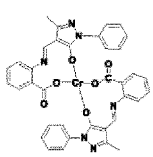 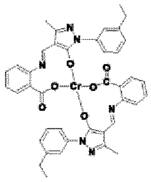 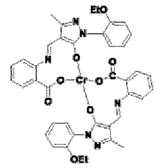 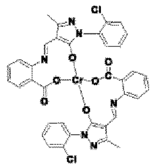 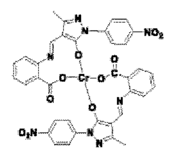 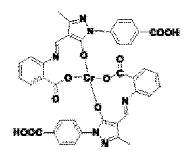

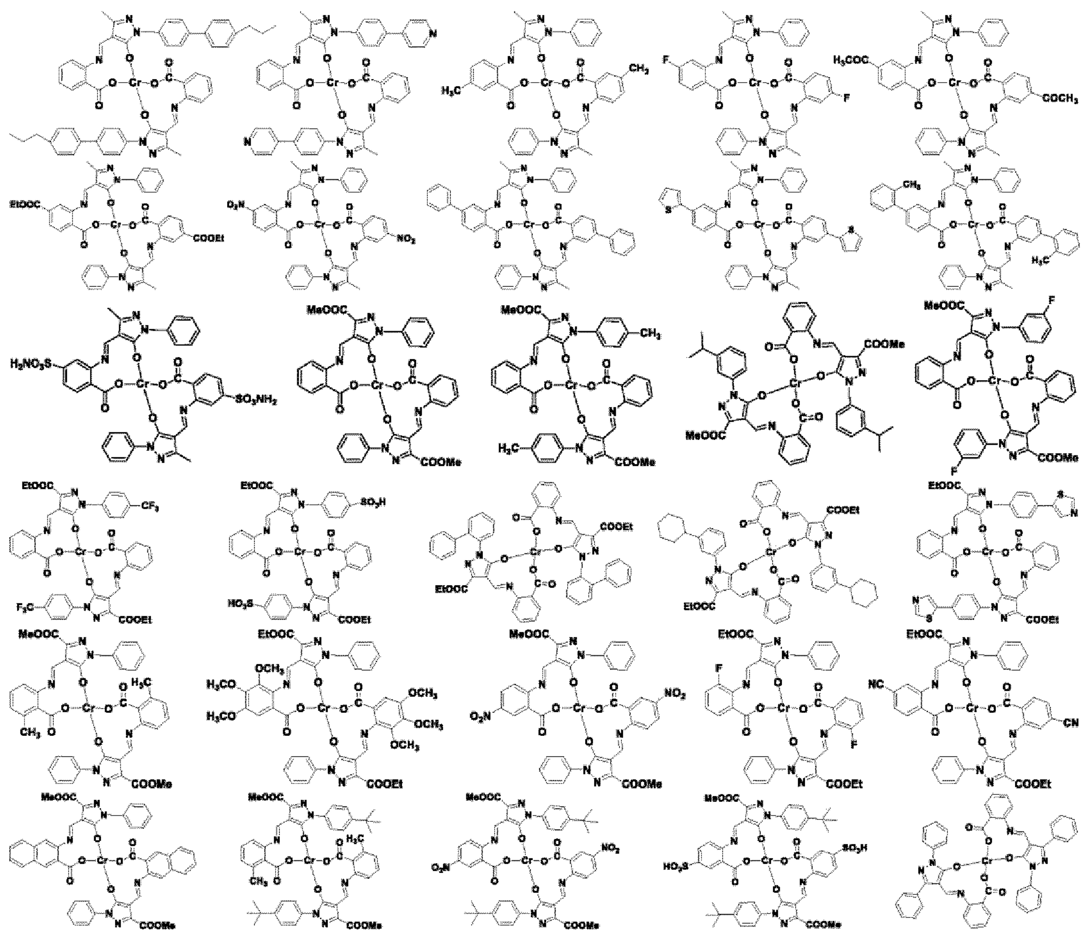

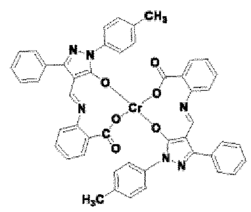

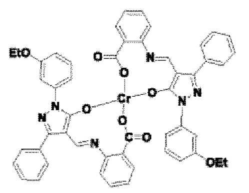

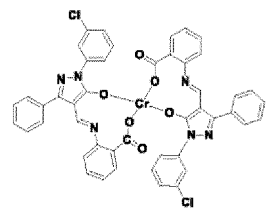

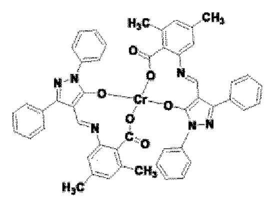

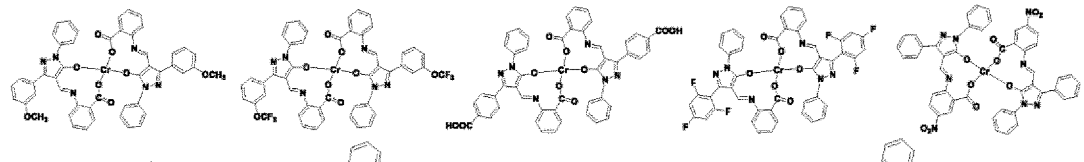

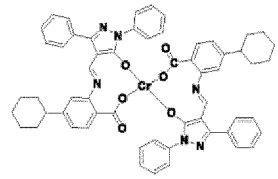

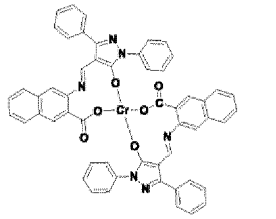

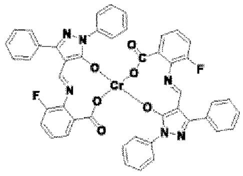

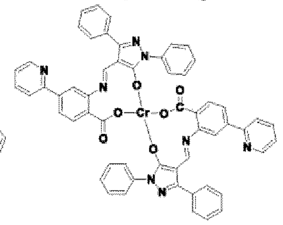

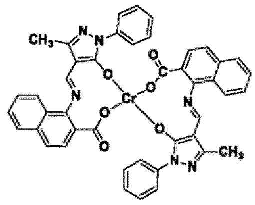

In the materials, Me means a methyl group and Et means an ethyl group.

According to one embodiment of the present disclosure, the coordination compound further includes $(B^+)m$.

According to one embodiment of the present disclosure, $B^+$ is $H^+$; $Na^+$; $K^+$; $P^+ReRfRgRh$; $N^+RiRjRkRl$; $S^+RmRnRo$; or a cationic heteroring, and Re to Ro are the same as or different from each other and each independently hydrogen; a substituted or unsubstituted alkyl group; or a substituted or unsubstituted cycloalkyl group.

According to one embodiment of the present disclosure, the cationic heteroring is any one selected from the group consisting of the following Structural Formulae B1 to B4.

[Structural Formula B1]

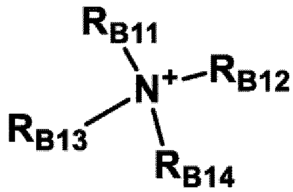

[Structural Formula B2]

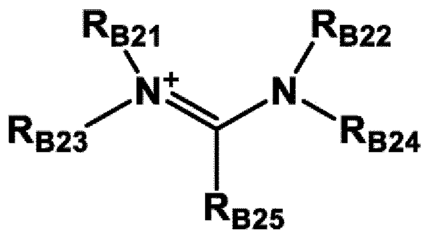

[Structural Formula B3]

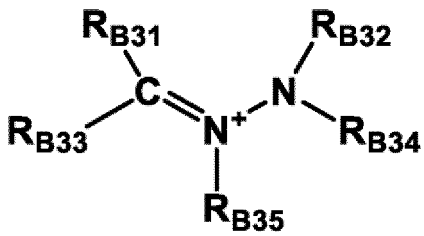

[Structural Formula B4]

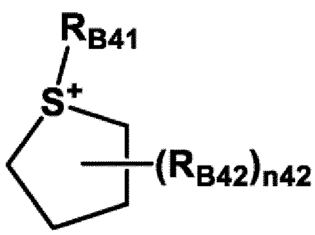

In Structural Formulae B1 to B4,

$R_{B11}$ and $R_{B12}$ bond to each other to form a substituted or unsubstituted heteroring having 4 to 30 carbon atoms, and $R_{B13}$ and $R_{B14}$ are the same as or different from each other and each independently hydrogen; a hydrocarbon group having 1 to 30 carbon atoms; or a substituent including one, two or more heteroatoms selected from the group consisting of N, O, P, S, Si and Se,
$R_{B21}$ and $R_{B22}$ bond to each other to form a substituted or unsubstituted heteroring having 2 to 30 carbon atoms, and $R_{B23}$ to $R_{B25}$ are the same as or different from each other and each independently hydrogen; a hydrocarbon group having 1 to 30 carbon atoms; or a substituent including one, two or more heteroatoms selected from the group consisting of N, O, P, S, Si and Se,
$R_{B31}$ and $R_{B32}$ bond to each other to form a substituted or unsubstituted heteroring having 2 to 30 carbon atoms, and $R_{B33}$ to $R_{B35}$ are the same as or different from each other and each independently hydrogen; a hydrocarbon group having 1 to 30 carbon atoms; or a substituent including one, two or more heteroatoms selected from the group consisting of N, O, P, S, Si and Se, and
$R_{B41}$ is hydrogen; a hydrocarbon group having 1 to 30 carbon atoms; or a substituent including one, two or more heteroatoms selected from the group consisting of N, O, P, S, Si and Se, $R_{B42}$ is hydrogen; a hydrocarbon group having 1 to 30 carbon atoms; or a substituent including one, two or more heteroatoms selected from the group consisting of N, O, P, S, Si and Se, and n42 is an integer of 1 to 8.

According to another embodiment of the present disclosure, $B^+$ is $H^+$; $Na^+$; $K^+$; a phosphonium cation; an ammonium cation; a sulfonium cation; or a cationic heteroring, and m is an integer of 1 or 2.
In another embodiment of the present disclosure, B is an alkali metal; $NR_4$; or $PR'_4$, and R and R' are the same as or different from each other and each independently hydrogen; a substituted or unsubstituted alkyl group having 1 to 10 carbon atoms; or a substituted or unsubstituted cycloalkyl group having 3 to 10 carbon atoms, or two or more Rs bond to each other to form a substituted or unsubstituted aromatic ring having 2 to 10 carbon atoms or a substituted or unsubstituted aliphatic ring having 2 to 10 carbon atoms.
In one embodiment of the present disclosure, B is hydrogen.
In one embodiment of the present disclosure, B is an alkali metal.
The alkali metal means a chemical element in Group 1 of the periodic table other than hydrogen, and examples thereof may include lithium, sodium, potassium, rubidium, cesium or francium.
In one embodiment of the present disclosure, B is Na or K.
In one embodiment of the present disclosure, B is $NR_4$.
In one embodiment of the present disclosure, B is $PR'_4$.
In one embodiment of the present disclosure, R and R' are the same as or different from each other, and each independently hydrogen; a substituted or unsubstituted alkyl group having 1 to 30 carbon atoms; or a substituted or unsubstituted cycloalkyl group having 3 to 30 carbon atoms, or two or more Rs bond to each other to form a substituted or unsubstituted aromatic heteroring having 6 to 30 carbon atoms or a substituted or unsubstituted aliphatic heteroring having 1 to 30 carbon atoms.
In one embodiment of the present disclosure, R and R' are the same as or different from each other, and each independently hydrogen; a substituted or unsubstituted alkyl group having 1 to 20 carbon atoms; or a substituted or unsubstituted cycloalkyl group having 3 to 20 carbon atoms, or two or more Rs bond to each other to form a substituted or unsubstituted aromatic heteroring having 6 to 20 carbon atoms or a substituted or unsubstituted aliphatic heteroring having 1 to 20 carbon atoms.
In one embodiment of the present disclosure, R and R' are the same as or different from each other, and each independently hydrogen; a substituted or unsubstituted alkyl group having 1 to 10 carbon atoms; or a substituted or unsubstituted cycloalkyl group having 3 to 10 carbon atoms, or two or more Rs bond to each other to form a substituted or unsubstituted aromatic heteroring having 6 to 12 carbon atoms or a substituted or unsubstituted aliphatic heteroring having 1 to 10 carbon atoms.
In one embodiment of the present disclosure, B is $NR_4$; or $PR'_4$, and R and R' are the same as or different from each other and each independently hydrogen; a substituted or unsubstituted alkyl group having 1 to 10 carbon atoms; or a substituted or unsubstituted cycloalkyl group having 3 to 10 carbon atoms, or two or more Rs bond to each other to form a substituted or unsubstituted aromatic ring having 2 to 10 carbon atoms or a substituted or unsubstituted aliphatic ring having 2 to 10 carbon atoms.
In one embodiment of the present disclosure, B is an alkali metal; $NR_4$; or $PR'_4$, and R and R' are the same as or different from each other and each independently hydrogen; a substituted or unsubstituted alkyl group having 1 to 10 carbon atoms; or a substituted or unsubstituted cycloalkyl group having 3 to 10 carbon atoms, or two or more Rs bond to each other to form a substituted or unsubstituted aromatic ring having 2 to 10 carbon atoms or a substituted or unsubstituted aliphatic ring having 2 to 10 carbon atoms.

In one embodiment of the present disclosure, R is hydrogen; a substituted or unsubstituted butyl group; a substituted or unsubstituted hexyl group; a substituted or unsubstituted decyl group; a substituted or unsubstituted dodecyl group; or a substituted or unsubstituted cyclohexyl group, or bonds to each other to form a compound represented by the following Chemical Formula N-1; or a compound represented by the following Chemical Formula N-2.

[Chemical Formula N-1]

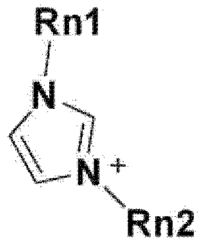

[Chemical Formula N-2]

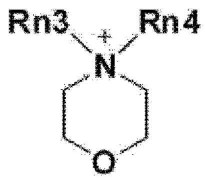

In Chemical Formulae N-1 and N-2,
Rn1 to Rn4 are the same as or different from each other, and each independently hydrogen; or a substituted or unsubstituted alkyl group.

In one embodiment of the present disclosure, R is hydrogen; an n-butyl group; an n-hexyl group; a hexyl group substituted with an ethyl group; an n-decyl group; an n-dodecyl group; or a cyclohexyl group, or bonds to each other to form the compound represented by Chemical Formula N-1; or the compound represented by Chemical Formula N-2.

In one embodiment of the present disclosure, R is hydrogen; an n-butyl group; an n-hexyl group; or a hexyl group substituted with an ethyl group, or bonds to each other to form the compound represented by Chemical Formula N-1; or the compound represented by Chemical Formula N-2.

The hexyl group substituted with an ethyl group may be a 2-ethylhexyl group.

In one embodiment of the present disclosure, R' is a substituted or unsubstituted butyl group.

In one embodiment of the present disclosure, R' is an n-butyl group.

In one embodiment of the present disclosure, B is $NR_4$, $NR_4$ is $NH_4$; $NHR''_3$; $NH_2R''_2$; $NR''_4$; a compound represented by the following Chemical Formula N-1; or a compound represented by the following Chemical Formula N-2, and R" is a substituted or unsubstituted alkyl group having 1 to 20 carbon atoms; or a substituted or unsubstituted cycloalkyl group having 3 to 20 carbon atoms.

[Chemical Formula N-1]

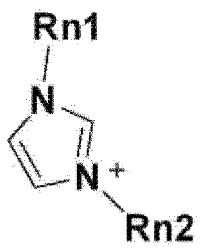

[Chemical Formula N-2]

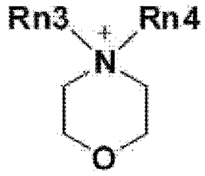

In Chemical Formulae N-1 and N-2,

Rn1 to Rn4 are the same as or different from each other, and each independently hydrogen; or a substituted or unsubstituted alkyl group.

In one embodiment of the present disclosure, Rn1 to Rn4 are the same as or different from each other, and each independently hydrogen; or a substituted or unsubstituted alkyl group having 1 to 30 carbon atoms.

In one embodiment of the present disclosure, Rn1 to Rn4 are the same as or different from each other, and each independently hydrogen; or a substituted or unsubstituted alkyl group having 1 to 20 carbon atoms.

In one embodiment of the present disclosure, Rn1 to Rn4 are the same as or different from each other, and each independently hydrogen; or a substituted or unsubstituted alkyl group having 1 to 10 carbon atoms.

In one embodiment of the present disclosure, Rn1 to Rn4 are the same as or different from each other, and each independently hydrogen; a substituted or unsubstituted methyl group; a substituted or unsubstituted ethyl group; or a substituted or unsubstituted butyl group.

In one embodiment of the present disclosure, Rn1 to Rn4 are the same as or different from each other, and each independently hydrogen; a methyl group; an ethyl group; or an n-butyl group.

In one embodiment of the present disclosure, Rn1 is an n-butyl group.

In one embodiment of the present disclosure, Rn2 is a methyl group.

In one embodiment of the present disclosure, Rn3 and Rn4 are hydrogen; or an ethyl group.

According to one embodiment of the present disclosure, the compound further including $(B^+)m$ is any one selected from the group consisting of the following compounds.

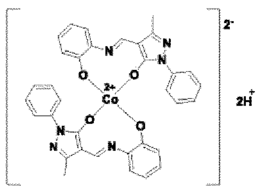
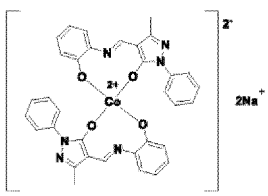
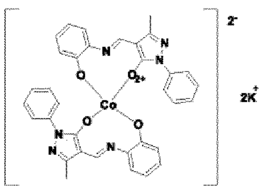
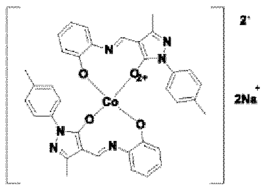

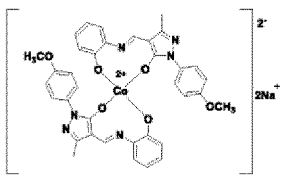
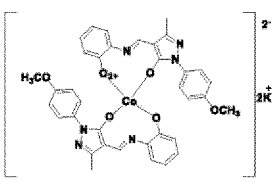
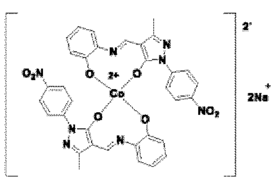
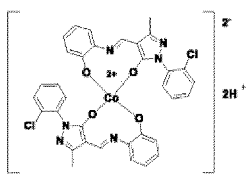

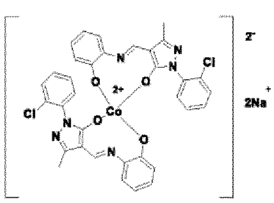
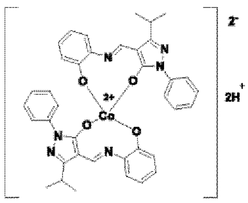
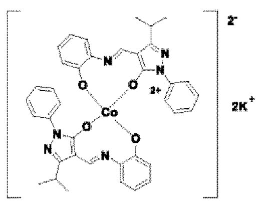
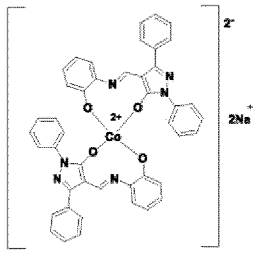

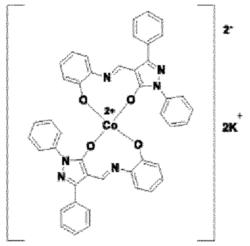
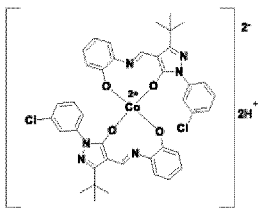
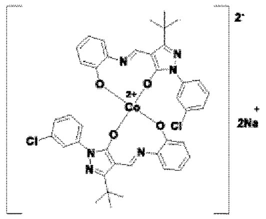
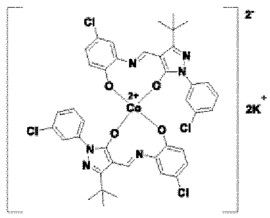

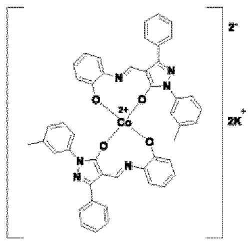
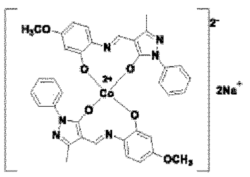
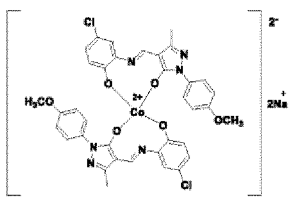
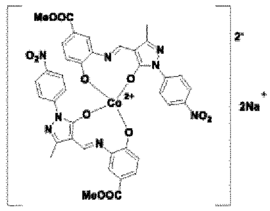

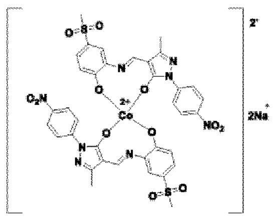
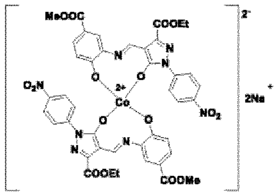
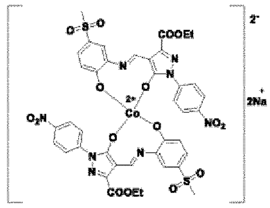
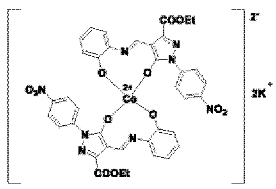

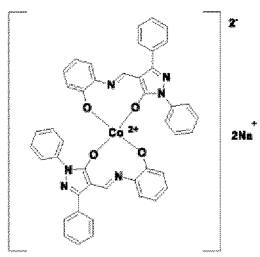
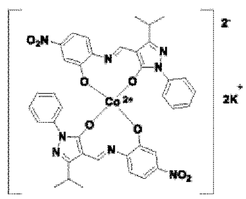
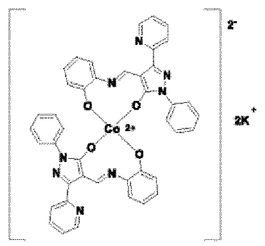
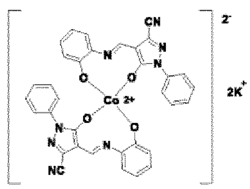

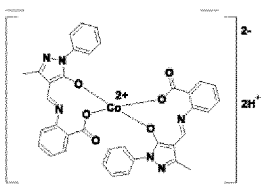
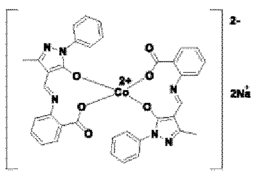
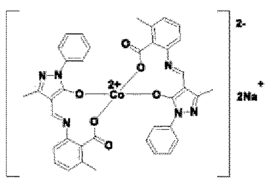
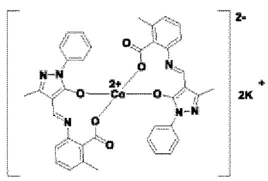

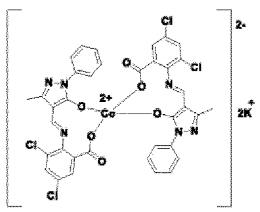
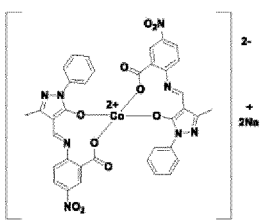
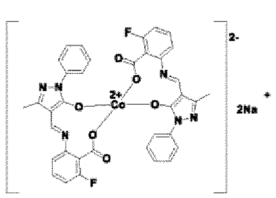
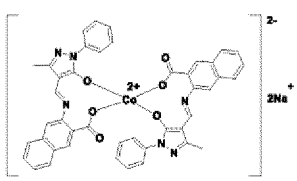

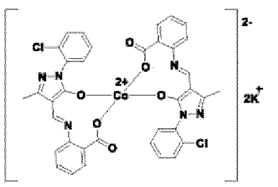
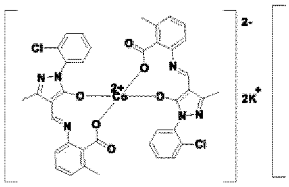
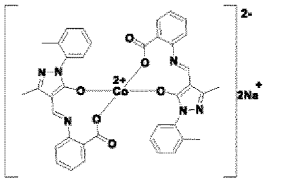
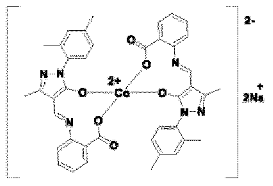

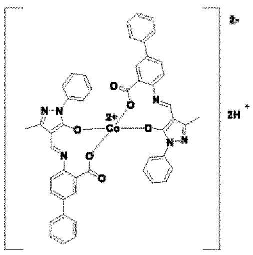
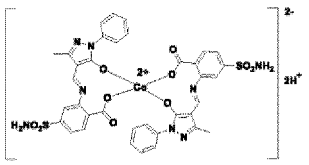
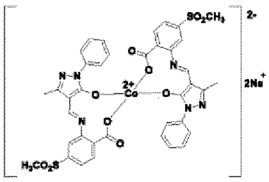
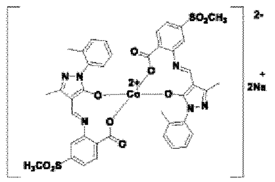

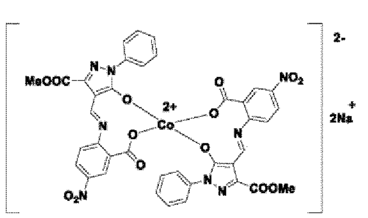
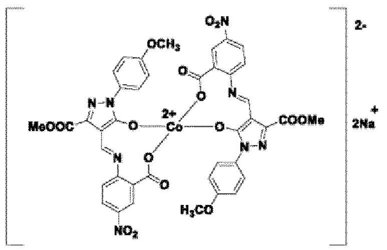
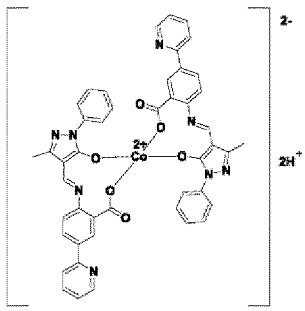

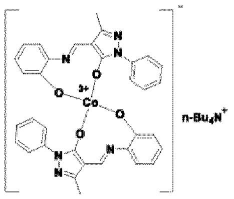
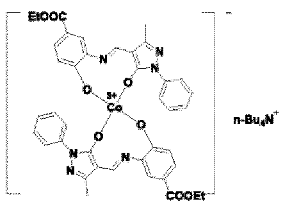
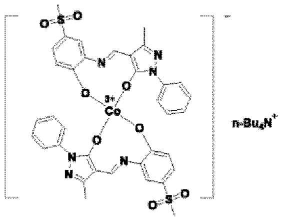
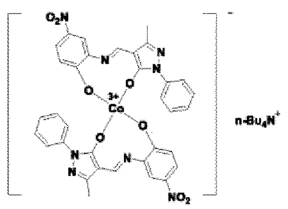

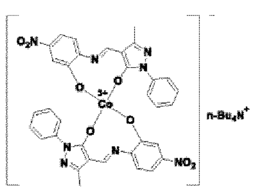
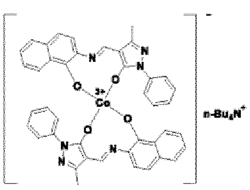
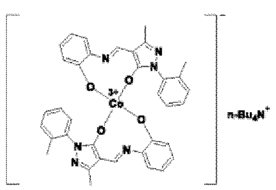
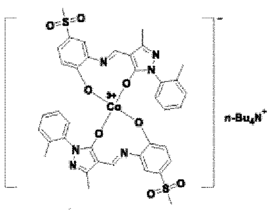

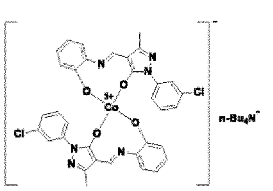
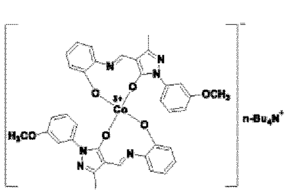
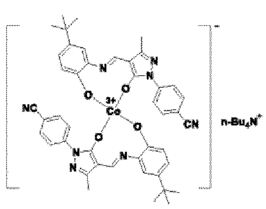
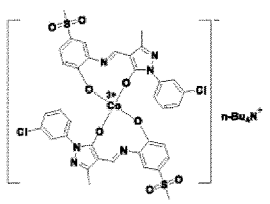

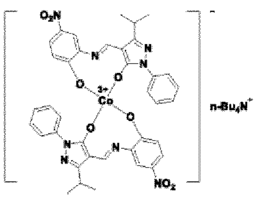
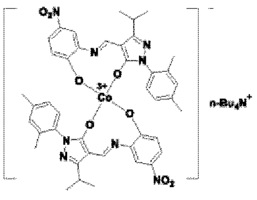
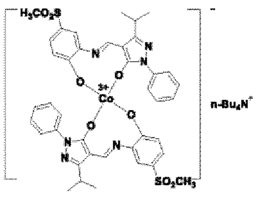
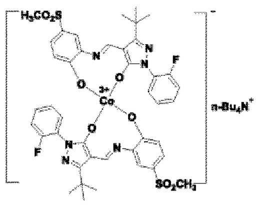

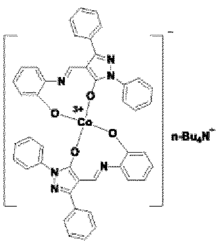
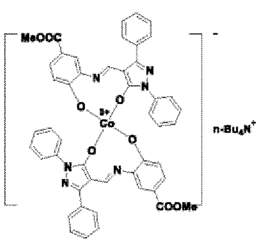
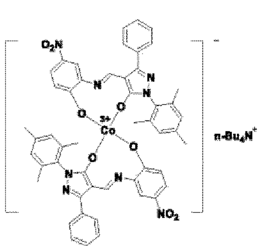
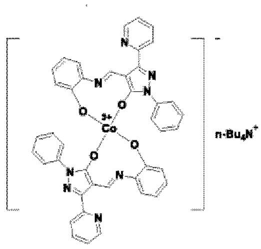

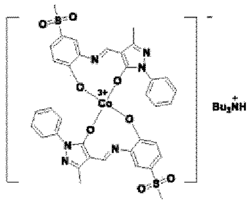
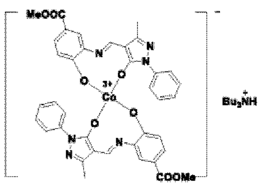
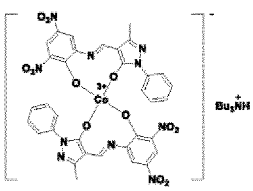
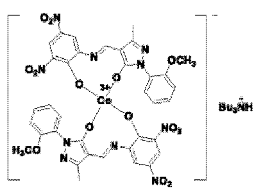

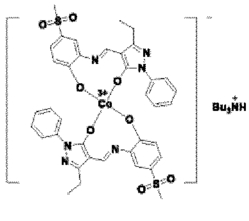
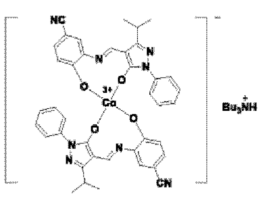
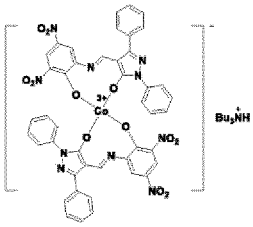
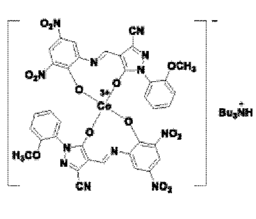

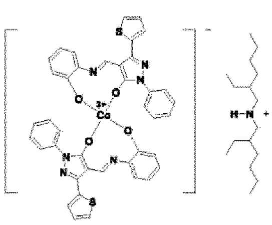
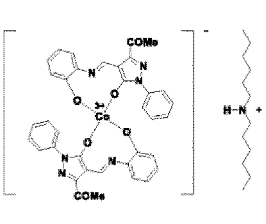
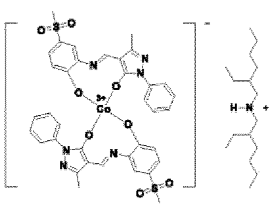
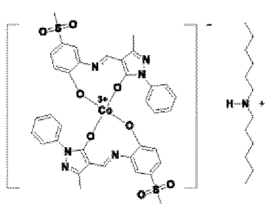

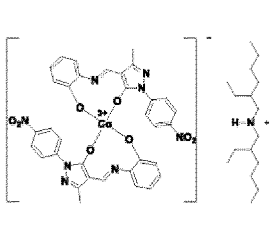
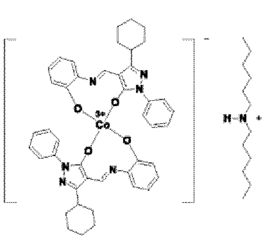
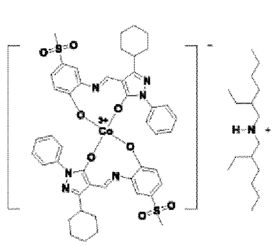
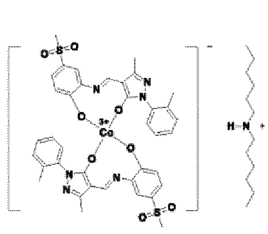

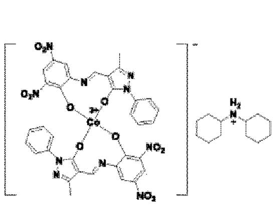
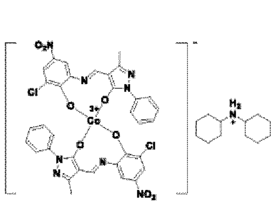
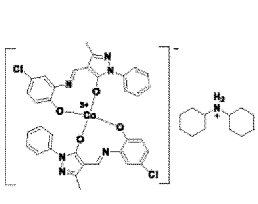
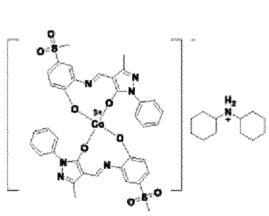

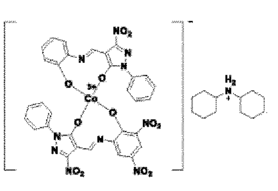
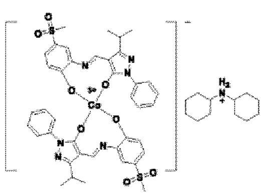
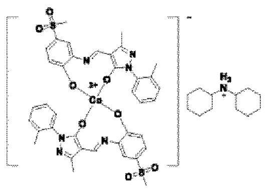
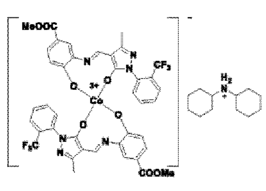
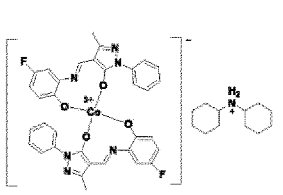
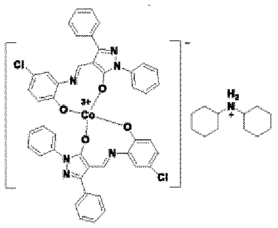
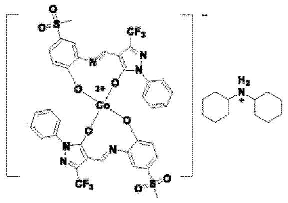
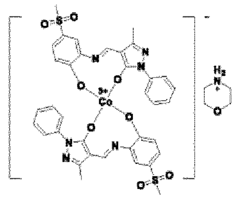
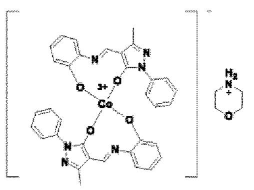
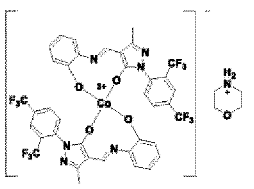
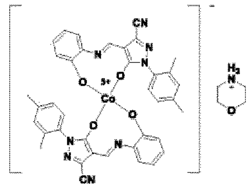
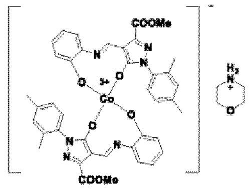
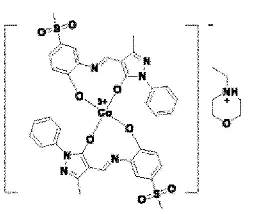
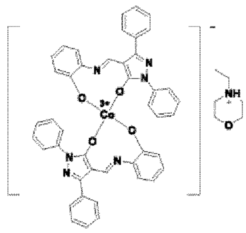
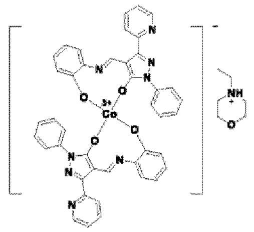
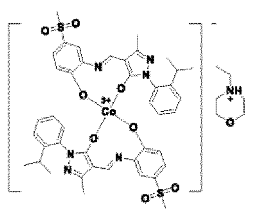
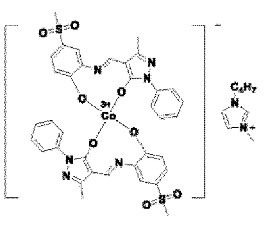
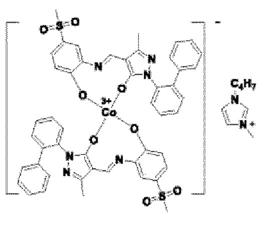
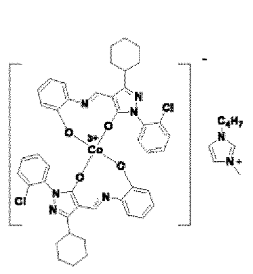
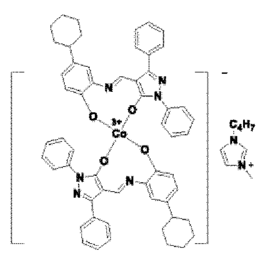
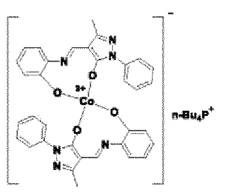
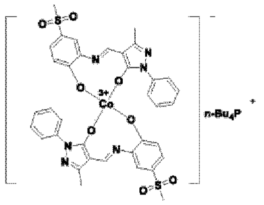
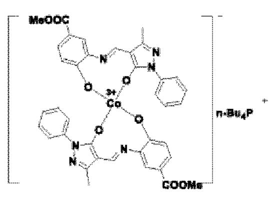
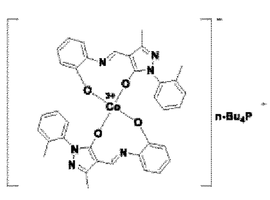

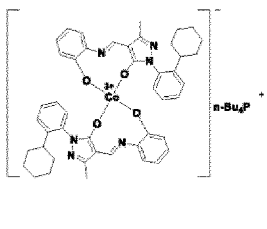
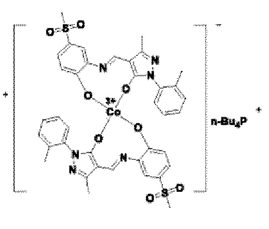
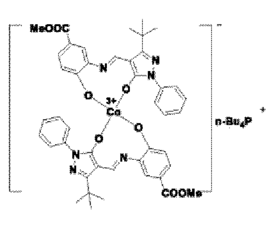
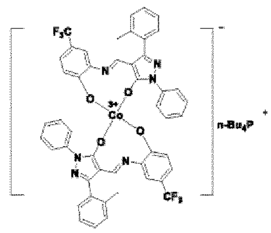

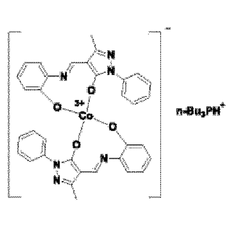
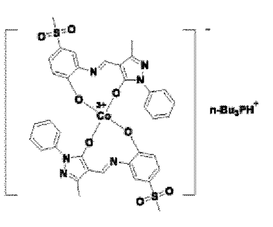
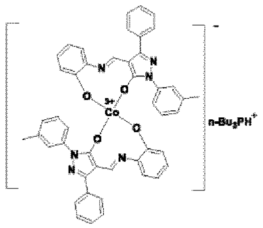
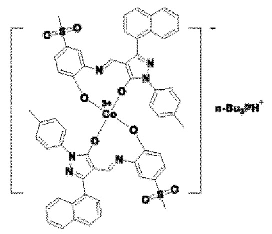

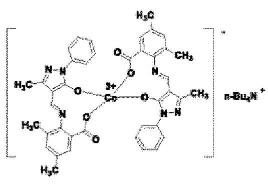
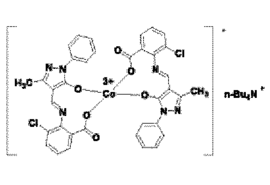
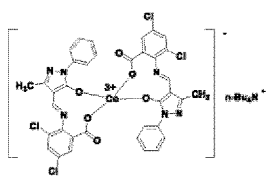
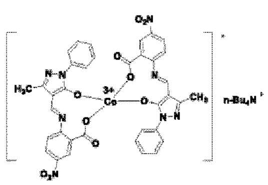

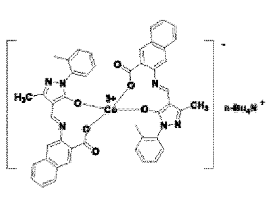
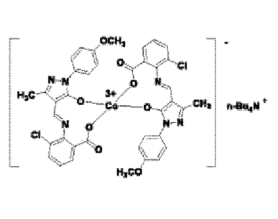
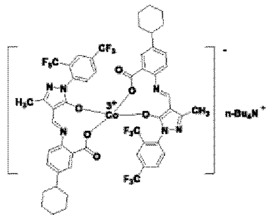
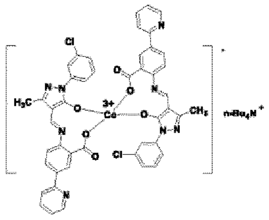

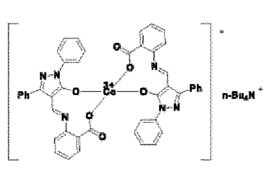
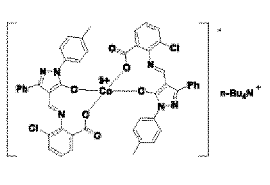
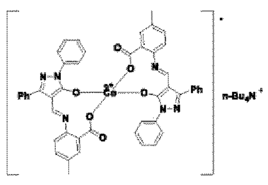
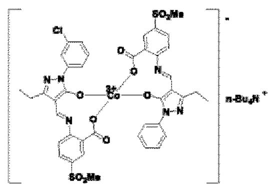

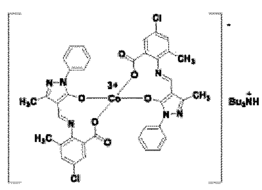
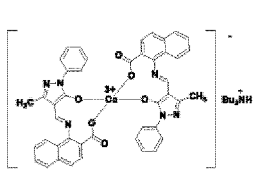
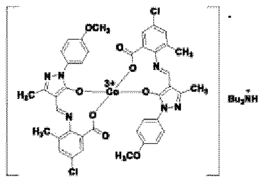
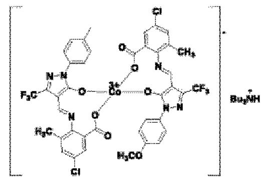

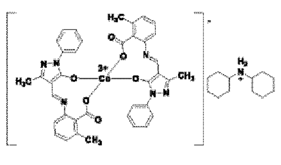
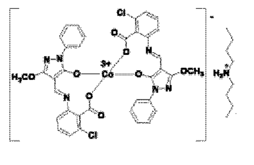
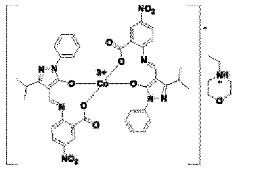
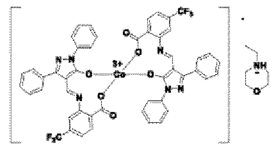

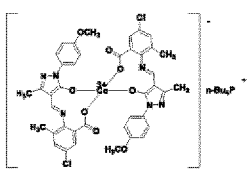
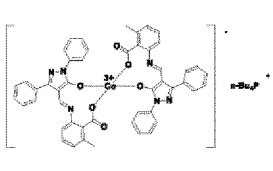
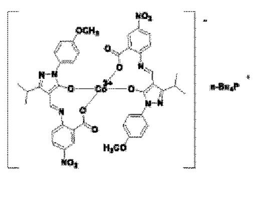
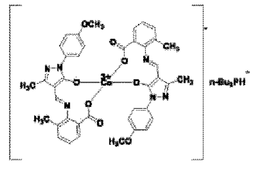

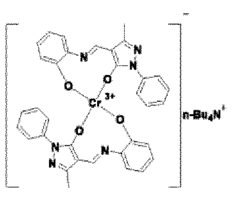
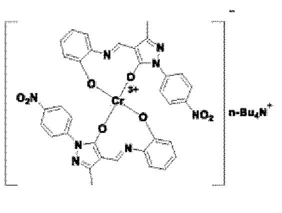
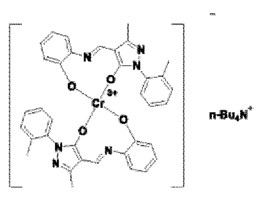
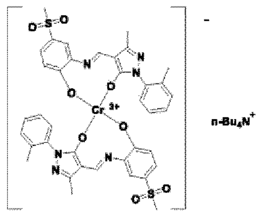

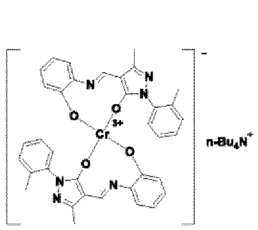
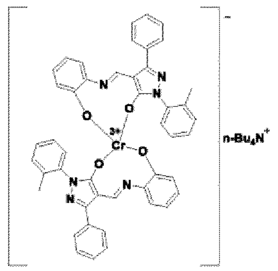
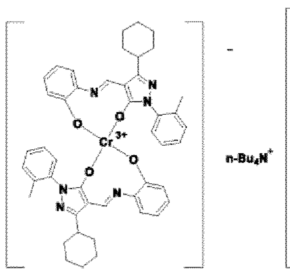
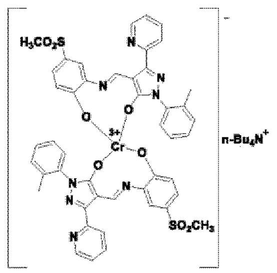

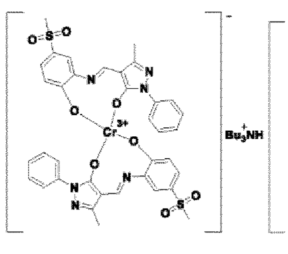
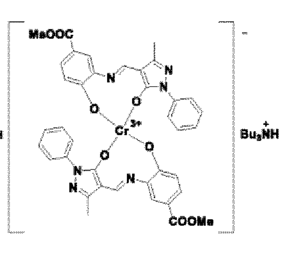
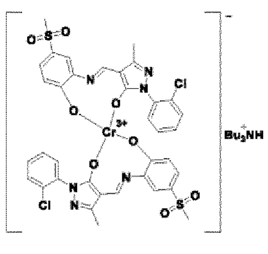
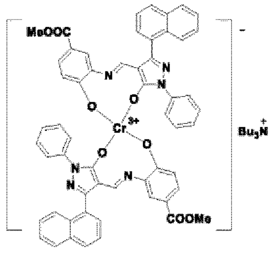

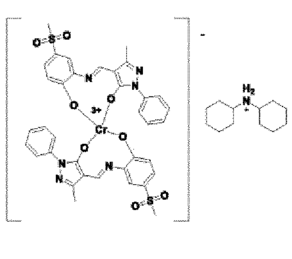
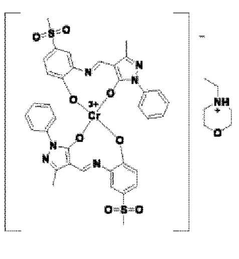
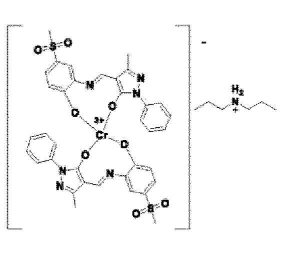
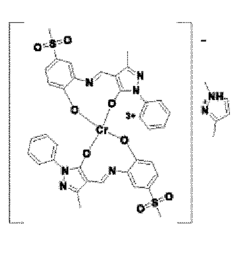

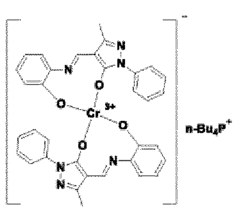
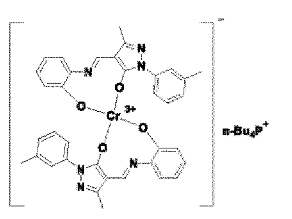
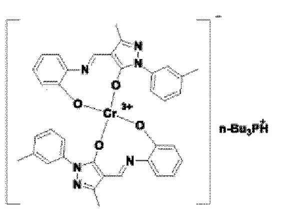
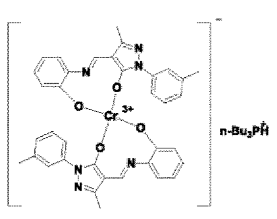

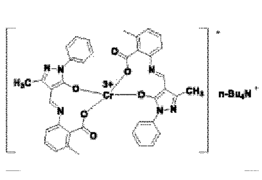
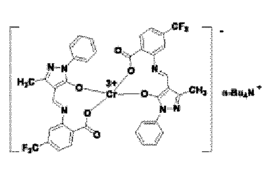
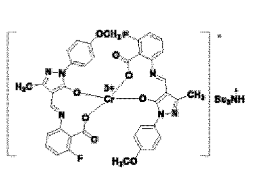
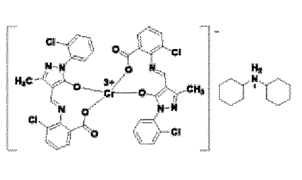

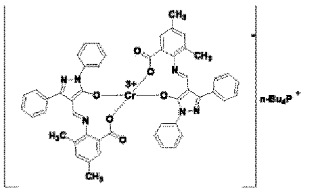 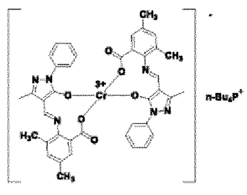 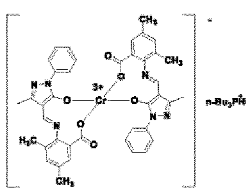 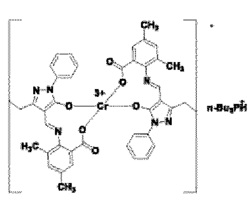

According to one embodiment of the present disclosure, the coordination compound described above is used as a light absorber.

Another embodiment of the present disclosure provides a light absorber including the coordination compound described above.

According to one embodiment of the present disclosure, the light absorber may further include a solvent.

As the solvent, materials known to enable formation of a light absorber from the coordination compound described above in the art relating to the present disclosure may be used without particular limit. For example, the solvent may be one or more types of compounds selected from the group consisting of esters, ethers, ketones, aromatic hydrocarbons and sulfoxides.

Examples of the ester-based solvent may include ethyl acetate, n-butyl acetate, isobutyl acetate, amyl formate, isoamyl acetate, isobutyl acetate, butyl propionate, isopropyl butyrate, ethyl butyrate, butyl butyrate, methyl lactate, ethyl lactate, gamma-butyrolactone, epsilon-caprolactone, delta-valerolactone, alkyl oxyacetate (example: methyl oxyacetate, ethyl oxyacetate, butyl oxyacetate (for example, methyl methoxyacetate, ethyl methoxyacetate, butyl methoxyacetate, methyl ethoxyacetate, ethyl ethoxyacetate or the like)), alkyl 3-oxypropionates (example: methyl 3-oxypropionate, ethyl 3-oxypropionate or the like (for example, methyl 3-methoxypropionate, ethyl 3-methoxypropionate, methyl 3-ethoxypropionate, ethyl 3-ethoxypropionate or the like)), alkyl 2-oxypropionates (example: methyl 2-oxypropionate, ethyl 2-oxypropionate, propyl 2-oxypropionate or the like (for example, methyl 2-methoxypropionate, ethyl 2-methoxypropionate, propyl 2-methoxypropionate, methyl 2-ethoxypropionate, ethyl 2-ethoxypropionate)), methyl 2-oxy-2-methylpropionate and ethyl 2-oxy-2-methylpropionate (for example, methyl 2-methoxy-2-methylpropionate, ethyl 2-ethoxy-2-methylpropionate or the like), methyl pyruvate, ethyl pyruvate, propyl pyruvate, methyl acetoacetate, ethyl acetoacetate, methyl 2-oxobutanoate, 2-ethyl 2-oxobutanoate and the like.

Examples of the ether-based solvent may include diethylene glycol dimethyl ether, tetrahydrofuran, ethylene glycol monomethyl ether, ethylene glycol monoethyl ether, methyl cellosolve acetate, ethyl cellosolve acetate, diethylene glycol monomethyl ether, diethylene glycol monoethyl ether, diethylene glycol monobutyl ether, propylene glycol monomethyl ether, propylene glycol monomethyl ether acetate, propylene glycol monoethyl ether acetate, propylene glycol monopropyl ether acetate and the like.

Examples of the ketone-based solvent may include methyl ethyl ketone, cyclohexanone, cyclopentanone, 2-heptanone, 3-heptanone, N-methyl-2-pyrrolidone and the like.

Examples of the aromatic hydrocarbon-based solvent may include toluene, xylene, anisole, limonene and the like.

Examples of the sulfoxide-based solvent may include dimethyl sulfoxide and the like.

The solvent is exemplified as above, however, the solvent is not limited to the solvents exemplified above, and any solvent capable of dissolving or dispersing the coordination compound represented by Chemical Formula A of present disclosure may be used. In addition, the solvent may be used either alone as one type, or as a mixture of two or more types of solvents.

According to one embodiment of the present disclosure, the coordination compound represented by Chemical Formula A has an organic metal complex structure, and the light absorber employing the same has an advantage of enhancing blue luminance transmittance or improving reflected color.

According to one embodiment of the present disclosure, the light absorber may absorb at least a portion of wavelengths selected from among 350 nm to 450 nm and more preferably 380 nm to 450 nm, however, the wavelength is not limited thereto.

One embodiment of the present disclosure provides an adhesive composition including the coordination compound described above. As one example, the adhesive composition is the light absorber described above. As another example, the adhesive composition includes the light absorber described above and other additives.

One embodiment of the present disclosure provides an adhesive composition including at least one selected from the group consisting of a binder resin, a crosslinking agent, a coupling agent, an antioxidant, an antistatic agent, a light stabilizer and a catalyst.

According to one embodiment of the present disclosure, the crosslinking agent induces a crosslinking reaction between an alkali-soluble polyimide resin or other additive components to increase heat resistance and chemical resistance of a produced film. Herein, compounds including a functional group such as an acrylic group or an isocyanate

group may be used as the crosslinking agent. In addition, the crosslinking agent may be, for example, a thermal crosslinking agent, and as such a thermal crosslinking agent, compounds including a thermally reactive functional group such as a methylol group or an epoxy group may be used. As a specific example, crosslinking agents generally used in the art such as DML-PC, DML-PEP, DML-OC, DML-OEP, DML-34X, DML-PTBP, DML-PCHP, DML-OCHP, DML-PFP, DML-PSBP, DML-POP, DML-MBOC, DML-MBPC,DML-MTrisPC, DML-BisOC-Z, DML-BisOCHP-Z, DML-BPC, DML-BisOC-P, DMOM-PC, DMOM-PTBP, DMOM-MBPC, TriML-P,TriML-35XL, TML-HQ, TML-BP, TML-pp-BPF, TML-BPE, TML-BPA, TML-BPAF, TML-BPAP, TMOM-BP, TMOM-BPE, TMOM-BPA, TMOM-BPAF, TMOM-BPAP, HML-TPPHBA, HML-TPHAP, HMOM-TPPHBA, HMOM-TPHAP (hereinbefore, trade names, manufactured by Honshu Chemical Industry Co., Ltd.), "NIKALAC" (registered trademark) MX-290, "NIKALAC" (registered trademark) MX-280, "NIKALAC" (registered trademark) MX-270, "NIKALAC" (registered trademark) MX-279, "NIKALAC" (registered trademark) MW-100LM, "NIKALAC" (registered trademark) MX-750LM (hereinbefore, trade names, manufactured by Sanwa Chemical Co., Ltd.) and T39M (Soken Chemical & Engineering Co., Ltd.) may be used.

According to one embodiment of the present disclosure, the coupling agent may be a silane-based coupling agent, but is not limited thereto, and those known in the art may be properly employed.

According to one embodiment of the present disclosure, the antioxidant may perform a role of preventing a chain reaction that generates radicals during polymer film formation. Herein, a phenol-based antioxidant or the like may be included as the antioxidant, and 2,2-thiobis(4-methyl-6-t-butylphenol), 2,6-g,t-butylphenol or the like, an antioxidant generally used in the art, may be used, however, the antioxidant is not limited thereto. According to a preferred embodiment of the present disclosure, Kinox-80 (Hannong Chemicals Inc.) may be used as the antioxidant, however, the antioxidant is not limited thereto.

According to one embodiment of the present disclosure, the antistatic agent is included in the adhesive composition to perform a role of providing antistatic performance, and known antistatic agents may all be used. Examples of the antistatic agent may include ionic compounds. Examples of the ionic compound may include metal salts or organic salts. Examples of the metal salt ionic compound may include alkali metal cations or alkaline-earth metal cations. Examples of the cation may include one, two or more types of a lithium ion ($Li^+$), a sodium ion ($Na^+$), a potassium ion ($K^+$), a rubidium ion ($Rb^+$), a cesium ion ($Cs^+$), a beryllium ion ($Be^{2+}$), a magnesium ion ($Mg^{2+}$), a calcium ion ($Ca^{2+}$), a strontium ion ($Sr^{2+}$) and a barium ion ($Ba^{2+}$), and for example, one, or more types of a lithium ion, a sodium ion, a potassium ion, a magnesium ion, a calcium ion and a barium ion may be used, or considering ion stability and mobility, a lithium ion may be used. Examples of the anion included in the metal salt may include $PF_6^-$, AsF-, $NO_2^-$, fluoride ($F^-$), chloride ($Cl^-$), bromide ($Br^-$), iodide ($I^-$), perchlorate ($ClO_4^-$), hydroxide ($OH^-$), carbonate ($CO_3^{2-}$), nitrate ($NO_3^-$), trifluoromethanesulfonate ($CF_3SO_3^-$), sulfonate ($SO_4^-$), hexafluorophosphate ($PF_6^-$), methylbenzenesulfonate ($CH_3(C_6H_4)SO_3^-$), p-toluenesulfonate ($CH_3C_6H_4SO_3^-$), tetraborate ($B_4O_7^{2-}$), carboxybenzenesulfonate ($COOH(C_6H_4)SO_3^-$), trifluoromethanesulfonate ($CF_3SO_2^-$), benzonate ($C_6H_5COO^-$), acetate ($CH_3COO^-$), trifluoroacetate ($CF_3COO^-$), tetrafluoroborate ($BF_4^-$), tetrabenzylborate ($B(C_6H_5)_4^-$), trispentafluoroethyl trifluorophosphate ($P(C_2F_5)_3F_3^-$) or the like. According to preferred one embodiment of the present disclosure, FC-4400 (3M Corporation) may be used as the antistatic agent, however, the antistatic agent is not limited thereto.

According to one embodiment of the present disclosure, the light stabilizer is a material that does not agglomerate even when the adhesive is left unattended under a high temperature condition, and thereby significantly improves storage stability of the adhesive composition by, without causing a phenomenon of increasing a concentration of the antistatic agent to describe below in the agglomerated cluster, preventing a problem of the bonding site being decomposed by heat and generating radicals, and known light stabilizers may be used. According to preferred one embodiment of the present disclosure, hindered amine compounds may be used as the light stabilizer, and specifically, Tinuvin 123 (BASF Corporation) may be used, however, the light stabilizer is not limited thereto.

According to one embodiment of the present disclosure, the catalyst is a material controlling a rate of the reaction preparing the adhesive composition according to one embodiment of the present disclosure. According to one embodiment of the present disclosure, dibutyltin dilaurate (Sigma-Aldrich) may be used as the catalyst, however, the catalyst is not limited thereto.

In one embodiment of the present disclosure, the coordination compound represented by Chemical Formula A is included in 0.1 parts by weight to 0.5 parts by weight based on 100 parts by weight of the total weight of the adhesive composition solid content.

Preferably, the coordination compound represented by Chemical Formula A is included in 0.2 parts by weight to 0.4 parts by weight based on 100 parts by weight of the total weight of the adhesive composition solid content. More preferably, the coordination compound represented by Chemical Formula A is included in 0.25 parts by weight to 0.35 parts by weight based on 100 parts by weight of the total weight of the adhesive composition solid content.

When the coordination compound represented by Chemical Formula A is included in the above-described content in the adhesive composition, excellent light resistance, heat resistance and moisture resistance are obtained. When the coordination compound represented by Chemical Formula A is included in less than 0.2 parts by weight, a sufficient effect of light resistance may not be expected, and including the coordination compound represented by

Chemical Formula A in greater than 0.4 parts by weight may cause a problem of precipitating the dye on the thin film.

In one embodiment of the present disclosure, the binder resin is included in 90 parts by weight to 99 parts by weight, the crosslinking agent is included in 0.05 parts by weight to 0.5 parts by weight, the coupling agent is included in 0.05 parts by weight to 0.5 parts by weight, the antioxidant is included in 0.2 parts by weight to 0.8 parts by weight, the antistatic agent is included in 0.5 parts by weight to 2.5 parts by weight, the light stabilizer is included in 1 parts by weight to 4 parts by weight and the catalyst is included in 0.001 parts by weight to 0.02 parts by weight, based on 100 parts by weight of the total weight of the adhesive composition solid content.

In one embodiment of the present disclosure, the binder resin is included in 93 parts by weight to 97 parts by weight based on 100 parts by weight of the total weight of the adhesive composition solid content.

In one embodiment of the present disclosure, the crosslinking agent is included in 0.3 parts by weight to 0.4 parts by weight based on 100 parts by weight of the total weight of the adhesive composition solid content.

In one embodiment of the present disclosure, the coupling agent is included in 0.1 parts by weight to 0.3 parts by weight based on 100 parts by weight of the total weight of the adhesive composition solid content.

In one embodiment of the present disclosure, the antioxidant is included in 0.4 parts by weight to 0.6 parts by weight based on 100 parts by weight of the total weight of the adhesive composition solid content.

In one embodiment of the present disclosure, the antistatic agent is included in 0.1 parts by weight to 2 parts by weight based on 100 parts by weight of the total weight of the adhesive composition solid content.

In one embodiment of the present disclosure, the light stabilizer is included in 1.5 parts by weight to 3.5 parts by weight based on 100 parts by weight of the total weight of the adhesive composition solid content.

In one embodiment of the present disclosure, the catalyst is included in 0.008 parts by weight to 0.02 parts by weight based on 100 parts by weight of the total weight of the adhesive composition solid content.

When the binder resin, the crosslinking agent, the coupling agent, the antioxidant, the antistatic agent, the light stabilizer or the catalyst is included in the above-described content in the adhesive composition, a film having a uniform thickness may be prepared using the adhesive composition.

According to one embodiment of the present disclosure, the adhesive composition may further include a solvent. As the solvent, the solvent described above in the light absorber may be used.

The solvent is not limited to the solvents exemplified above, and any solvent capable of dissolving or dispersing the coordination compound represented by Chemical Formula A of present disclosure may be used.

According to preferred one embodiment of the present disclosure, the solvent included in the adhesive composition is ethyl acetate or methyl ethyl ketone.

In addition, the solvent may be used either alone as one type, or as a mixture of two or more types of solvents. For example, according to one embodiment of the present disclosure, the solvent included in the adhesive composition is a mixed solution of ethyl acetate or methyl ethyl ketone. Herein, the solvents of the mixed solution may be mixed and used in a ratio of 1:3 to 3:1, however, this is just an example, and the mixing ratio is not limited to the example.

According to preferred one embodiment of the present disclosure, methyl ethyl ketone (MEK) may be used as the solvent, however, the solvent is not limited thereto.

According to one embodiment of the present disclosure, the solvent may be included in 10 parts by weight to 50 parts by weight or 20 parts by weight to 40 parts by weight based on 100 parts by weight of the total weight of the adhesive composition. According to preferred one embodiment of the present disclosure, the solvent may be included in 20 parts by weight to 30 parts by weight based on the total parts by weight of the adhesive composition, however, the content is not limited thereto.

When the solvent is included in the adhesive composition in the above-described range, there is no concern for precipitation of a dye or the like, and performance of the adhesive film including the adhesive composition may be readily identified.

In one embodiment of the present disclosure, the adhesive composition may further include a binder resin.

As the binder resin, known materials commonly used in the art may be used as long as it is a material providing adhesiveness and usable in a film for a display. Examples of the binder resin may include an acryl-based polymer, an acrylate-based copolymer, a silicone-based polymer, polyester, polyurethane, polyether, an epoxy resin and the like, but are not limited thereto.

According to one embodiment of the present disclosure, the binder resin may be an acrylate-based resin. The acrylate-based resin may be an acrylate-based polymer or an acrylate-based copolymer. Examples of the acrylate-based copolymer may include butyl acrylate/hydroxyethyl acrylate, but are not limited thereto.

According to a preferred embodiment of the present disclosure, AD-701 of LG Chem. may be used as the binder resin, however, the binder resin is not limited thereto.

According to one embodiment of the present disclosure, the binder resin may be included in 93 parts by weight to 97 parts by weight; or 94 parts by weight to 96 parts by weight based on 100 parts by weight of the total weight of the adhesive composition solid content. According to preferred one embodiment of the present disclosure, the binder resin may be included in 95 parts by weight to 96 parts by weight based on 100 parts by weight of the total weight of the

adhesive composition solid content.

When the binder resin is included in the adhesive composition in the above-mentioned range, adhesiveness of the adhesive film including the adhesive composition is maintained, and an adhesive film having a uniform thickness may be prepared from the adhesive composition.

In the present disclosure, the 'adhesive composition solid content' means excluding the solvent from the adhesive composition including the coordination compound represented by Chemical Formula A.

According to another embodiment of the present disclosure, the coordination compound of Chemical Formula A may be used together with other types of dyes. As the dye that may be used together with the coordination compound of Chemical Formula A, dyes commonly used in the art may be used. As a specific example of the dye that may be used together with the coordination compound of Chemical Formula A, one or more types may be selected from the group consisting of metal-complex-based compounds; azo-based compounds; metal azo-based compounds; quinophthalone-based compounds; isoindoline-based compounds; methine-based compounds; phthalocyanine-based compounds; metal phthalocyanine-based compounds; porphyrin-based compounds; metal porphyrin-based compounds; tetra-azaporphyrin-based compounds; metal tetra-azaporphyrin-based compounds; cyanine-based compounds; xanthene-based compounds; metal dipyrromethane-based compounds; boron dipyrromethane-based compounds; metal dipyrromethane-based compounds; anthraquinone-based compounds; diketopyrrolopyrrole-based compounds; triarylmethane-based compounds; and perylene-based compounds. However, the dye is not limited thereto.

One embodiment of the present disclosure provides an adhesive film including the light absorber.

According to one embodiment of the present disclosure, the adhesive film including the light absorber may include the light absorber as it is.

According to another embodiment of the present disclosure, the adhesive film including the light absorber may include the light absorber in a state having the solvent removed by drying.

Another embodiment of the present disclosure provides an adhesive film including the adhesive composition.

According to one embodiment of the present disclosure, the adhesive film may be used as an optical film used in a display, an electronic device or the like. The optical film may be used as a retardation film, an anti-reflection film, an anti-glare film, an ultraviolet absorption film, an infrared absorption film, an optical compensation film, a brightness enhancement film or the like.

According to one embodiment of the present disclosure, the adhesive film including the adhesive composition may include the adhesive composition as it is.

According to another embodiment of the present disclosure, the adhesive film including the adhesive composition may include the adhesive composition in a state having the solvent removed by drying.

According to still another embodiment of the present disclosure, the adhesive film including the adhesive composition may include a cured material of the adhesive composition.

In one embodiment of the present disclosure, a maximum absorption wavelength of the adhesive film is a wavelength of at least one of 380 nm to 450 nm.

In still another embodiment of the present disclosure, a maximum absorption wavelength of the adhesive film is a wavelength of at least one of 380 nm to 440 nm.

When the adhesive film has a wavelength of at least one of 380 nm to 450 nm as a maximum absorption wavelength, a decrease in the blue luminance may be minimized by absorbing a longer wavelength region compared to an existing blue light blocking film, and efficiency may be maximized.

One embodiment of the present disclosure provides an optical film including the light absorber.

According to one embodiment of the present disclosure, the optical film includes an adhesive film and a binder resin film.

According to one embodiment of the present disclosure, the adhesive film includes the light absorber.

According to one embodiment of the present disclosure, the optical film includes an adhesive film and a binder resin film, and the adhesive film includes the light absorber.

Another embodiment of the present disclosure provides an optical film including the adhesive film and other films.

Examples of the other films may include a release film, an anti-reflection film, a binder resin film and the like.

Specifically, one embodiment of the present disclosure provides an optical film including an adhesive film and a binder resin film.

The optical film may further include a base as necessary.

FIG. 2 illustrates an optical film according to one embodiment of the present disclosure in which a base (1); an adhesive film (2); and a binder resin film (3) are consecutively laminated.

In the present disclosure, transmittance means transmittance for light.

In the present disclosure, a change in the transmittance for light is obtained by, while employing transmittance of an optical film that does not include the coordination compound represented by Chemical Formula A as 100%, measuring transmittance of the optical film including the coordination compound represented by Chemical Formula A, and measuring the changed value.

Specifically, the changed value may be calculated by the following calculation formula.

ΔT(%)={(transmittance measured immediately after preparing the optical film-transmittance remeasured after exposing the optical film to each condition of light resistance, heat resistance and moisture resistance)/transmittance measured immediately after preparing the optical film}X100

According to one embodiment of the present disclosure, the adhesive film is used in a display, an electronic device or the like instead of a polarizing plate, which more effectively reduces a change in the transmittance for each wavelength compared to an optical film including a polarizing plate. In addition, the adhesive film includes an adhesive composition including the coordination compound of Chemical Formula A, which more effectively reduces a change in the transmittance for each wavelength compared to an optical film including other compositions. Accordingly, color tones of the emitted color may be flexibly adjusted.

According to one embodiment of the present disclosure, the adhesive film including the adhesive composition may have a form in which an adhesive composition is laminated and/or coated on one surface of a base.

According to one embodiment of the present disclosure, the base may be selected from the group consisting of PET (polyethylene terephthalate), polyester, PC (polycarbonate), PI (polyimide), PEN (polyethylene naphthalate), PEEK (polyether ether ketone), PAR (polyarylate), PCO (polycyclicolefin), polynorbornene, PES (polyethersulphone) and COP (cycloolefin polymer).

In addition, the base is preferably transparent. The base being transparent referred herein means that light transmittance of visible light having a wavelength range of 400 nm to 700 nm is 80% or greater. When the base has light transmittance in the above-mentioned range, the laminated adhesive film may be thin filmed.

According to one embodiment of the present disclosure, the adhesive film including the adhesive composition has a thickness of 10 μm to 40 μm; 15 μm to 30 μm; or 20 μm to 25 μm. According to preferred one embodiment of the present disclosure, the adhesive film including the adhesive composition has a thickness of 22 μm to 23 μm.

According to one embodiment of the present disclosure, the adhesive film may be prepared by coating the adhesive composition on the base using a bar coater. As a specific example, FIG. 1 illustrates a case of the base (1) and the adhesive film (2) according to one embodiment of the present disclosure being consecutively laminated.

According to one embodiment of the present disclosure, the release film may be included on another surface other than the one surface of the base on which the adhesive composition is laminated and/or coated.

As the release film, a hydrophobic film may be used. The release film refers to, as a film for protecting a very thin adhesive film, a transparent layer attached to one surface of the adhesive film, and films having excellent mechanical strength, thermal stability, moisture shielding properties, isotropy and the like may be used. For example, as the release film, acetate-based such as triacetyl cellulose (TAC), polyester-based, polyethersulphone-based, polycarbonate-based, polyamide-based, polyimide-based, polyolefin-based, cycloolefin-based, polyurethane-based and acryl-based resin films, and the like may be used, and commercially available silicone-treated release films may be used. However, the release film is not limited to these examples.

According to one embodiment of the present disclosure, the anti-reflection film performs a role of improving reflected color sense by adjusting the degree of light reflection generated from an external light source.

According to one embodiment of the present disclosure, the anti-reflection film has a lowest reflection wavelength of 550 nm or less; or 530 nm or less. According to preferred one embodiment of the present disclosure, the anti-reflection film has a lowest reflection wavelength of 500 nm or less.

A material of the anti-reflection film may be properly selected to satisfy properties of the lowest reflection wavelength. For example, the anti-reflection film may include a low refractive index layer. For example, the lowest reflection wavelength of the anti-reflection film tends to move to a longer wavelength as a thickness of the low refractive index layer increases, and tends to move to a shorter wavelength as a thickness of the low refractive index layer decreases. For example, the lowest reflectivity of the anti-reflection film tends to decrease as a refractive index of a low refractive material decreases.

The low refractive index layer may include a low refractive material. In one example, the low refractive material may be a low refractive inorganic particle. In another example, the low refractive inorganic particle may be a silica-based

particle. Examples of the silica-based particle may include hollow silica, mesoporous silica and the like, but are not limited thereto. In another example, magnesium fluoride ($MgF_2$) may be used as the low refractive inorganic particle.

According to one embodiment of the present disclosure, the binder resin film may include a TAC (cellulose triacetate) film or the like, but is not limited thereto, and known binder resin films commonly used in the art may be used.

The binder resin film has a thickness of 20 μm to 60 μm; 30 μm to 50 μm; or 35 μm to 45 μm.

In one embodiment of the present disclosure, the adhesive film may be provided on the base, and the binder resin film may be further provided on a surface opposite to the surface on which the adhesive film and the base are in contact with each other.

One embodiment of the present disclosure provides an electronic device including the light absorber described above.

One embodiment of the present disclosure provides an electronic device including the adhesive film.

According to one embodiment of the present disclosure, the electronic device includes an adhesive film, and the adhesive film includes the light absorber.

The electronic device may include all of an interlayer insulating film of a semiconductor device, a color filter, a black matrix, an overcoat, a column spacer, a passivation film, a buffer coat film, an insulating film for a multilayer print substrate, a cover coat of a flexible copper clad plate, a buffer coat film, an insulating film solder resist film for a multilayer print substrate, an insulating film of an OLED, a protective film of a thin film transistor of a liquid crystal display apparatus, an electrode protective film of an organic EL device and a semiconductor protective film, an OLED insulating film, an LCD insulating film, a semiconductor insulating film, a display apparatus and the like, but is not limited thereto.

One embodiment of the present disclosure provides a display apparatus including the light absorber.

Another embodiment of the present disclosure provides a display apparatus including the adhesive film.

One embodiment of the present disclosure provides an organic light emitting display apparatus including the light absorber.

Another embodiment of the present disclosure provides an organic light emitting display apparatus including the adhesive film.

One embodiment of the present disclosure provides an organic light emitting display apparatus including an adhesive film including the adhesive composition described above; a substrate provided on one surface of the adhesive film; and an organic light emitting layer provided on a surface opposite to the surface in contact with the adhesive film of the substrate.

Mode for Carrying Out the Invention

Hereinafter, the present disclosure will be described in detail with reference to examples in order to specifically describe the present disclosure. However, the examples according to the present disclosure may be modified to various different forms, and the scope of the present disclosure is not to be construed as being limited to the examples described below. Examples of the present disclosure are provided in order to more fully describe the present disclosure to those having average knowledge in the art.

**<Synthesis Example>**

The coordination compound represented by Chemical Formula A may be prepared using, for example, a preparation method as the following Reaction Formula 1. The preparation method may be more specified in preparation examples to describe below.

[Reaction Formula 1]

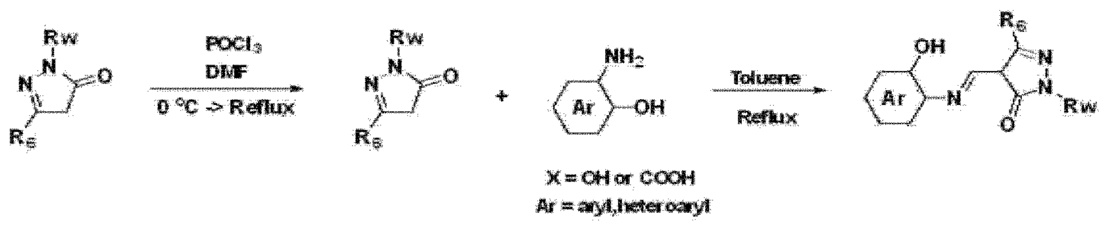


A B C D

1) Synthesis of Compound B

Phosphoryl chloride (1.2 equivalents) was diluted in an excess dimethylformamide (DMF) solvent, and stirred for 30 minutes under nitrogen while maintaining a temperature at 0°C. When the temperature was sufficiently stabilized, Compound A (1 equivalent) was slowly added thereto, and after raising the reaction temperature to 80°C, the mixture was sufficiently reacted under nitrogen. After identifying the completion of the reaction through thin layer chromatography (TLC), the reaction temperature was lowered to room temperature, and the result was introduced to prepared ice water and sufficiently stirred. After that, a produced solid compound was secured through vacuum filtration, and extracted using chloroform and water. Anhydrous magnesium sulfate was introduced to the extracted organic layer to remove water, and the result was concentrated and recrystallized with chloroform and ethanol to obtain a product.

2) Synthesis of Compound D

Secured Compound B (1 equivalent) was diluted in a toluene solution together with synthesized or commercially available Compound C (1.2 equivalents), and the mixture was stirred under nitrogen while heating to a temperature of 120°C to proceed a reaction. After the reaction was completed, the temperature was lowered to room temperature, and a formed solid product was vacuum filtered while sufficiently washing with ethanol and hexane to obtain a product. The solvent was sufficiently removed from Secured Compound D through oven drying.

**Preparation Example: Preparation of Compound**

**Preparation Example 1: Synthesis of Compound P1**

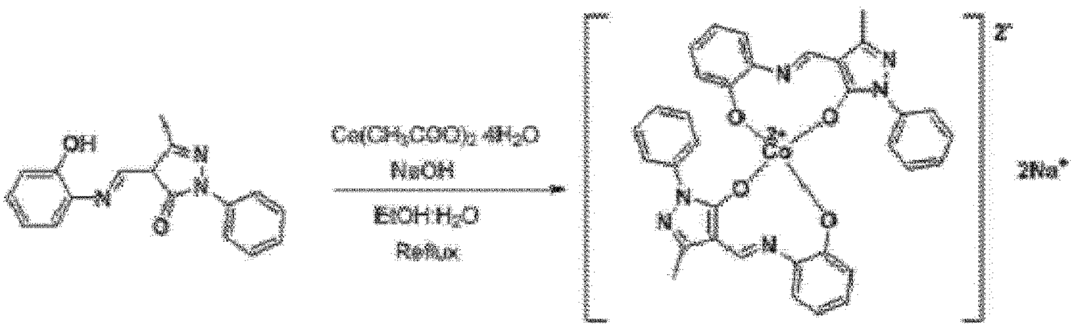


D1-a P1

Compound D1-a (100 mg, 1 equivalent) synthesized using the method of Reaction Formula 1 was diluted in ethanol (5 mL), and stirred while heating under nitrogen. After that, cobalt acetate tetrahydrate (42.5 mg, 0.5 equivalents) dissolved in water (5 mL) was added thereto, and sodium hydroxide (10 mg, 2 equivalents) was further introduced thereto to proceed a reaction. After the reaction was finished, the result was concentrated, and extracted using chloroform and water. From the organic layer secured through extraction, water was removed using anhydrous magnesium sulfate, and the result was concentrated through vacuum distillation. The concentrated product was recrystallized using chloroform and ethanol to secure Compound P1 (86 mg, yield: 72%).

HR LC/MS/MS m/z calculated for $C_{34}H_{26}C_ON_6Na_2O4$ (M+) : 687.1143; found: 687.1143

**Preparation Example 2: Synthesis of Compound P2**

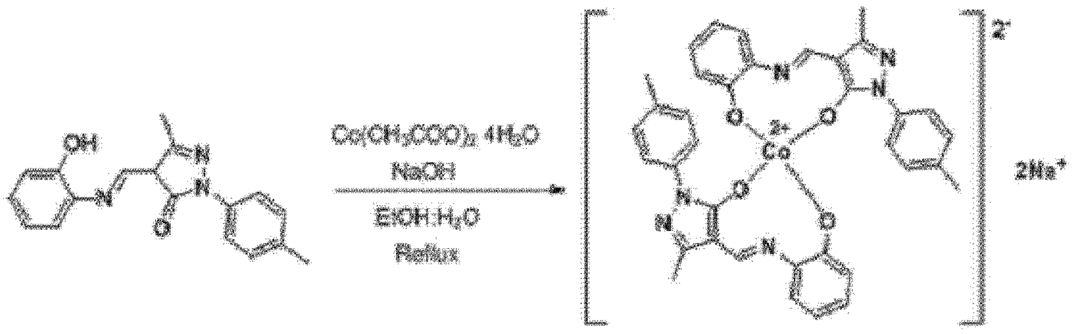


D1-b P2

Compound P2 (156 mg, yield: 67%) was secured by conducting a reaction in the same manner as in Preparation Example 1 except that D1-b (200 mg, 1 equivalent) was used instead of D1-a.

HR LC/MS/MS m/z calculated for $C_{36}H_{30}CoN_6Na_2O_4$ (M+) : 715.1456; found: 715.1455

**Preparation Example 3: Synthesis of Compound P3**

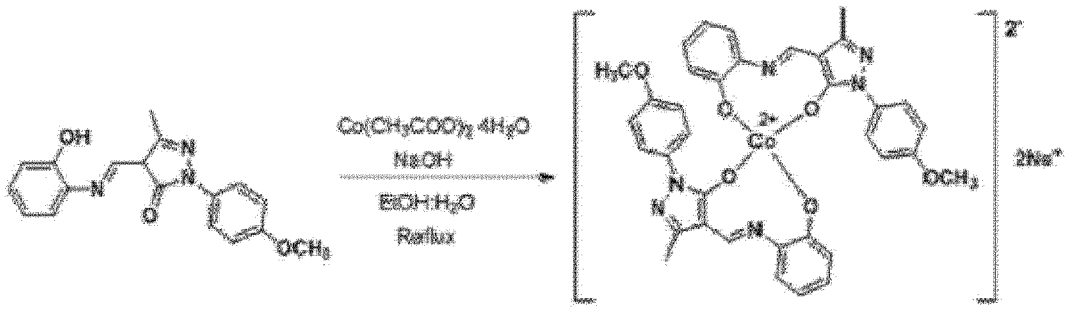


D1-c P3

Compound P3 (100 mg, yield: 58%) was secured by conducting a reaction in the same manner as in Preparation Example 1 except that D1-c (150 mg, 1 equivalent) was used instead of D1-a.

HR LC/MS/MS m/z calculated for $C_{36}H_{30}CoN_6Na_2O_6$ (M+) : 747.1354; found: 747.1354

**Preparation Example 4: Synthesis of Compound P4**

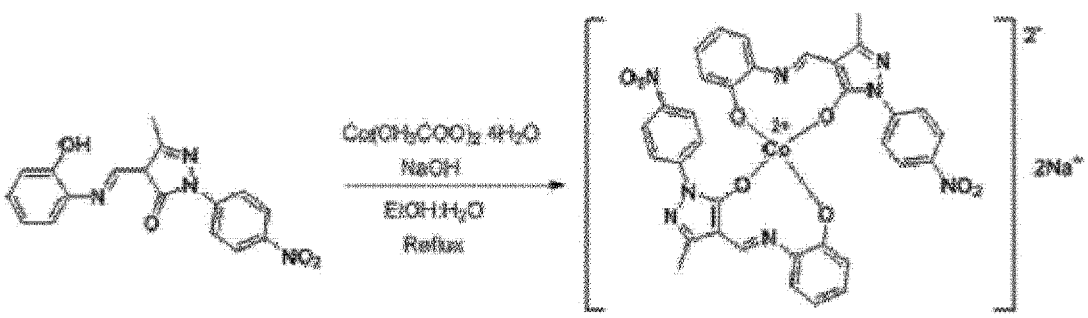


D1-d P4

Compound P4 (122 mg, yield: 71%) was secured by conducting a reaction in the same manner as in Preparation

Example 1 except that D1-d (150 mg, 1 equivalent) was used instead of D1-a.

HR LC/MS/MS m/z calculated for $C_{34}H_{24}C_{O}N_{B}Na_{2}O_{8}$ (M+) : 777.0844; found: 777.0844

**Preparation Example 5: Synthesis of Compound P5**

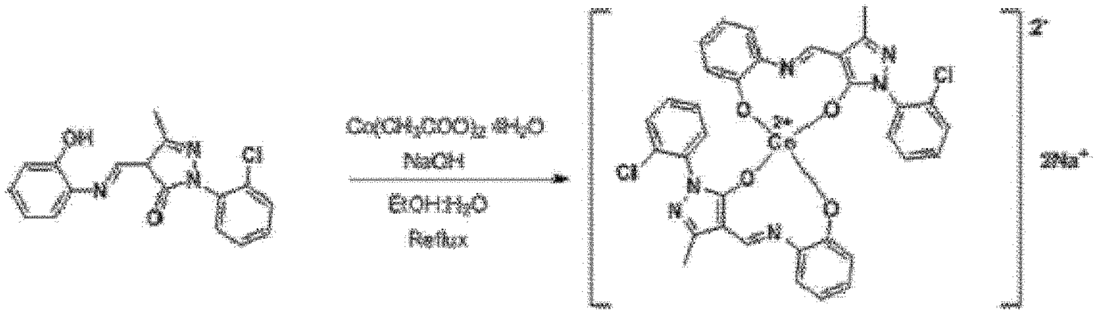


D1-e P5

Compound P5 (103 mg, yield: 45%) was secured by conducting a reaction in the same manner as in Preparation Example 1 except that D1-e (200 mg, 1 equivalent) was used instead of D1-a.

HR LC/MS/MS m/z calculated for $C_{34}H_{24}Cl_2CoN_6Na_2O_4$ (M+) : 755.0358; found: 755.0358

**Preparation Example 6: Synthesis of Compound P6**

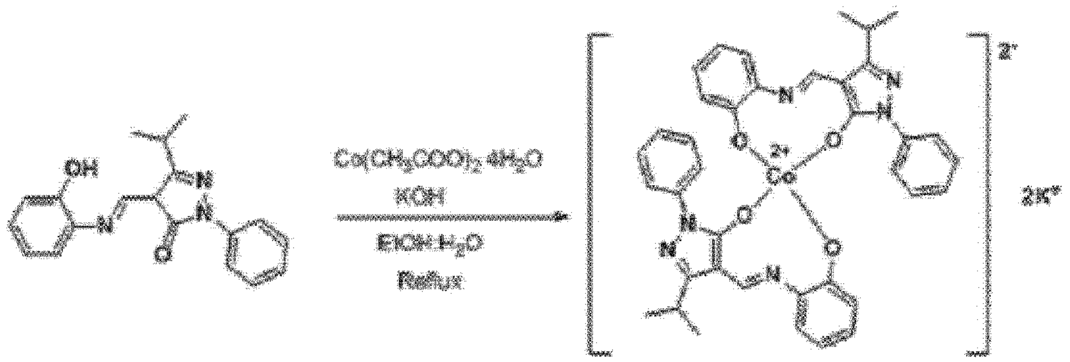


D2-a P6

Compound P6 (142 mg, yield: 59%) was secured by conducting a reaction in the same manner as in Preparation Example 1 except that D2-a (200 mg, 1 equivalent) was used instead of D1-a, and potassium hydroxide (69.8 mg, 2 equivalents) was used instead of sodium hydroxide.

HR LC/MS/MS m/z calculated for $C_{38}H_{34}CoK_2N_6O_4$ (M+) : 775.1248; found: 775.1248

**Preparation Example 7: Synthesis of Compound P7**

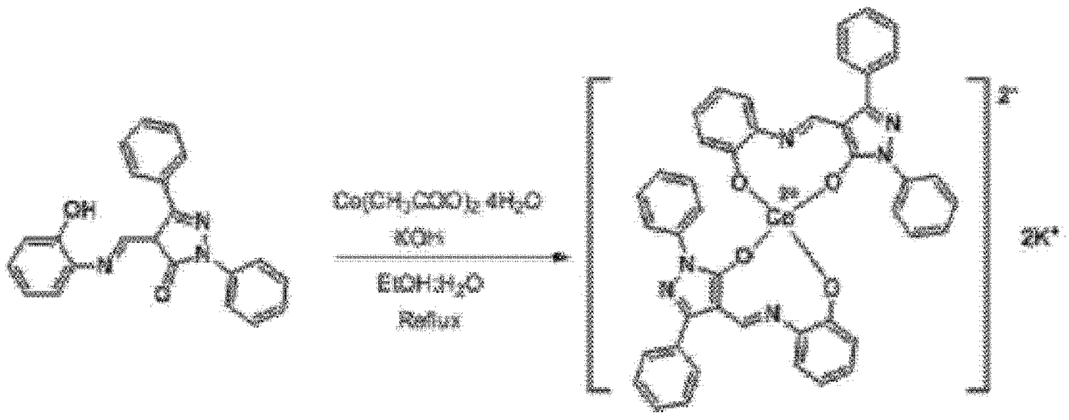


D2-b P7

Compound P7 (171 mg, yield: 72%) was secured by conducting a reaction in the same manner as in Preparation Example 1 except that D2-b (200 mg, 1 equivalent) was used instead of D1-a, and potassium hydroxide (69.8 mg, 2 equivalents) was used instead of sodium hydroxide.

HR LC/MS/MS m/z calculated for $C_{44}H_{30}CoK_2N_6O_4$ (M+) : 843.8918; found: 843.8918

**Preparation Example 8: Synthesis of Compound P8**

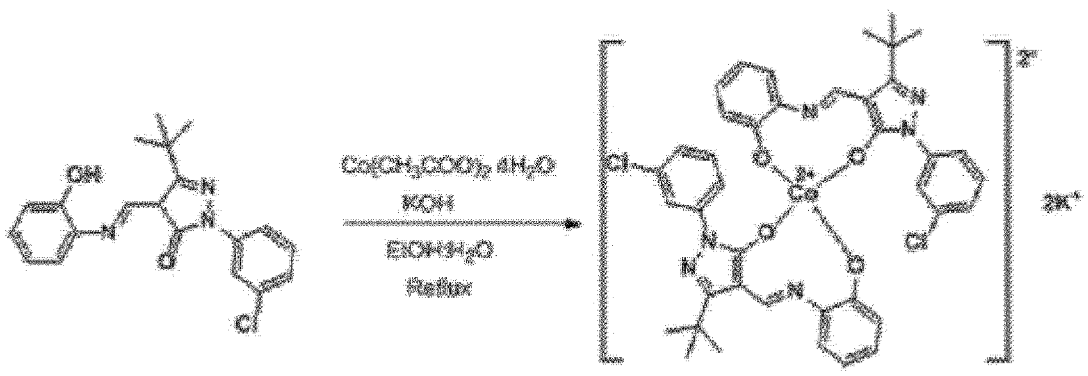


D2-c P8

Compound P8 (146 mg, yield: 62%) was secured by conducting a reaction in the same manner as in Preparation Example 1 except that D2-c (200 mg, 1 equivalent) was used instead of D1-a, and potassium hydroxide (69.8 mg, 2 equivalents) was used instead of sodium hydroxide.

HR LC/MS/MS m/z calculated for $C_{40}H_{36}Cl_2CoK_2N_6O_4$ (M+): 871.0781; found: 871.0781

**Preparation Example 9: Synthesis of Compound P9**

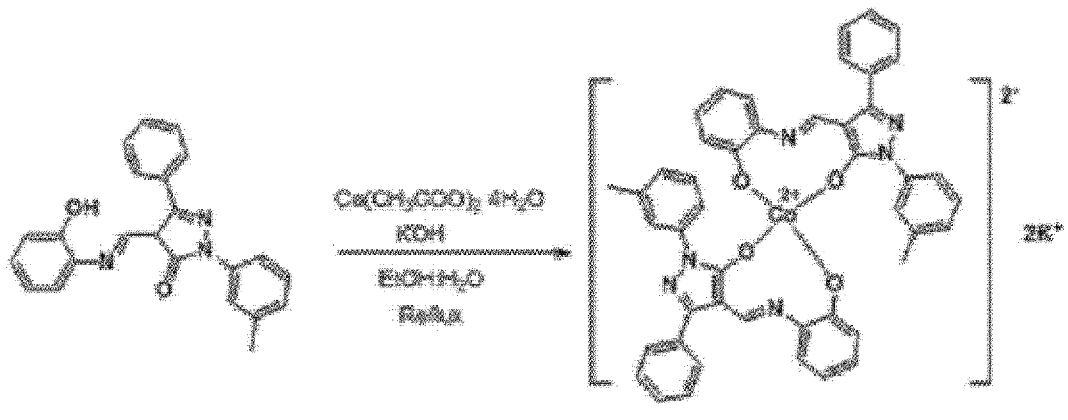


D2-d P9

Compound P9 (137 mg, yield: 58%) was secured by conducting a reaction in the same manner as in Preparation Example 1 except that D2-d (200 mg, 1 equivalent) was used instead of D1-a, and potassium hydroxide (69.8 mg, 2 equivalents) was used instead of sodium hydroxide.

HR LC/MS/MS m/z calculated for $C_{46}H_{34}CoK_2N_6O_4$ (M+) : 871.1248; found: 871.1248

**Preparation Example 10: Synthesis of Compound P10**

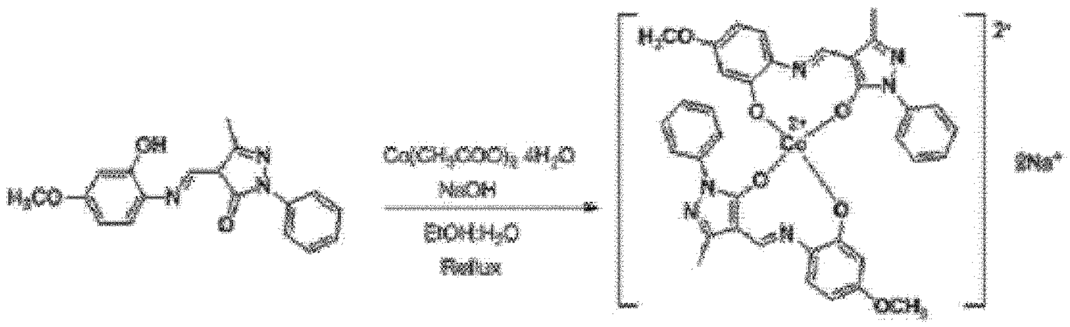


D3-a P10

Compound P10 (146 mg, yield: 42%) was secured by conducting a reaction in the same manner as in Preparation Example 1 except that D3-a (300 mg, 1 equivalent) was used instead of D1-a.

HR LC/MS/MS m/z calculated for $C_{36}H_{30}CoN_6Na_2O_6$ (M+) : 747.5847; found: 747.5846

**Preparation Example 11: Synthesis of Compound P11**

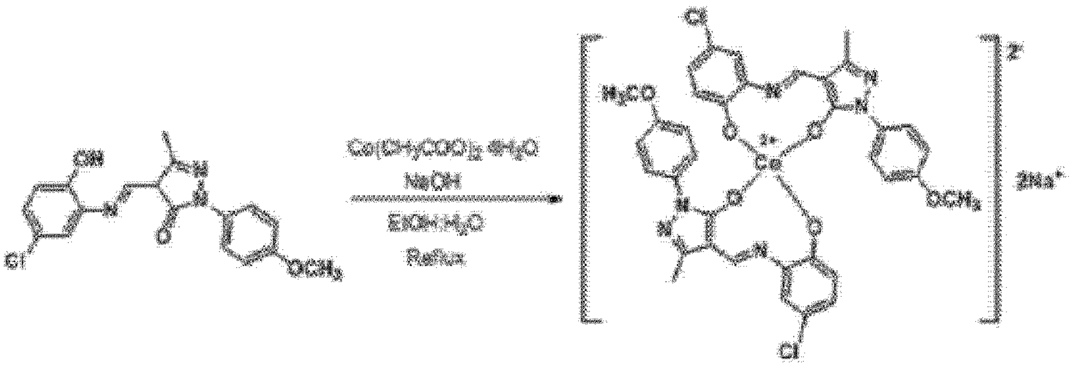


D3-b P11

Compound P11 (110 mg, yield: 48%) was secured by conducting a reaction in the same manner as in Preparation Example 1 except that D3-b (200 mg, 1 equivalent) was used instead of D1-a.

HR LC/MS/MS m/z calculated for $C_{36}H_{28}Cl_2CoN_6Na_2O_6$ (M+) : 815.4687; found: 815.4687

## Preparation Example 12: Synthesis of Compound P12

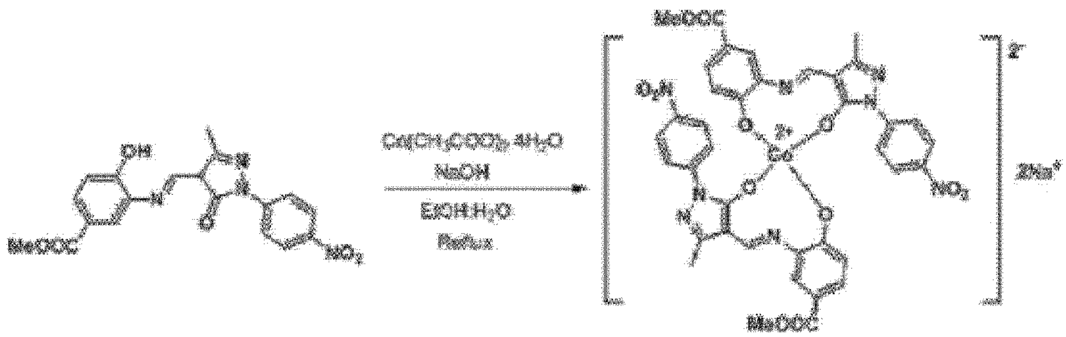


D3-c P12

Compound P12 (189 mg, yield: 67%) was secured by conducting a reaction in the same manner as in Preparation Example 1 except that D3-c (250 mg, 1 equivalent) was used instead of D1-a.

HR LC/MS/MS m/z calculated for $C_{38}H_{28}CoN_8Na_2O_{12}$ (M+) : 893.09542; found: 893.0954

## Preparation Example 13: Synthesis of Compound P13

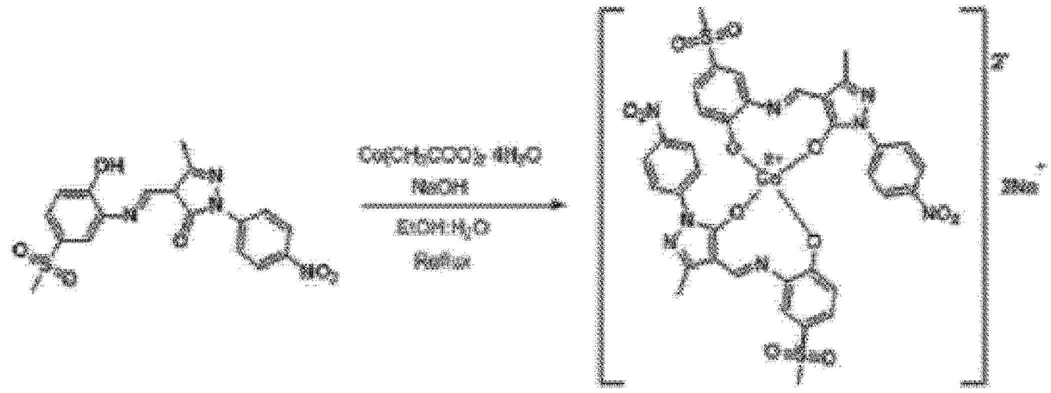


D3-d P13

Compound P13 (117 mg, yield: 52%) was secured by conducting a reaction in the same manner as in Preparation Example 1 except that D3-d (200 mg, 1 equivalent) was used instead of D1-a.

HR LC/MS/MS m/z calculated for $C_{36}H_{28}CoN_8Na_2O_{12}S_2$ (M+) : 933.0395; found: 933.0395

## Preparation Example 14: Synthesis of Compound P14

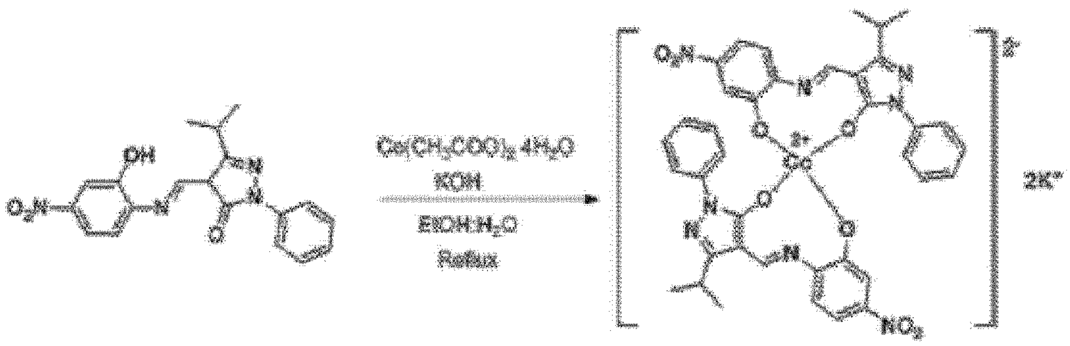


D3-e P14

Compound P14 (191 mg, yield: 81%) was secured by conducting a reaction in the same manner as in Preparation Example 1 except that D3-e (200 mg, 1 equivalent) was used instead of D1-a, and potassium hydroxide (69.8 mg, 2 equivalents) was used instead of sodium hydroxide.

HR LC/MS/MS m/z calculated for $C_{38}H_{32}CoK_2N_8O_8$ (M+) : 865.0949; found: 865.0949

## Preparation Example 15: Synthesis of Compound P15

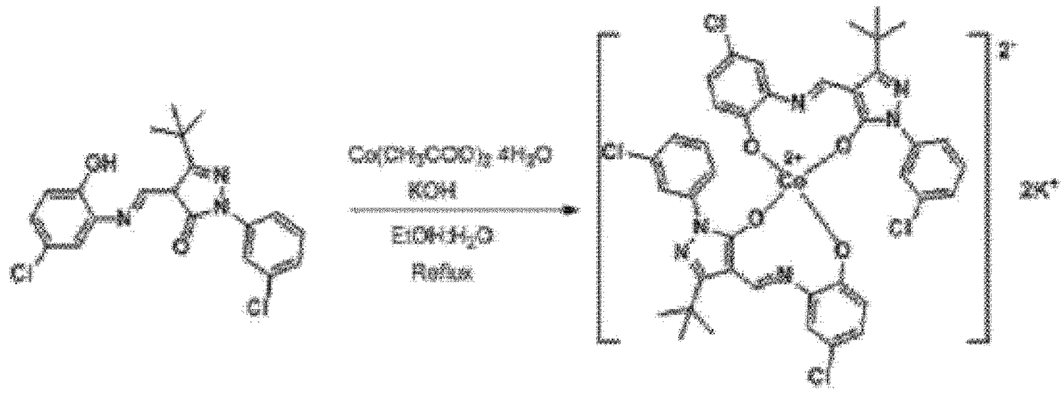


D3-f P15

Compound P15 (258 mg, yield: 74%) was secured by conducting a reaction in the same manner as in Preparation Example 1 except that D3-f (300 mg, 1 equivalent) was used instead of D1-a, and potassium hydroxide (69.8 mg, 2 equivalents) was used instead of sodium hydroxide.

HR LC/MS/MS m/z calculated for $C_{40}H_{34}Cl_4CoK_2N_6O_4$ (M+) : 940.9972; found: 940.9972

**Preparation Example 16: Synthesis of Compound P16**

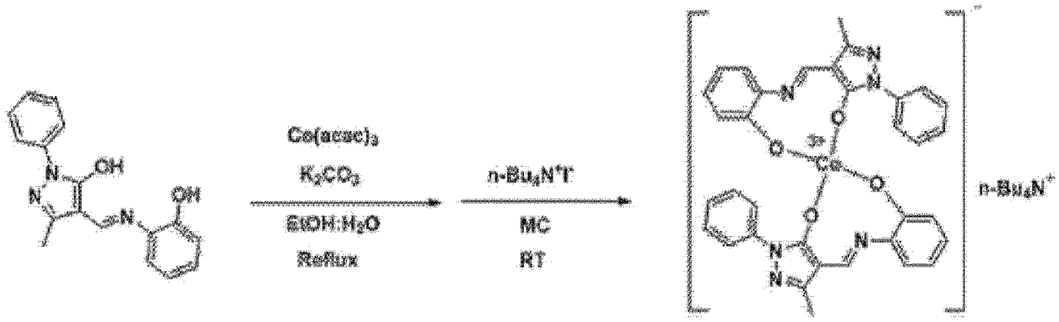


D1-a P16

Compound D1-a (500 mg, 1 equivalent) synthesized using the method of Reaction Formula 1 was diluted in ethanol (10 mL), and after adding potassium carbonate (471 mg, 2 equivalents) dissolved in water (10 mL) thereto, the mixture was stirred while heating under nitrogen. After that, tris(acetylacetonato)cobalt(III) (0.304 g, 0.5 equivalents) was added thereto, and the reaction proceeded until the reactant D1-a was not visible on thin layer chromatography (TLC). After concentrating the solvent from the completed reaction material, a dichloromethane solvent (20 mL) was added thereto, then tetra-n-butylammonium iodine (0.378 g, 0.6 equivalents) was introduced thereto, and the result was stirred at room temperature. After the reaction was completed, the result was extracted by adding water thereto. From the organic layer secured therethrough, water was removed using anhydrous magnesium sulfate, and the result was concentrated through vacuum distillation. The concentrated product was diluted in ethanol, and then water was added thereto to secure Compound P16 (407 mg, yield: 54%).

HR LC/MS/MS m/z calculated for $C_{50}H_{62}CoN_7O_4$ (M+): 883.4195; found: 883.4194

**Preparation Example 17: Synthesis of Compound P17**

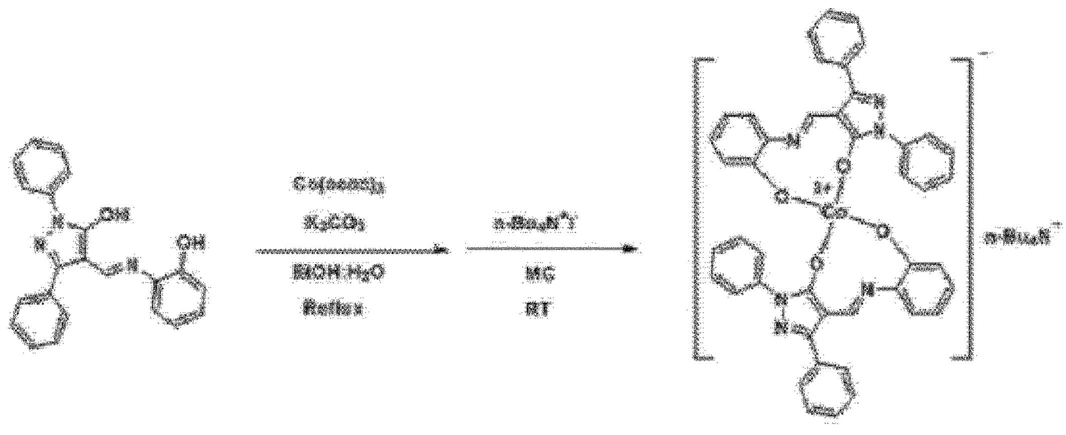


D2-b

P17

Compound P17 (440 mg, yield: 62%) was secured by conducting a reaction in the same manner as in Preparation Example 16 except that D2-b (500 mg, 1 equivalent) was used instead of D1-a.

HR LC/MS/MS m/z calculated for $C_{60}H_{66}CoN_7O_4$ (M+): 1007.4508; found: 1007.4508

## Preparation Example 18: Synthesis of Compound P18

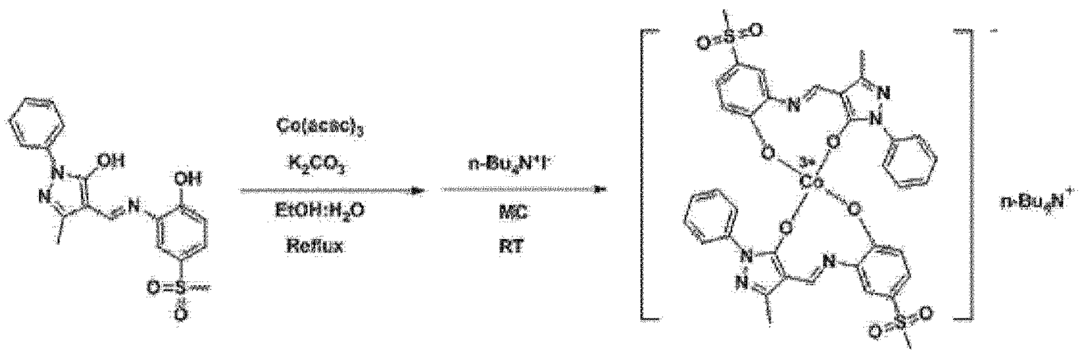


D3-g

P18

Compound P18 (336 mg, yield: 48%) was secured by conducting a reaction in the same manner as in Preparation Example 16 except that D3-g (500 mg, 1 equivalent) was used instead of D1-a.

HR LC/MS/MS m/z calculated for $C_{52}H_{66}CoN_7O_8S_2$ (M+) : 1039.3746; found: 1039.3746

## Preparation Example 19: Synthesis of Compound P19

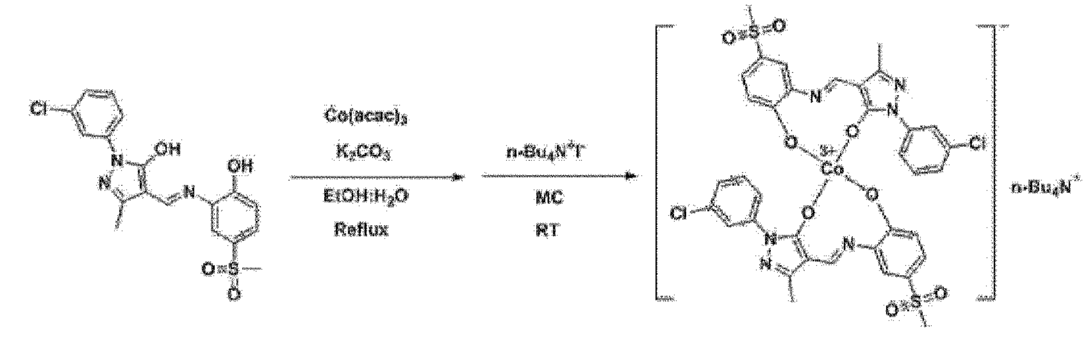


D3-h P19

Compound P19 (376 mg, yield: 55%) was secured by conducting a reaction in the same manner as in Preparation Example 16 except that D3-h (500 mg, 1 equivalent) was used instead of D1-a.

HR LC/MS/MS m/z calculated for $C_{52}H_{64}Cl_2CoN_7O_8S_2$ (M+) : 1107.2967; found: 1107.2967

## Preparation Example 20: Synthesis of Compound P20

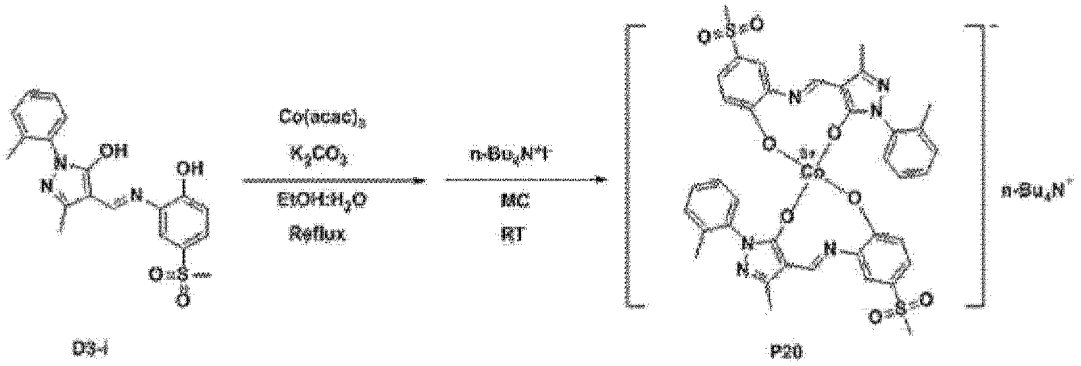


D3-i P20

Compound P20 (270 mg, yield: 39%) was secured by conducting a reaction in the same manner as in Preparation Example 16 except that D3-i (500 mg, 1 equivalent) was used instead of D1-a.

HR LC/MS/MS m/z calculated for $C_{54}H_{70}CoN_7O_8S_2$ (M+) : 1067.4059; found: 1067.4058

## Preparation Example 21: Synthesis of Compound P21

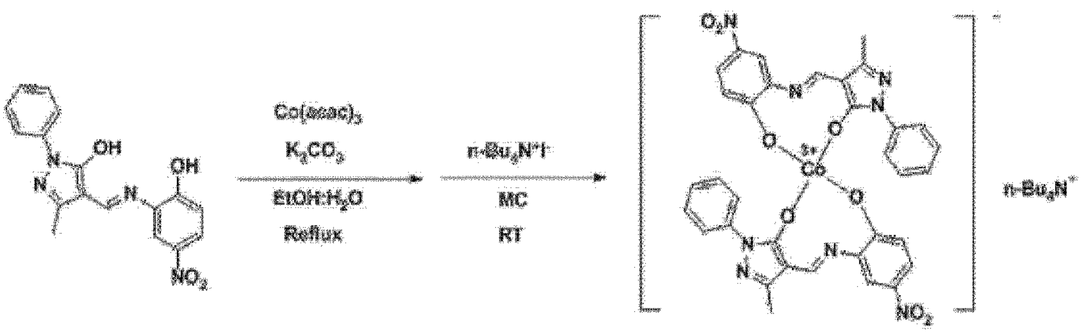


D3-j P21

Compound P21 (331 mg, yield: 46%) was secured by conducting a reaction in the same manner as in Preparation Example 16 except that D3-j (500 mg, 1 equivalent) was used instead of D1-a.

HR LC/MS/MS m/z calculated for $C_{50}H_{60}CoN_9O_8$ (M+): 973.3897; found: 973.3897

**Preparation Example 22: Synthesis of Compound P22**

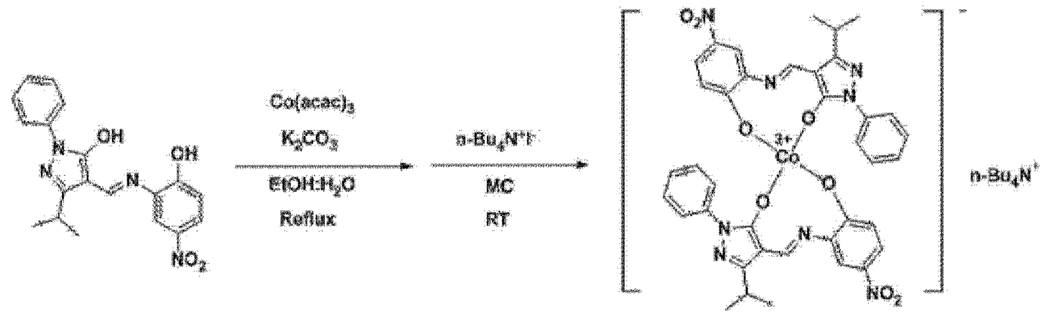


D3-e P22

Compound P22 (492 mg, yield: 70%) was secured by conducting a reaction in the same manner as in Preparation Example 16 except that D3-e (500 mg, 1 equivalent) was used instead of D1-a.

HR LC/MS/MS m/z calculated for $C_{54}H_{68}CoN_9O_8$ (M+): 1029.4523; found: 1029.4253

**Preparation Example 23: Synthesis of Compound P23**

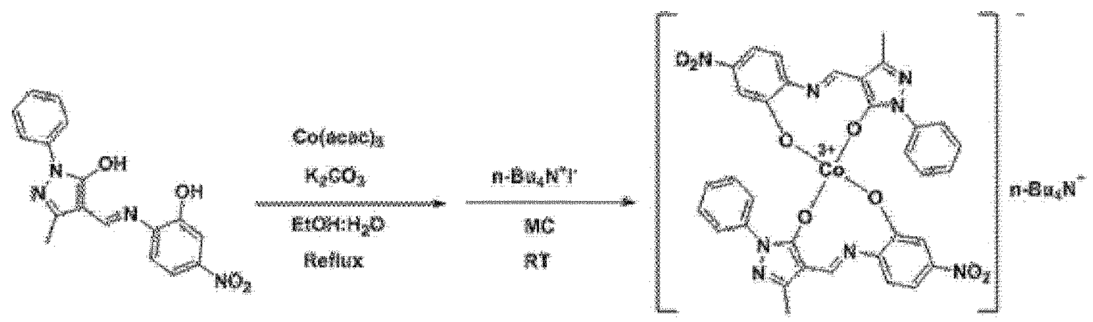


D3-k P23

Compound P23 (461 mg, yield: 64%) was secured by conducting a reaction in the same manner as in Preparation Example 16 except that D3-k (500 mg, 1 equivalent) was used instead of D1-a.

HR LC/MS/MS m/z calculated for $C_{50}H_{60}CoN_9O_8$ (M+): 973.3897; found: 973.3897

**Preparation Example 24: Synthesis of Compound P24**

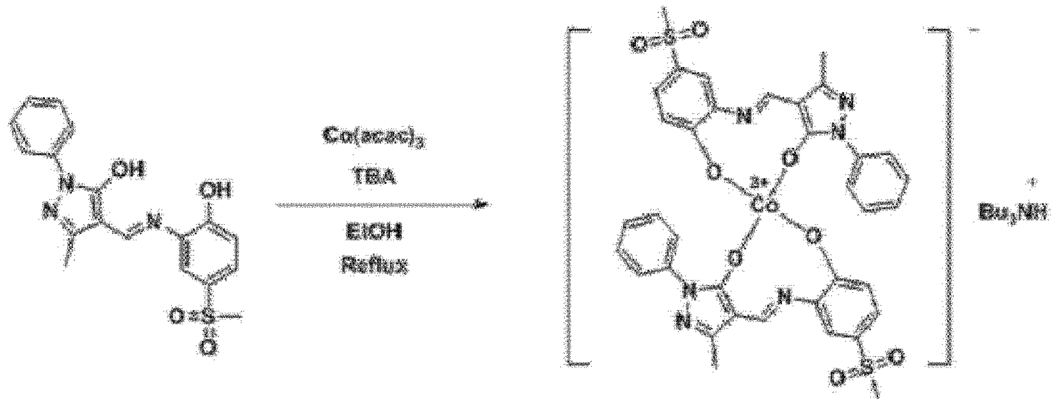


D3-g P24

Compound D3-g (400 mg, 1 equivalent) synthesized using the method of Reaction Formula 1 was dissolved in ethanol (10 mL), and after adding tributylamine (0.31 mL, 2 equivalents) and tris(acetylacetonato)cobalt(III) (0.192 g, 0.5 equivalents) thereto, the mixture was stirred while heating under nitrogen. After the reaction was finished, the result was concentrated, and extracted using chloroform and water. From the organic layer secured through extraction, water was removed using anhydrous magnesium sulfate, and the result was concentrated through vacuum distillation. Compound P24 (302 mg, yield: 57%) was secured from the concentrated product using ethanol and water.

HR LC/MS/MS m/z calculated for $C_{48}H_{58}CoN_7O_8S_2$ (M+) : 983.3120; found: 983.3121

## Preparation Example 25: Synthesis of Compound P25

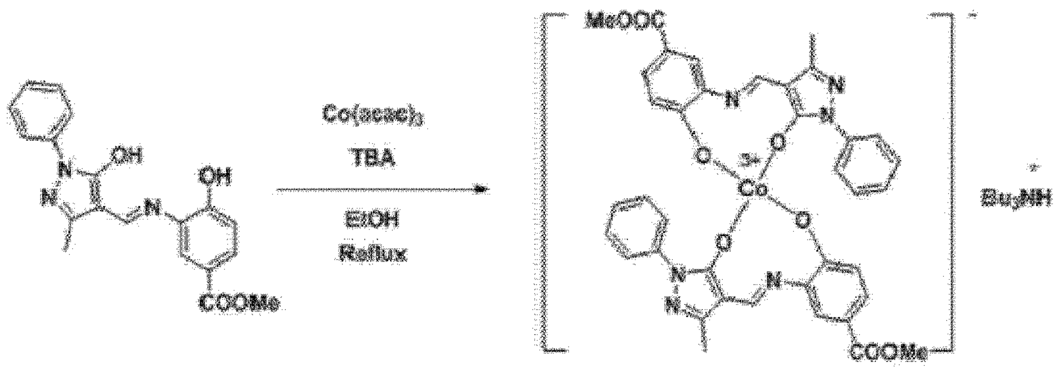


D3-l P25

Compound P25 (457 mg, yield: 68%) was secured by conducting a reaction in the same manner as in Preparation Example 24 except that D3-1 (500 mg, 1 equivalent) was used instead of D3-g.

HR LC/MS/MS m/z calculated for $C_{50}H_{58}CoN_7O_8$ (M+): 943.3680; found: 943.3679

## Preparation Example 26: Synthesis of Compound P26

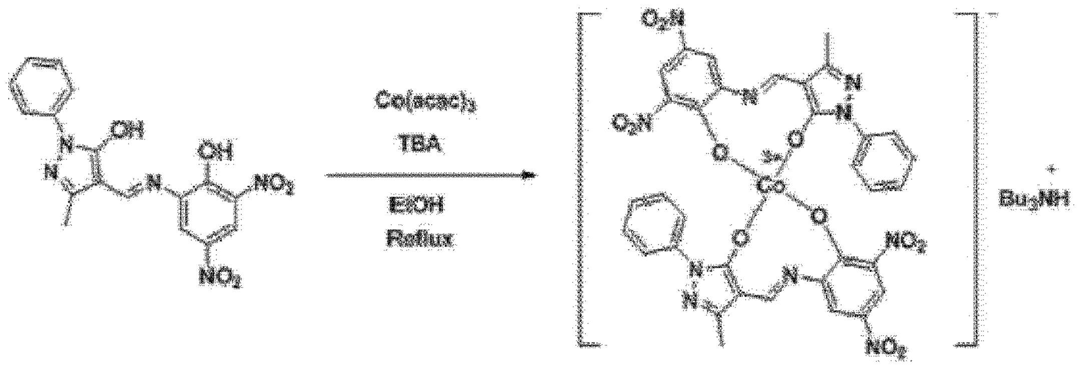


D3-n P26

Compound P26 (427 mg, yield: 65%) was secured by conducting a reaction in the same manner as in Preparation Example 24 except that D3-n (500 mg, 1 equivalent) was used instead of D3-g.

HR LC/MS/MS m/z calculated for $C_{46}H_{50}CoN_{11}O_{12}$ (M+) : 1007.2972; found: 1007.2972

## Preparation Example 27: Synthesis of Compound P27

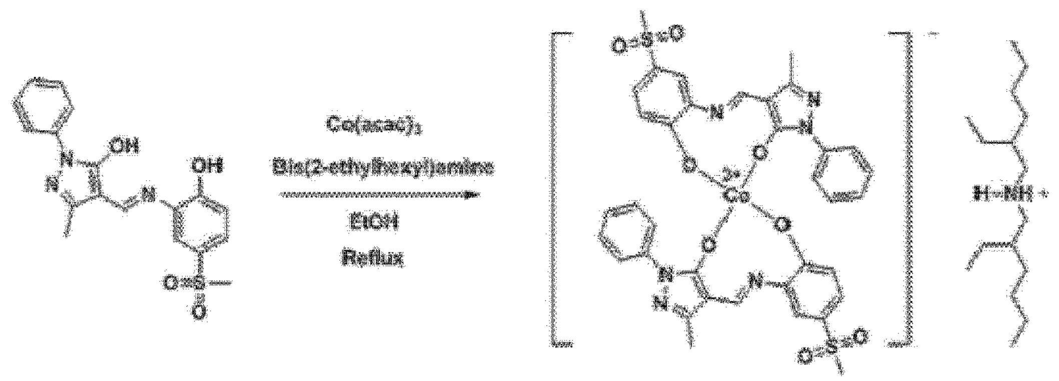


D3-g P27

Compound P27 (269 mg, yield: 48%) was secured by conducting a reaction in the same manner as in Preparation Example 24 except that bis(2-ethylhexyl)amine (0.3 mL, 2 equivalents) was used instead of tributylamine (TBA).

HR LC/MS/MS m/z calculated for $C_{52}H_{66}CoN_7O_8S_2$ (M+) : 1038.3746; found: 1038.3746

## Preparation Example 28: Synthesis of Compound P28

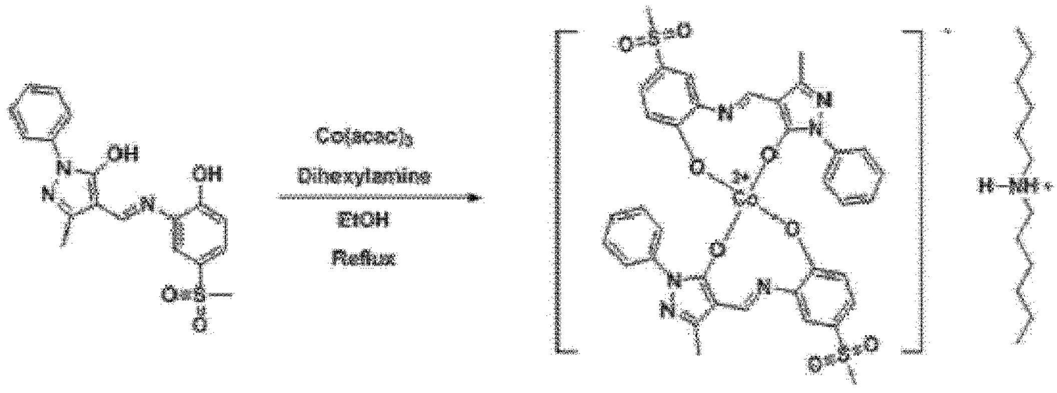


D3-g P28

Compound P28 (371 mg, yield: 56%) was secured by conducting a reaction in the same manner as in Preparation Example 24 except that dihexylamine (0.38 mL, 2 equivalents) was used instead of tributylamine (TBA).

HR LC/MS/MS m/z calculated for $C_{48}H_{58}CoN_7O_8S_2$ (M+) : 983.3120; found: 983.3121

## Preparation Example 29: Synthesis of Compound P29

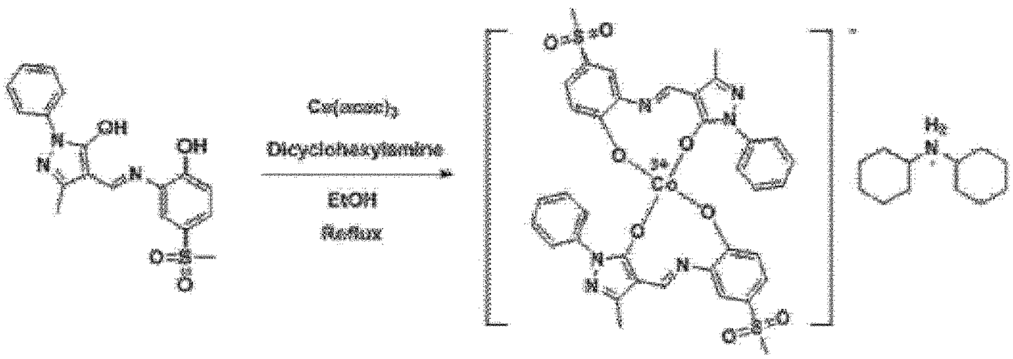


D3-g P29

Compound P29 (382 mg, yield: 58%) was secured by conducting a reaction in the same manner as in Preparation Example 24 except that dicyclohexylamine (490 mg, 2 equivalents) was used instead of tributylamine (TBA).

HR LC/MS/MS m/z calculated for $C_{48}H_{54}CoN_7O_8S_2$ (M+) : 979.2807; found: 979.2807

## Preparation Example 30: Synthesis of Compound P30

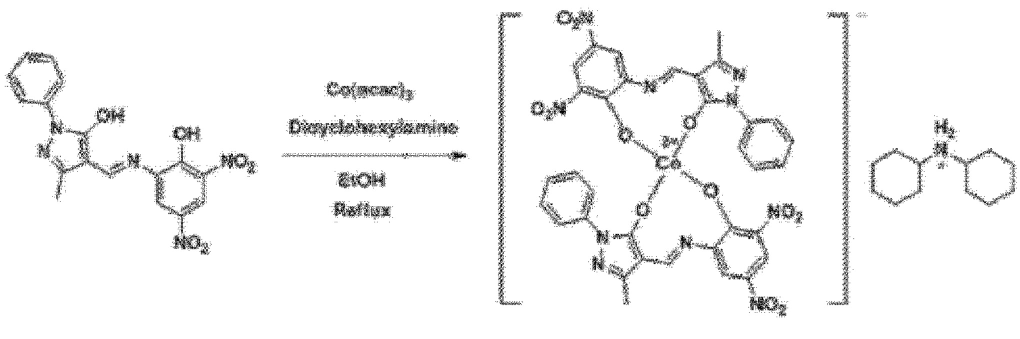


D3-a P30

Compound P30 (420 mg, yield: 65%) was secured by conducting a reaction in the same manner as in Preparation Example 24 except that D3-n (500 mg, 1 equivalent) was used instead of D3-g, and dicyclohexylamine (470 mg, 2 equivalents) was used instead of tributylamine (TBA).

HR LC/MS/MS m/z calculated for $C_{46}H_{46}CoN_{11}O_{12}$ (M+) : 1003.2659; found: 1003.2659

## Preparation Example 31: Synthesis of Compound P31

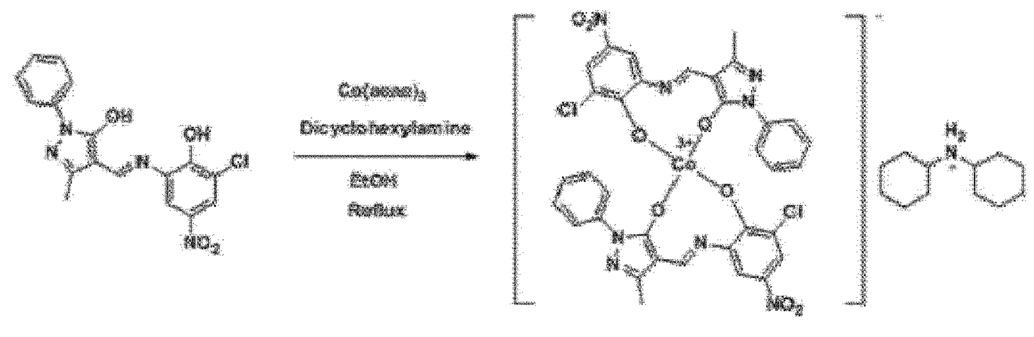


D3-o P31

Compound P31 (468 mg, yield: 71%) was secured by conducting a reaction in the same manner as in Preparation Example 24 except that D3-o (500 mg, 1 equivalent) was used instead of D3-g, and dicyclohexylamine (487 mg, 2 equivalents) was used instead of tributylamine (TBA).

HR LC/MS/MS m/z calculated for $C_{46}H_{46}Cl_2CoN_9O_8$ (M+) : 981.2178; found: 981.2179

## Preparation Example 32: Synthesis of Compound P32

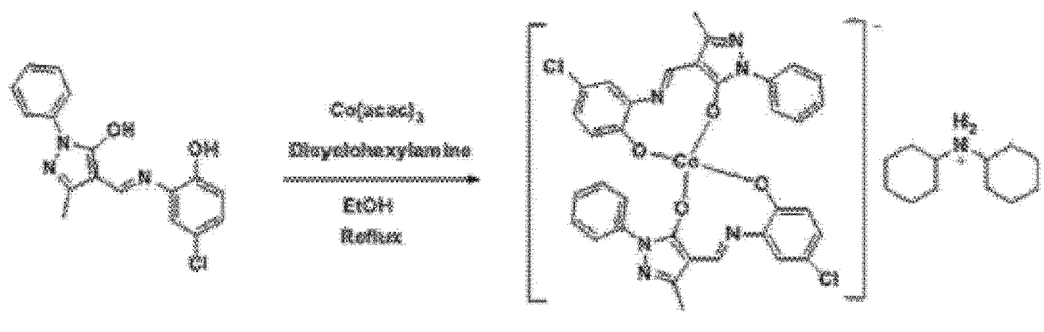


D3-p P32

Compound P32 (354 mg, yield: 52%) was secured by conducting a reaction in the same manner as in Preparation Example 24 except that D3-p (500 mg, 1 equivalent) was used instead of D3-g, and dicyclohexylamine (553 mg, 2 equivalents) was used instead of tributylamine (TBA).

HR LC/MS/MS m/z calculated for $C_{46}H_{46}Cl_2CoN_7O_4$ (M+) : 891.2477; found: 891.2477

## Preparation Example 33: Synthesis of Compound P33

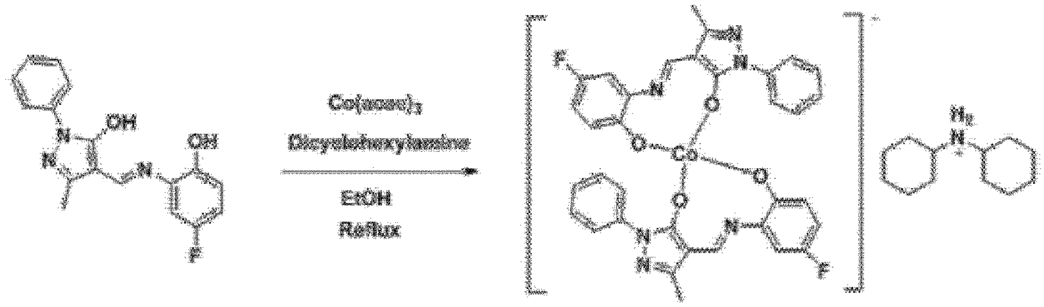


D3-q P33

Compound P33 (317 mg, yield: 46%) was secured by conducting a reaction in the same manner as in Preparation Example 24 except that D3-q (500 mg, 1 equivalent) was used instead of D3-g, and dicyclohexylamine (583 mg, 2 equivalents) was used instead of tributylamine (TBA).

HR LC/MS/MS m/z calculated for $C_{46}H_{48}CoF_2N_7O_4$ (M+) : 859.3068; found: 859.3068

**Preparation Example 34: Synthesis of Compound P34**

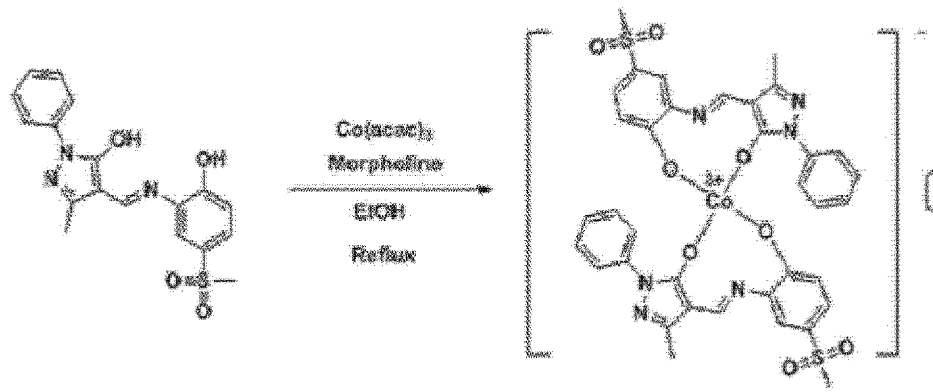


D3-g P34

Compound P34 (334 mg, yield: 56%) was secured by conducting a reaction in the same manner as in Preparation Example 24 except that morpholine (235 mg, 2 equivalents) was used instead of tributylamine (TBA), and D3-g (500 mg, 1 equivalent) used in Preparation Example 24 was used.

HR LC/MS/MS m/z calculated for $C_{40}H_{40}CoN_7O_9S_2$ (M+) : 885.1661; found: 885.1661

**Preparation Example 35: Synthesis of Compound P35**

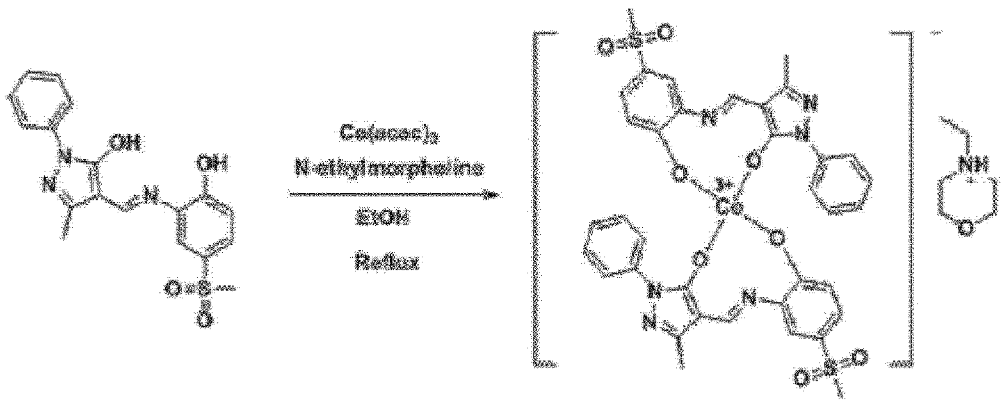


D3-g P35

Compound P35 (455 mg, yield: 74%) was secured by conducting a reaction in the same manner as in Preparation Example 24 except that N-ethyl morpholine (310 mg, 2 equivalents) was used instead of tributylamine (TBA), and D3-g (500 mg, 1 equivalent) used in Preparation Example 24 was used.

HR LC/MS/MS m/z calculated for $C_{42}H_{44}CoN_7O_9S_2$ (M+) : 913.1974; found: 913.1973

**Preparation Example 36: Synthesis of Compound P36**

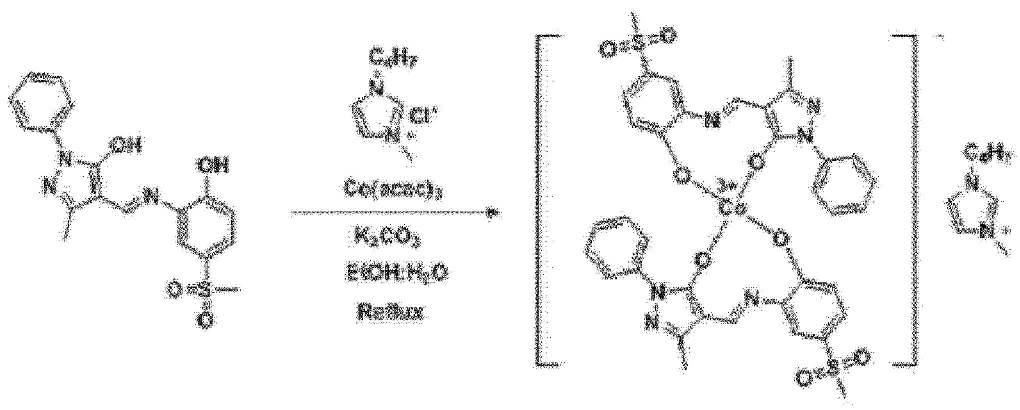


D3-g P36

Compound D3-g (400 mg, 1 equivalent) synthesized using the method of Reaction Formula 1 was dissolved in ethanol (10 mL), and potassium carbonate (372 mg, 2 equivalents) dissolved in water (10 mL) was added thereto. After sufficiently stirring the solution, tris(acetylacetonato)cobalt(III) (0.192 mg, 0.5 equivalents) and 1-butyl-3-methylimidazolium chloride (140 mg, 0.6 equivalents) were added thereto, and the mixture was stirred while heating under nitrogen. After the reaction was finished, the result was concentrated, and extracted using chloroform and water. From the organic layer secured through extraction, water was removed using anhydrous magnesium sulfate, then water was removed using anhydrous magnesium sulfate from the organic layer secured through vacuum distillation, and the result was concentrated through vacuum distillation. Compound P36 (183 mg, yield: 29%) was secured from the concentrated product using ethanol and water.

HR LC/MS/MS m/z calculated for $C_{44}H_{43}CoN_8O_8S_2$ (M+) : 934.1972; found: 934.1972

**Preparation Example 37: Synthesis of Compound P37**

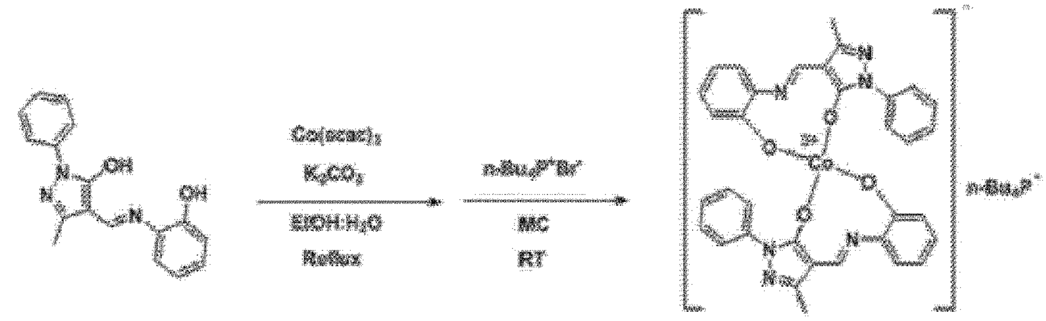


D1-a

P37

Compound P37 (369 mg, yield: 48%) was secured by conducting a reaction in the same manner as in Preparation Example 16 except that n-butylphosphine bromide (0.303 mg, 0.6 equivalents) was used instead of tetra-n-butylammonium iodine.

HR LC/MS/MS m/z calculated for $C_{50}H_{62}CoN_6O_4P$ (M+): 900.3902; found: 900.3903

**Preparation Example 38: Synthesis of Compound P38**

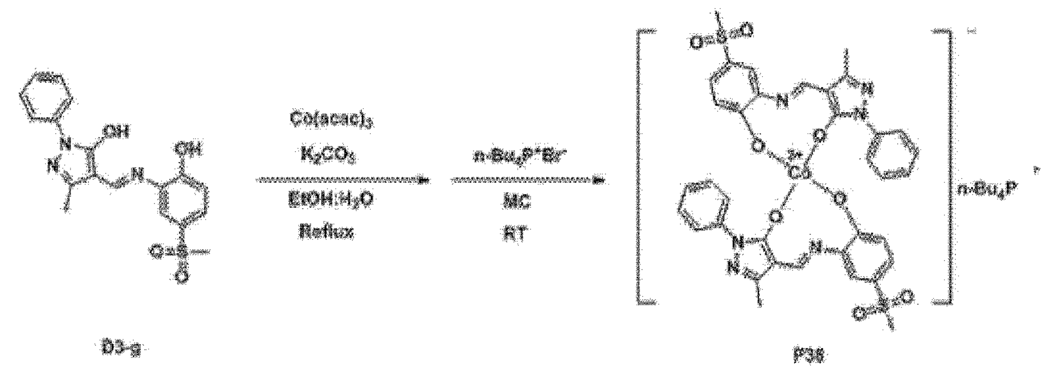


D3-g

P38

Compound P38 (278 mg, yield: 39%) was secured by conducting a reaction in the same manner as in Preparation Example 37 except that D3-g (500 mg, 1 equivalent) was used instead of D1-a.

HR LC/MS/MS m/z calculated for $C_{52}H_{66}CoN_6O_8PS_2$ (M+) : 1056.3453; found: 1056.3453

**Preparation Example 39: Synthesis of Compound P39**

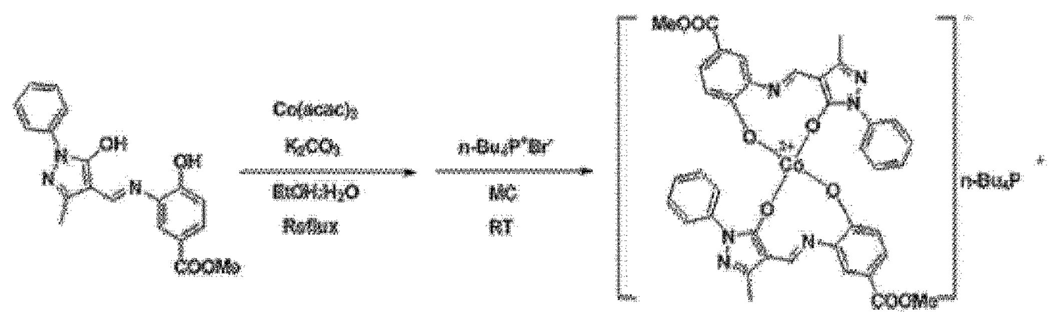


D3-l

P39

Compound P39 (391 mg, yield: 54%) was secured by conducting a reaction in the same manner as in Preparation Example 37 except that D3-l (500 mg, 1 equivalent) was used instead of D1-a.

HR LC/MS/MS m/z calculated for $C_{54}H_{66}CoN_6O_8P$ (M+) : 1016.4012; found: 1016.4012

**Preparation Example 40: Synthesis of Compound P40**

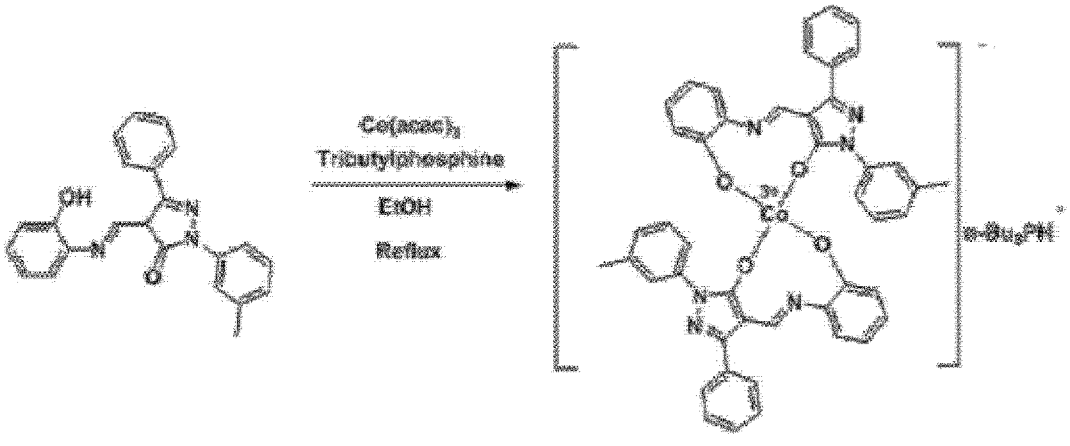


D2-d

P40

Compound P40 (351 mg, yield: 65%) was secured by conducting a reaction in the same manner as in Preparation Example 24 except that D2-d (400 mg, 1 equivalent) was used instead of D3-g, and tributylphosphine (0.438 mg, 2 equivalents) was used instead of tributylamine (TBA).

HR LC/MS/MS m/z calculated for $C_{58}H_{58}CoN_6O_4P$ (M+): 996.3902; found: 996.3902

**Preparation Example 41: Synthesis of Compound P41**

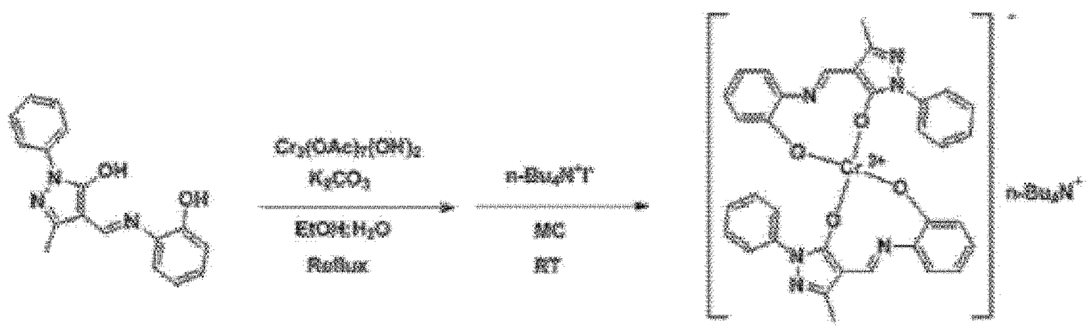


D1-a

P41

Compound D1-a (500 mg, 1 equivalent) synthesized using the method of Reaction Formula 1 was diluted in ethanol (10 mL), and after adding potassium carbonate (471 mg, 2 equivalents) dissolved in water (10 mL) thereto, the mixture was stirred while heating under nitrogen. After that, chromium acetate hydroxide (193 mg, 0.5 equivalents) was added thereto, and the reaction proceeded until the reactant D1-a was not visible on thin layer chromatography (TLC). After concentrating the solvent from the completed reaction material, a dichloromethane solvent (20 mL) was added thereto, then tetra-n-butylammonium iodine (378 mg, 0.6 equivalents) was introduced thereto, and the result was stirred at room temperature. After the reaction was completed, the result was extracted by adding water thereto, and from the organic layer secured therethrough, water was removed using anhydrous magnesium sulfate, and the result was concentrated through vacuum distillation. The concentrated product was diluted in ethanol, and then water was added thereto to secure Compound P41 (389 mg, yield: 52%).

HR LC/MS/MS m/z calculated for $C_{50}H_{62}CrN_7O_4$ (M+): 876.4268; found: 876.4268

**Preparation Example 42: Synthesis of Compound P42**

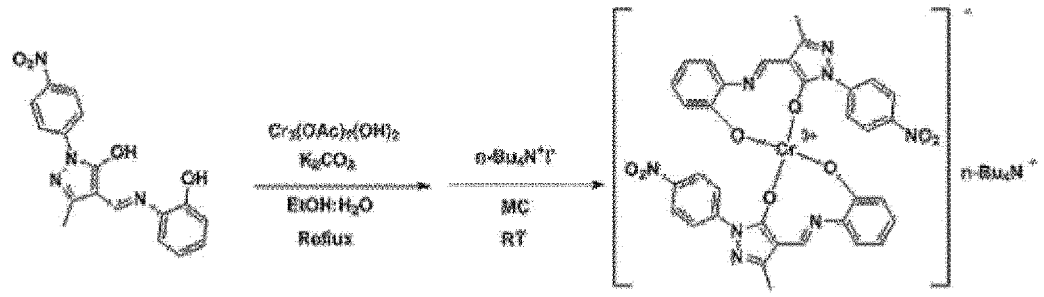


D1-d P42

Compound P42 (443 mg, yield: 62%) was secured by conducting a reaction in the same manner as in Preparation Example 41 except that D1-d (500 mg, 1 equivalent) was used instead of D1-a.

HR LC/MS/MS m/z calculated for $C_{50}H_{60}CrN_9O_8$ (M+): 966.3970; found: 966.3971

**Preparation Example 43: Synthesis of Compound P43**

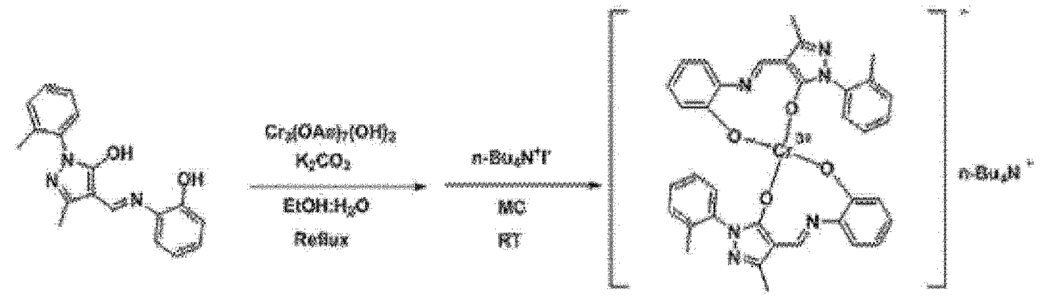


D1-f P43

Compound P43 (398 mg, yield: 54%) was secured by conducting a reaction in the same manner as in Preparation Example 41 except that D1-f (500 mg, 1 equivalent) was used instead of D1-a.

HR LC/MS/MS m/z calculated for $C_{52}H_{66}CrN_7O_4$ (M+): 904.4581; found: 904.4581

**Preparation Example 44: Synthesis of Compound P44**

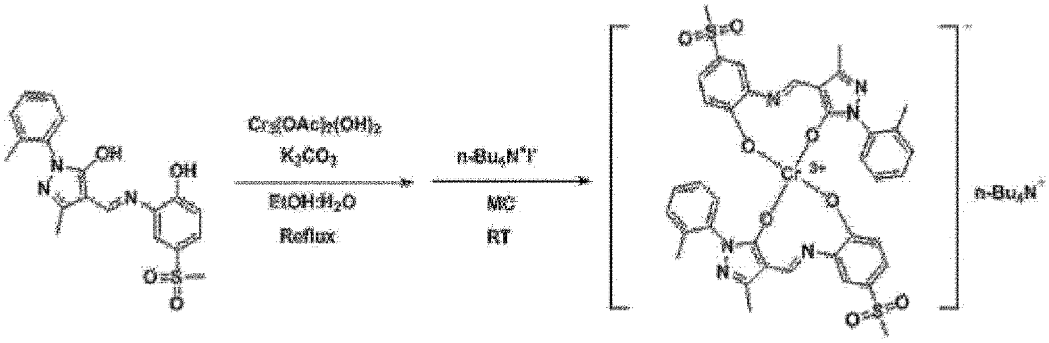


D3-i P44

Compound P44 (399 mg, yield: 58%) was secured by conducting a reaction in the same manner as in Preparation Example 41 except that D3-i (500 mg, 1 equivalent) was used instead of D1-a.

HR LC/MS/MS m/z calculated for $C_{54}H_{70}CrN_7O_8S_2$ (M+) : 1060.4132; found: 1060.4133

**Preparation Example 45: Synthesis of Compound P45**

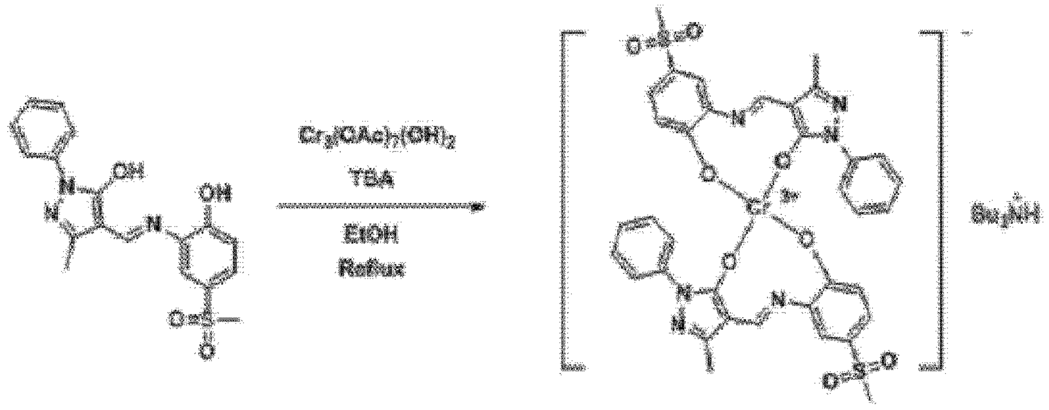


D3-g P45

Compound D3-g (400 mg, 1 equivalent) synthesized using the method of Reaction Formula 1 was dissolved in ethanol (10 mL), and tributylamine (499 mg, 2 equivalents) and chromium acetate hydroxide (406 mg, 0.5 equivalents) were added thereto, and the mixture was stirred while heating under nitrogen. After the reaction was finished, the result was concentrated, and extracted using chloroform and water. From the organic layer secured through extraction, water was removed using anhydrous magnesium sulfate, and the result was concentrated through vacuum distillation. Compound P45 (401 mg, yield: 61%) was secured from the concentrated product using ethanol and water.

HR LC/MS/MS m/z calculated for $C_{48}H_{58}CrN_7O_8S_2$ (M+) : 976.3193; found: 976.3193

**Preparation Example 46: Synthesis of Compound P46**

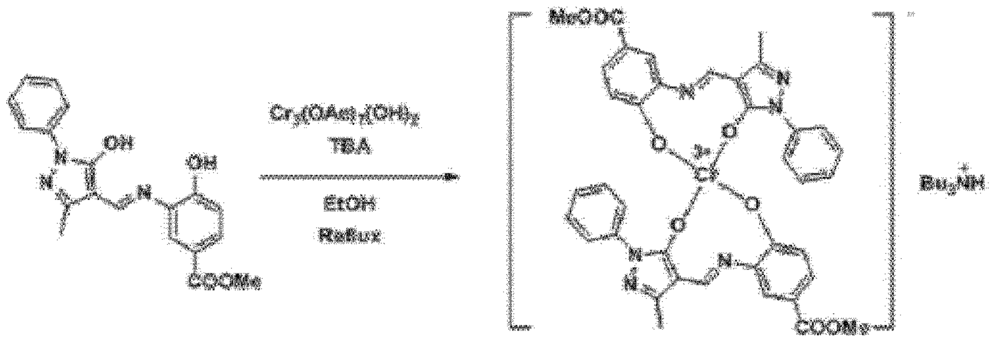


D3-l P46

Compound P46 (387 mg, yield: 58%) was secured by conducting a reaction in the same manner as in Preparation Example 45 except that D3-1 (500 mg, 1 equivalent) was used instead of D3-g.

HR LC/MS/MS m/z calculated for $C_{50}H_{58}CrN_7O_8$ (M+): 936.3752; found: 936.3751

**Preparation Example 47: Synthesis of Compound P47**

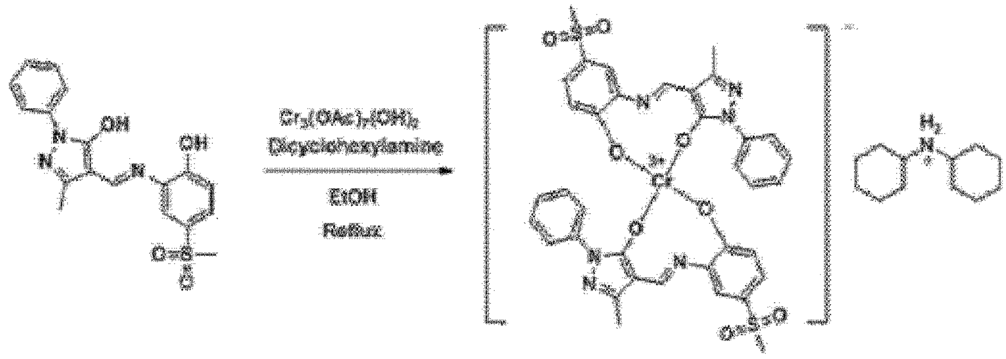


D3-g P47

Compound P47 (360 mg, yield: 55%) was secured by conducting a reaction in the same manner as in Preparation Example 45 except that dicyclohexylamine (488 mg, 2 equivalents) was used instead of tributylamine.

HR LC/MS/MS m/z calculated for $C_{48}H_{54}CrN_7O_8S_2$ (M+) : 972.2880; found: 972.2881

**Preparation Example 48: Synthesis of Compound P48**

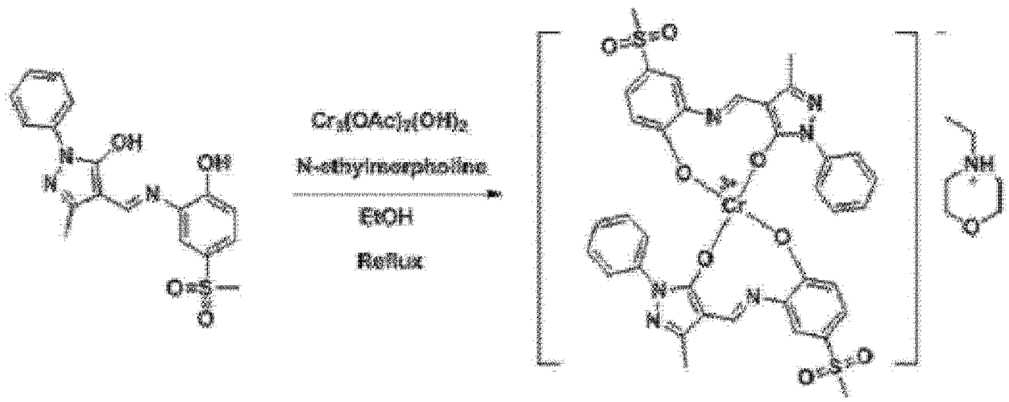


D3-g P48

Compound P48 (433 mg, yield: 71%) was secured by conducting a reaction in the same manner as in Preparation Example 45 except that N-ethyl morpholine (310 mg, 2 equivalents) was used instead of tributylamine.

HR LC/MS/MS m/z calculated for $C_{42}H_{44}CrN_7O_9S_2$ (M+) : 906.2047; found: 906.2047

**Preparation Example 49: Synthesis of Compound P49**

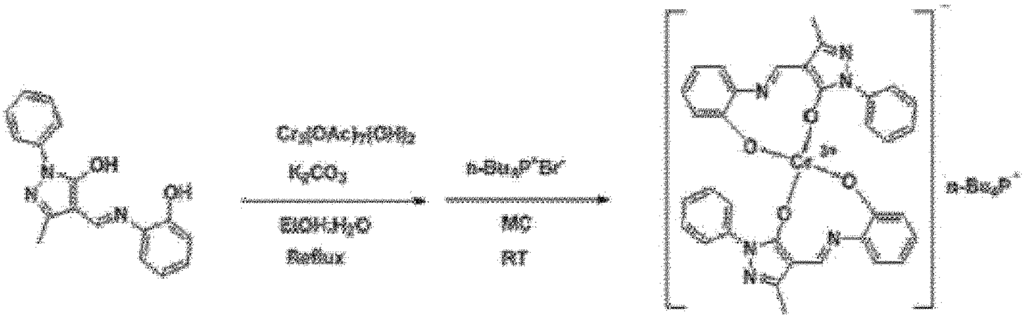


D1-a P49

Compound P49 (488 mg, yield: 64%) was secured by conducting a reaction in the same manner as in Preparation Example 41 except that n-tetrabutylphosphine bromide (471 mg, 0.6 equivalents) was used instead of n-tetrabutylammonium iodine.

HR LC/MS/MS m/z calculated for $C_{50}H_{62}CrN_6O_4P$ (M+): 893.3975; found: 893.3975

**Preparation Example 50: Synthesis of Compound P50**

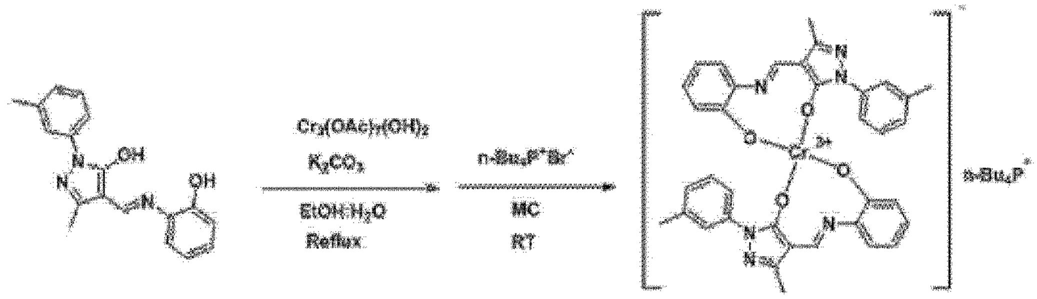


D1-g P50

Compound P50 (406 mg, yield: 55%) was secured by conducting a reaction in the same manner as in Preparation Example 49 except that D1-g (500 mg, 1 equivalent) was used instead of D1-a.

HR LC/MS/MS m/z calculated for $C_{51}H_{63}CrN_6O_4P$ (M+): 906.4054; found: 906.4054

**Preparation Example 51: Synthesis of Compound P51**

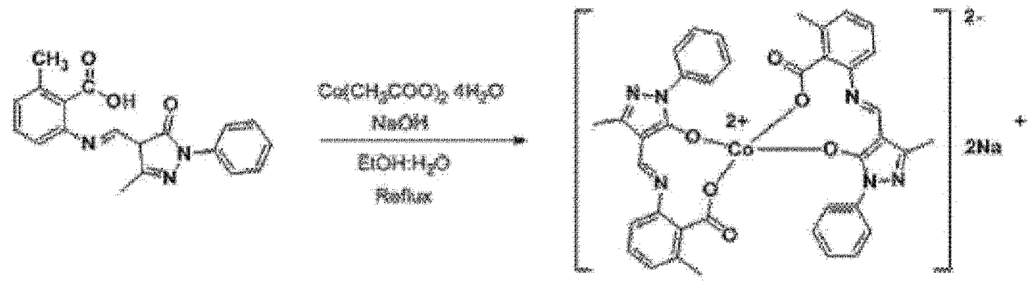


D4-a P51

Compound P51 (276 mg, yield: 48%) was secured by conducting a reaction in the same manner as in Preparation Example 1 except that D4-a (500 mg, 1 equivalent) was used instead of D1-a.

HR LC/MS/MS m/z calculated for $C_{38}H_{30}CoN_6Na_2O_6$ (M+) : 771.1354; found: 771.1354

**Preparation Example 52: Synthesis of Compound P52**

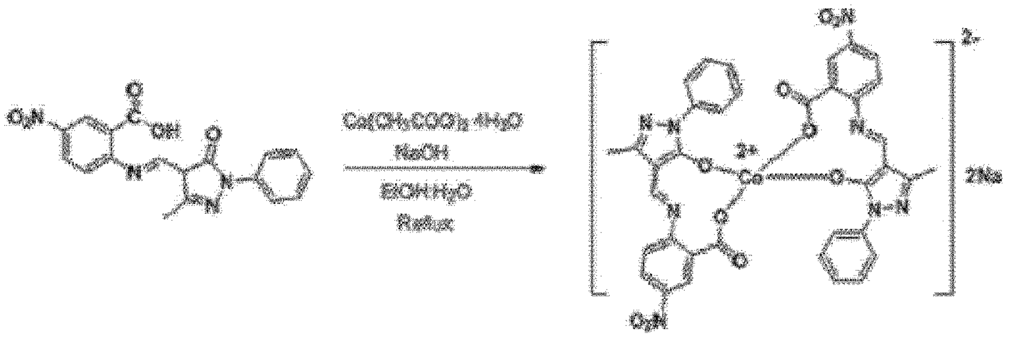


D4-c

P52

Compound P52 (296 mg, yield: 52%) was secured by conducting a reaction in the same manner as in Preparation Example 1 except that D4-c (500 mg, 1 equivalent) was used instead of D1-a.

HR LC/MS/MS m/z calculated for $C_{36}H_{24}CoN_8Na_2O_{10}$ (M+) : 833.0743; found: 833.0743

**Preparation Example 53: Synthesis of Compound P53**

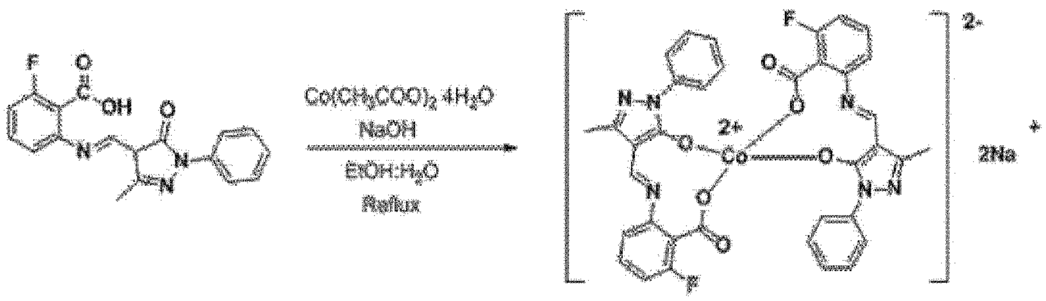


D4-f

P53

Compound P53 (224 mg, yield: 39%) was secured by conducting a reaction in the same manner as in Preparation Example 1 except that D4-f (500 mg, 1 equivalent) was used instead of D1-a.

HR LC/MS/MS m/z calculated for $C_{36}H_{24}CoF_2N_6Na_2O_6$ (M+) : 779.0853; found: 779.0853

**Preparation Example 54: Synthesis of Compound P54**

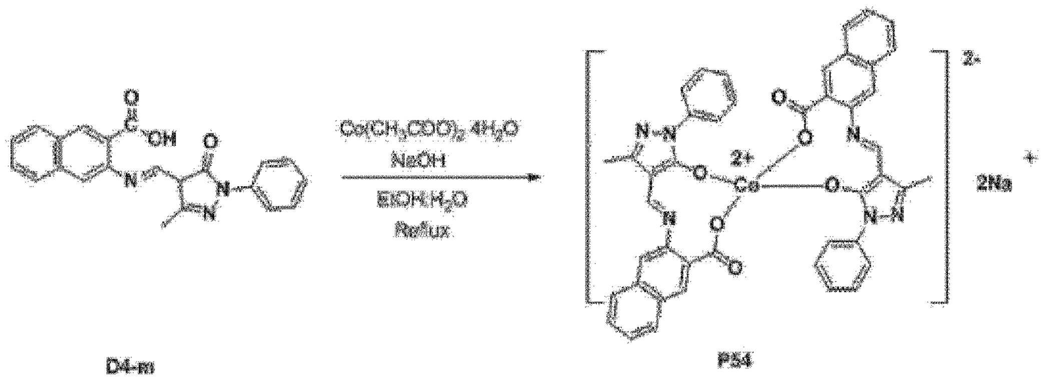


D4-m

P54

Compound P54 (339 mg, yield: 62%) was secured by conducting a reaction in the same manner as in Preparation Example 1 except that D4-m (500 mg, 1 equivalent) was used instead of D1-a.

HR LC/MS/MS m/z calculated for $C_{44}H_{30}CoN_6Na_2O_6$ (M+) : 843.1354; found: 843.1354

**Preparation Example 55: Synthesis of Compound P55**

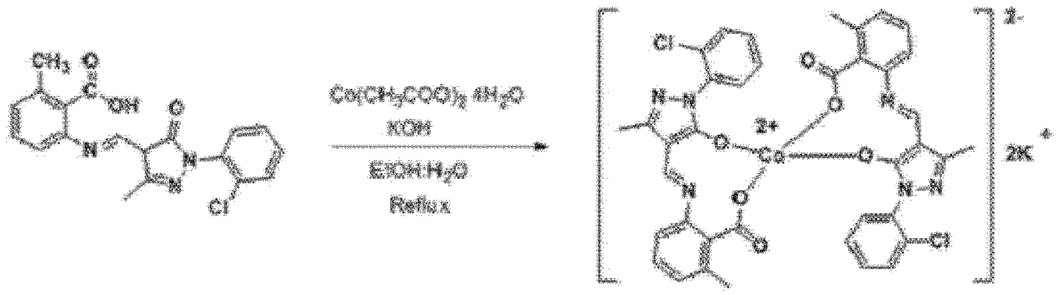


D4-p P55

Compound P55 (342 mg, yield: 58%) was secured by conducting a reaction in the same manner as in Preparation Example 1 except that D4-p (500 mg, 1 equivalent) was used instead of D1-a, and potassium hydroxide (151 mg, 2 equivalents) was used instead of sodium hydroxide (NaOH).

HR LC/MS/MS m/z calculated for $C_{38}H_{28}Cl_2CoK_2N_6O_6$ (M+) : 871.0054; found: 871.0054

**Preparation Example 56: Synthesis of Compound P56**

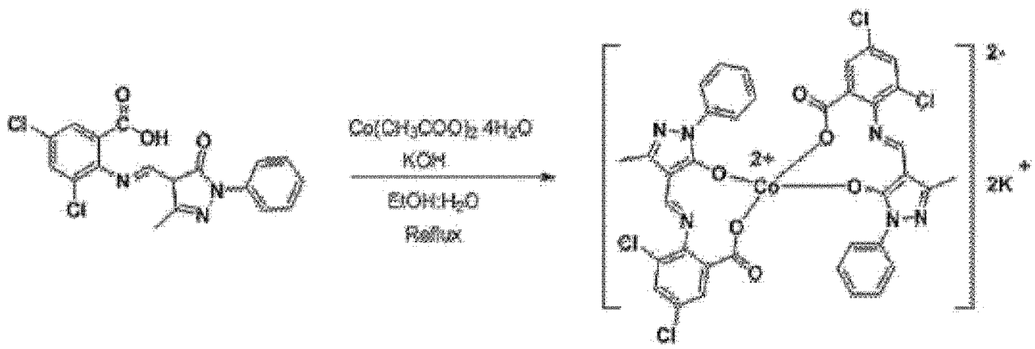


D4-t P56

Compound P56 (259 mg, yield: 49%) was secured by conducting a reaction in the same manner as in Preparation Example 1 except that D4-t (500 mg, 1 equivalent) was used instead of D1-a, and potassium hydroxide (130 mg, 2 equivalents) was used instead of sodium hydroxide (NaOH).

HR LC/MS/MS m/z calculated for $C_{36}H_{22}Cl_4CoK_2N_6O_6$ (M+) : 910.8961; found: 910.8960

**Preparation Example 57: Synthesis of Compound P57**

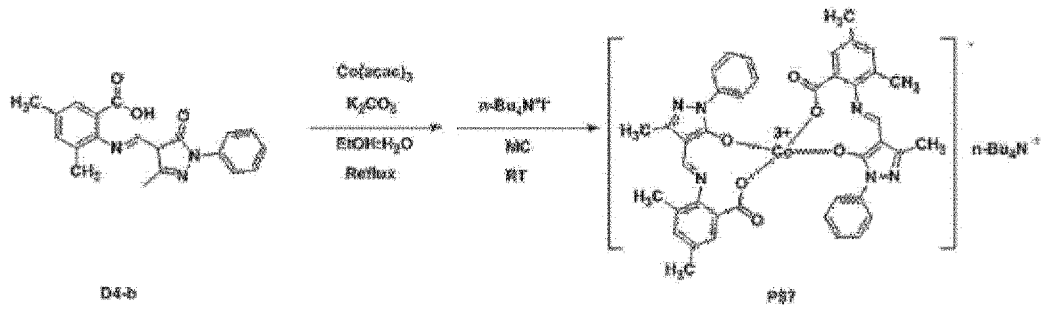


D4-b P57

Compound P57 (385 mg, yield: 54%) was secured by conducting a reaction in the same manner as in Preparation Example 16 except that D4-b (500 mg, 1 equivalent) was used instead of D1-a.

HR LC/MS/MS m/z calculated for $C_{56}H_{70}CoN_7O_6$ (M+): 995.4714; found: 995.4714

## Preparation Example 58: Synthesis of Compound P58

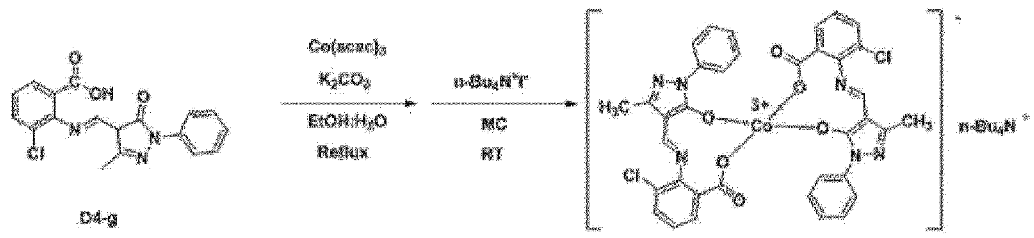


D4-g

P58

Compound P58 (440 mg, yield: 62%) was secured by conducting a reaction in the same manner as in Preparation Example 16 except that D4-g (500 mg, 1 equivalent) was used instead of D1-a.

HR LC/MS/MS m/z calculated for $C_{52}H_{60}Cl_2CoN_7O_6$ (M+) : 1007.3309; found: 1007.3308

## Preparation Example 59: Synthesis of Compound P59

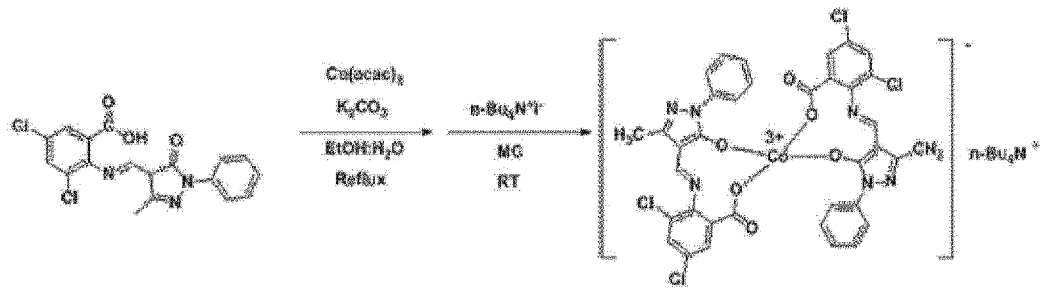


D4-h P59

Compound P59 (359 mg, yield: 52%) was secured by conducting a reaction in the same manner as in Preparation Example 16 except that D4-h (500 mg, 1 equivalent) was used instead of D1-a.

HR LC/MS/MS m/z calculated for $C_{52}H_{58}Cl_4CoN_7O_6$ (M+) : 1077.2500; found: 1077.2501

## Preparation Example 60: Synthesis of Compound P60

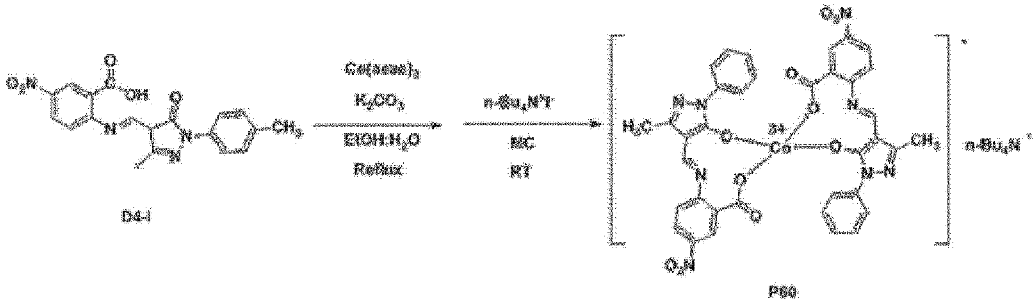


D4-l

P60

Compound P60 (251 mg, yield: 37%) was secured by conducting a reaction in the same manner as in Preparation Example 16 except that D4-1 (500 mg, 1 equivalent) was used instead of D1-a.

HR LC/MS/MS m/z calculated for $C_{52}H_{60}CoN_9O_{10}$ (M+) : 1029.3790; found: 1029.3789

**Preparation Example 61: Synthesis of Compound P61**

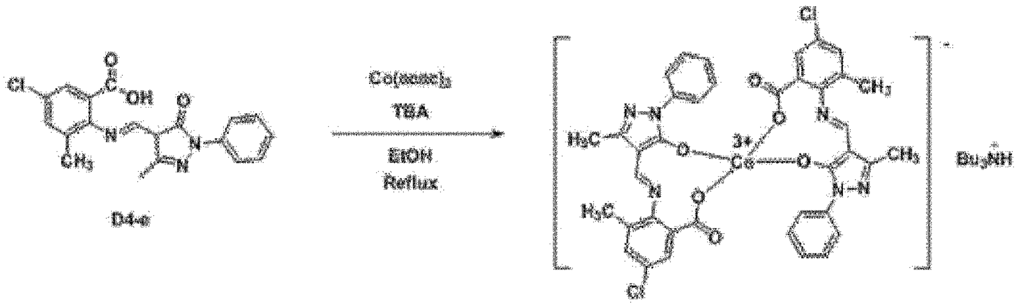


D4-e

P61

Compound P61 (298 mg, yield: 45%) was secured by conducting a reaction in the same manner as in Preparation Example 24 except that D4-e (500 mg, 1 equivalent) was used instead of D3-g.

HR LC/MS/MS m/z calculated for $C_{50}H_{55}Cl_2CoN_7O_6$ (M+) : 979.2996; found: 979.2996

**Preparation Example 62: Synthesis of Compound P62**

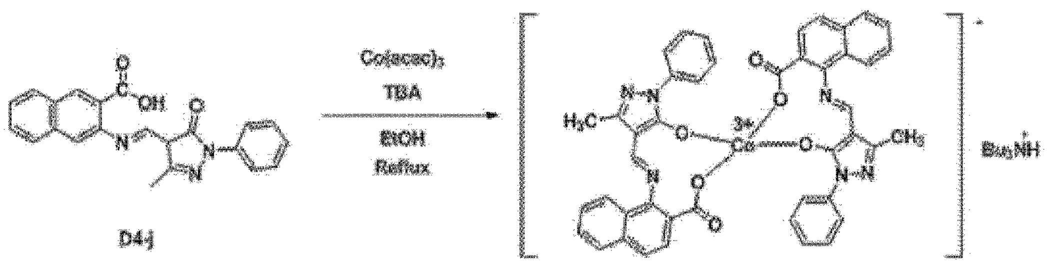


D4-j

P62

Compound P62 (450 mg, yield: 68%) was secured by conducting a reaction in the same manner as in Preparation Example 24 except that D4-j (500 mg, 1 equivalent) was used instead of D3-g.

HR LC/MS/MS m/z calculated for $C_{56}H_{58}CoN_7O_6$ (M+): 983.3775; found: 983.3774

**Preparation Example 63: Synthesis of Compound P63**

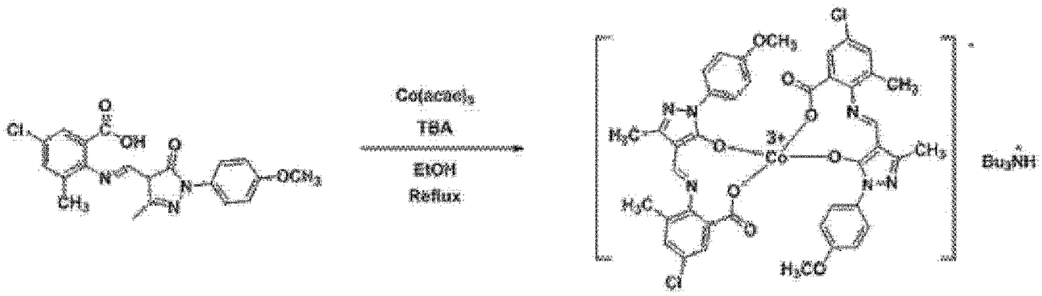


D4-n P63

Compound P63 (469 mg, yield: 72%) was secured by conducting a reaction in the same manner as in Preparation Example 24 except that D4-n (500 mg, 1 equivalent) was used instead of D3-g.

HR LC/MS/MS m/z calculated for $C_{52}H_{60}Cl_2CoN_7O_8$ (M+) : 1039.3207; found: 1039.3207

## Preparation Example 64: Synthesis of Compound P64

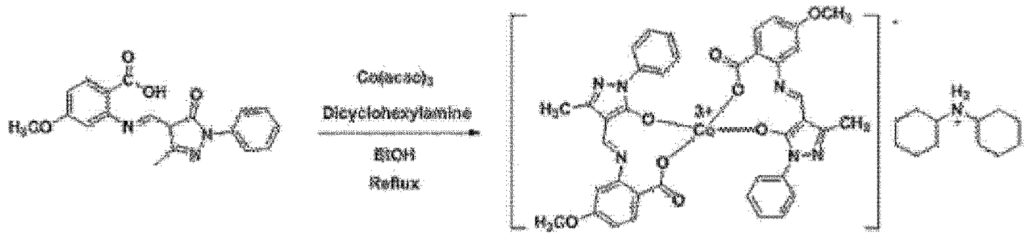


D4-d P64

Compound P64 (388 mg, yield: 58%) was secured by conducting a reaction in the same manner as in Preparation Example 24 except that D4-d (500 mg, 1 equivalent) was used instead of D3-g, and dicyclohexylamine (516 mg, 2 equivalents) was used instead of tributylamine (TBA).

HR LC/MS/MS m/z calculated for $C_{50}H_{54}CoN_7O_8$ (M+): 939.3360; found: 939.3360

## Preparation Example 65: Synthesis of Compound P65

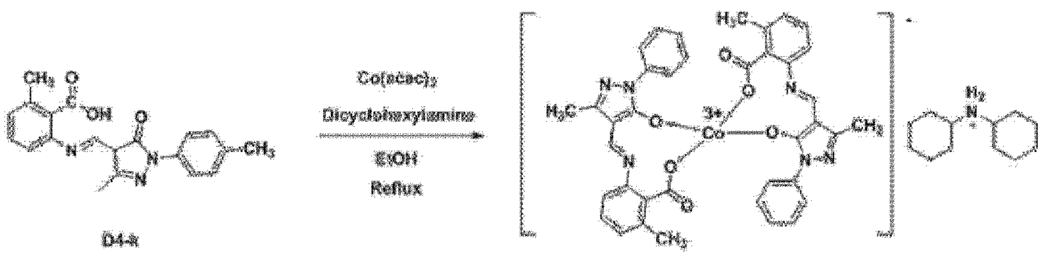


D4-k P65

Compound P65 (403 mg, yield: 62%) was secured by conducting a reaction in the same manner as in Preparation Example 24 except that D4-k (500 mg, 1 equivalent) was used instead of D3-g, and dicyclohexylamine (519 mg, 2 equivalents) was used instead of tributylamine (TBA).

HR LC/MS/MS m/z calculated for $C_{50}H_{54}CoN_7O_6$ (M+): 907.3462; found: 907.3462

**Preparation Example 66: Synthesis of Compound P66**

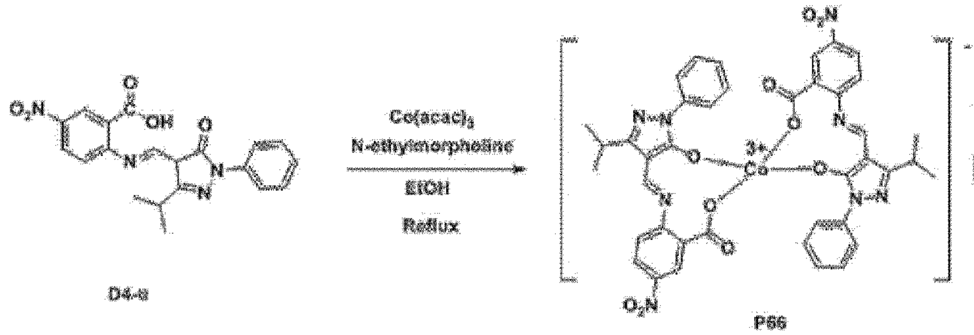


D4-u

P66

Compound P66 (268 mg, yield: 44%) was secured by conducting a reaction in the same manner as in Preparation Example 24 except that D4-u (500 mg, 1 equivalent) was used instead of D3-g, and N-ethyl morpholine (292 mg, 2 equivalents) was used instead of tributylamine (TBA).

HR LC/MS/MS m/z calculated for $C_{46}H_{46}CoN_9O_{11}$ (M+): 959.2600; found: 959.2601

**Preparation Example 67: Synthesis of Compound P67**

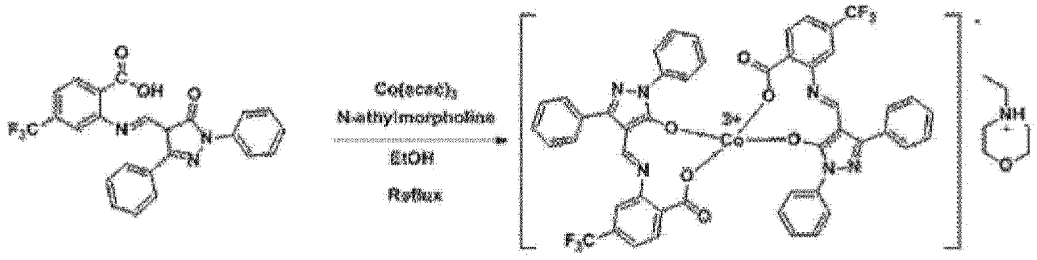


D4-v

P67

Compound P67 (351 mg, yield: 59%) was secured by conducting a reaction in the same manner as in Preparation Example 24 except that D4-v (500 mg, 1 equivalent) was used instead of D3-g, and N-ethyl morpholine (255 mg, 2 equivalents) was used instead of tributylamine (TBA).

HR LC/MS/MS m/z calculated for $C_{54}H_{42}CoF_6N_7O_7$ (M+) : 1073.2376; found: 1073.2376

**Preparation Example 68: Synthesis of Compound P68**

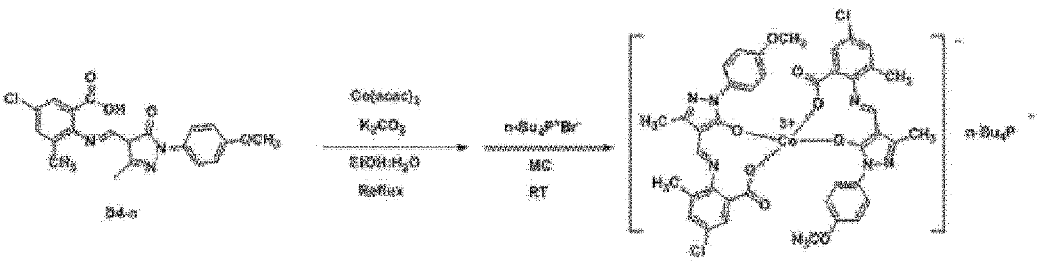


D4-n

P68

Compound P68 (502 mg, yield: 72%) was secured by conducting a reaction in the same manner as in Preparation Example 16 except that D4-n (500 mg, 1 equivalent) was used instead of D1-a, and n-tetrabutylphosphine bromide (130

mg, 0.6 equivalents) was used instead of n-tetrabutylammonium iodine.

HR LC/MS/MS m/z calculated for $C_{56}H_{68}Cl_2CoN_6O_BP$ (M+): 1112.3540; found: 1112.3541

## Preparation Example 69: Synthesis of Compound P69

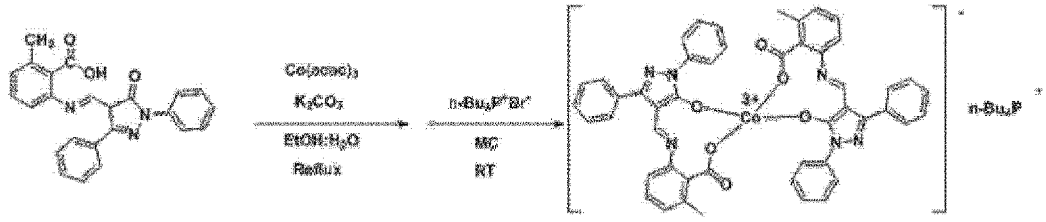


D4-r

P69

Compound P69 (405 mg, yield: 72%) was secured by conducting a reaction in the same manner as in Preparation Example 16 except that D4-r (500 mg, 1 equivalent) was used instead of D1-a, and n-tetrabutylphosphine bromide (130 mg, 0.6 equivalents) was used instead of n-tetrabutylammonium iodine.

HR LC/MS/MS m/z calculated for $C_{64}H_{70}CoN_6O_6P$ (M+) : 1108.4421; found: 1108.4421

## Preparation Example 70: Synthesis of Compound P70

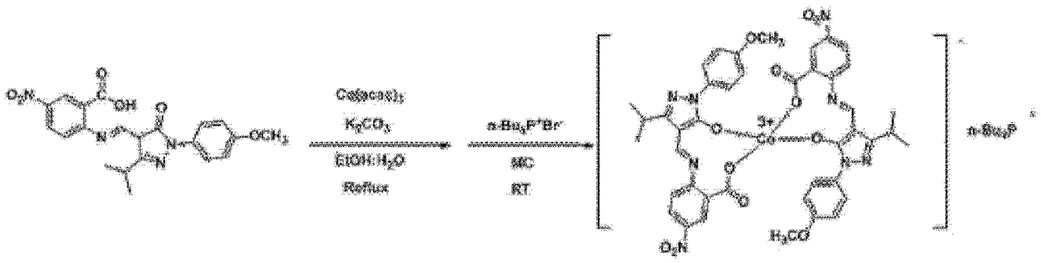


D4-w

P70

Compound P70 (267 mg, yield: 39%) was secured by conducting a reaction in the same manner as in Preparation Example 16 except that D4-w (500 mg, 1 equivalent) was used instead of D1-a, and n-tetrabutylphosphine bromide (122 mg, 0.6 equivalents) was used instead of n-tetrabutylammonium iodine.

HR LC/MS/MS m/z calculated for $C_{58}H_{72}CoN_8O_{12}P$ (M+) : 1162.4334; found: 1162.4334

## Preparation Example 71: Synthesis of Compound P71

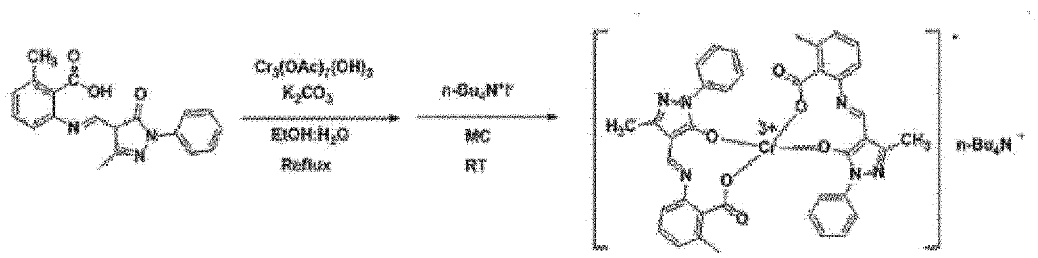


D4-a

P71

Compound P71 (322 mg, yield: 45%) was secured by conducting a reaction in the same manner as in Preparation Example 41 except that D4-a (500 mg, 1 equivalent) was used instead of D1-a.

HR LC/MS/MS m/z calculated for $C_{54}H_{66}CrN_7O_6$ (M+): 960.4474; found: 960.4474

## Preparation Example 72: Synthesis of Compound P72

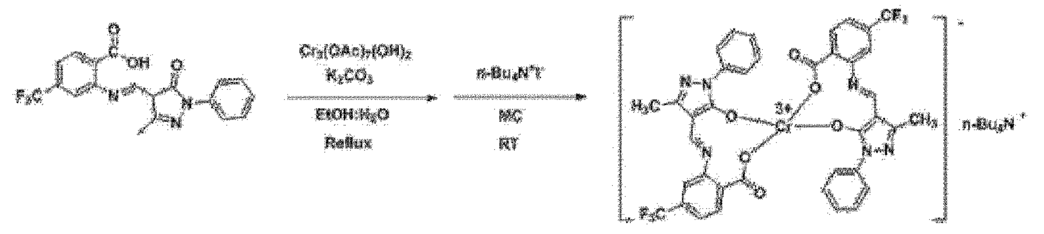


D4-i P72

Compound P72 (378 mg, yield: 55%) was secured by conducting a reaction in the same manner as in Preparation Example 41 except that D4-i (500 mg, 1 equivalent) was used instead of D1-a.

HR LC/MS/MS m/z calculated for $C_{54}H_{60}CrF_6N_7O_6$ (M+) : 1068.3909; found: 1068.3909

## Preparation Example 73: Synthesis of Compound P73

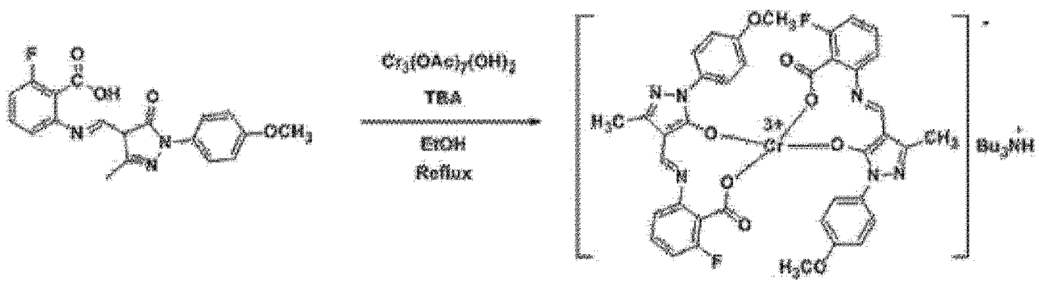


D4-o P73

Compound P73 (402 mg, yield: 61%) was secured by conducting a reaction in the same manner as in Preparation Example 45 except that D4-o (500 mg, 1 equivalent) was used instead of D3-g.

HR LC/MS/MS m/z calculated for $C_{50}H_{56}CrF_2N_7O_8$ (M+) : 972.3558; found: 972.3558

## Preparation Example 74: Synthesis of Compound P74

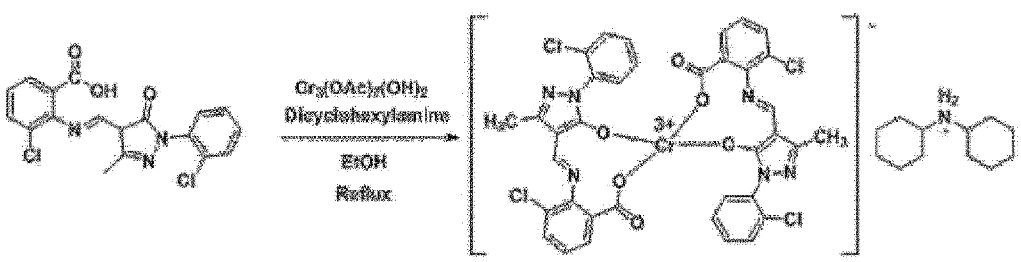


D4-q P74

Compound P74 (330 mg, yield: 51%) was secured by conducting a reaction in the same manner as in Preparation Example 45 except that D4-q (500 mg, 1 equivalent) was used instead of D3-g, and dicyclohexylamine (465 mg, 2 equivalents) was used instead of tributylamine.

HR LC/MS/MS m/z calculated for $C_{66}H_{74}Cl_4CrN_7O_6$ (M+) : 1129.4807; found: 1129.4807

**Preparation Example 75: Synthesis of Compound P75**

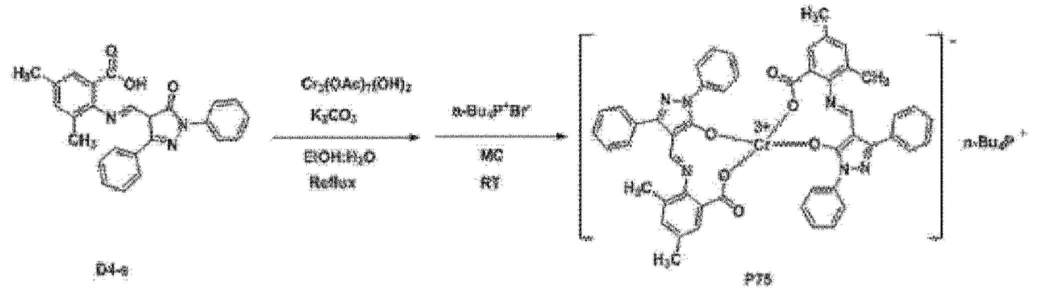


D4-s

P75

Compound P75 (261 mg, yield: 38%) was secured by conducting a reaction in the same manner as in Preparation Example 41 except that D4-s (500 mg, 1 equivalent) was used instead of D1-a, and n-tetrabutylphosphine bromide (269 mg, 0.6 equivalents) was used instead of n-tetrabutylammonium iodine.

HR LC/MS/MS m/z calculated for $C_{66}H_{74}CrN_6O_6P$ (M+) : 1129.4807; found: 1129.4807

**Preparation Example 76: Synthesis of Comparative Compound P76**

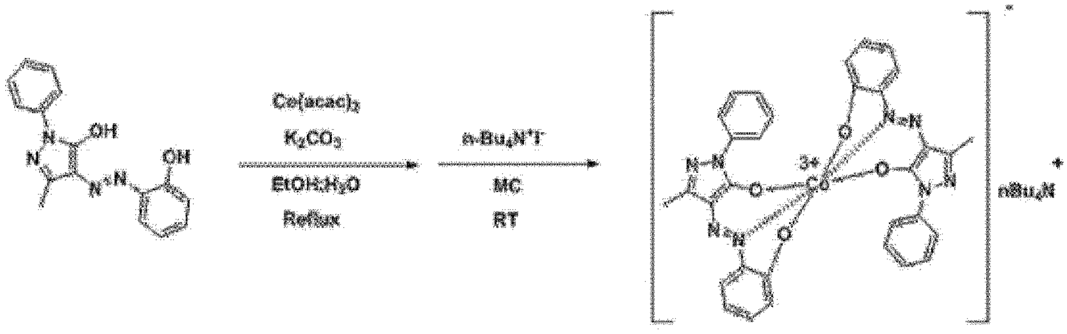


A1

P76

Comparative Compound P76 (565 mg, yield: 75%) was secured by conducting a reaction in the same manner as in Preparation Example 16 except that A1 (500 mg, 1 equivalent) was used instead of D1-a.

HR LC/MS/MS m/z calculated for $C_{48}H_{46}CoN_9O_4$ (M+): 885.4100; found: 885.4100

**Preparation Example 77: Synthesis of Comparative Compound P77**

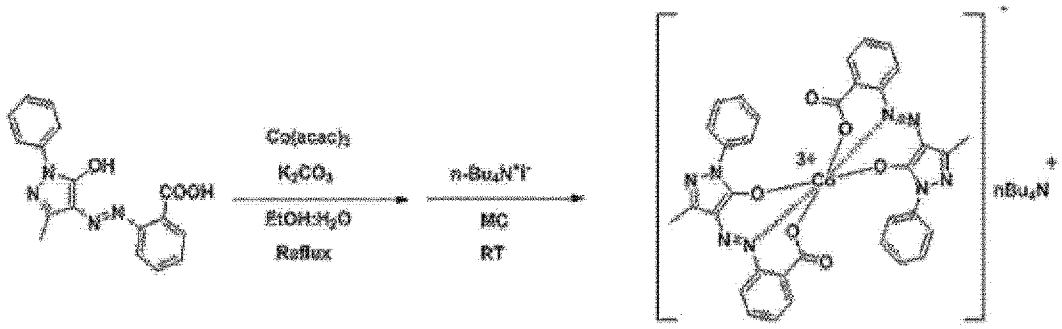


A2 P77

Comparative Compound P77 (482 mg, yield: 66%) was secured by conducting a reaction in the same manner as in Preparation Example 16 except that A2 (500 mg, 1 equivalent) was used instead of D1-a.

HR LC/MS/MS m/z calculated for $C_{50}H_{60}CoN_9O_6$ (M+): 941.3999; found: 941.3999

## Preparation Example 78: Synthesis of Comparative Compound P78

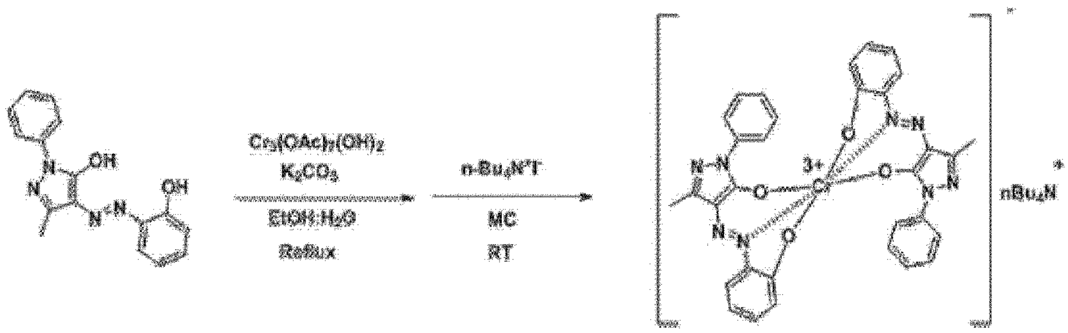


A1 P78

Comparative Compound P78 (336 mg, yield: 45%) was secured by conducting a reaction in the same manner as in Preparation Example 41 except that A1 was used instead of D1-a.

HR LC/MS/MS m/z calculated for $C_{48}H_{60}CrN_9O_4$ (M+): 878.4173; found: 878.4173

## Preparation Example 79: Synthesis of Comparative Compound P79

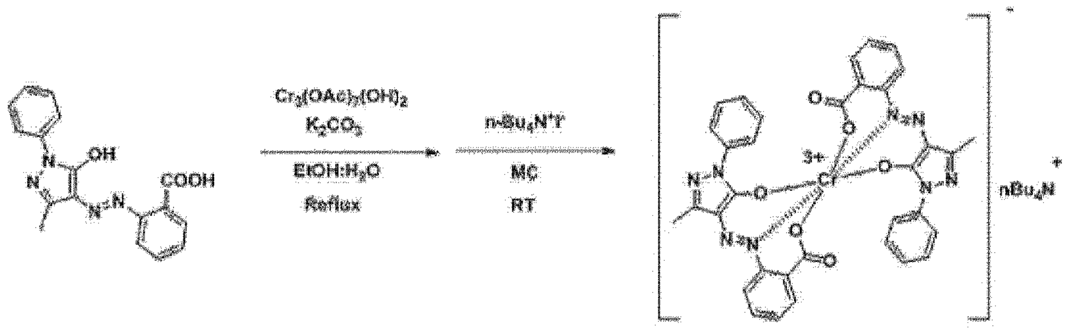


A2 P79

Comparative Compound P79 (370 mg, yield: 51%) was secured by conducting a reaction in the same manner as in Preparation Example 41 except that A2 was used instead of D1-a.

HR LC/MS/MS m/z calculated for $C_{50}H_{60}CrN_9O_6$ (M+): 934.4072; found: 934.4072

**<Experimental Example>**

**Example 1**

Based on 100 parts by weight of a solid content excluding a solvent in an adhesive composition, 95.4 parts by weight of an adhesive material (AD-701 solid content, LG Chem.), 0.29 parts by weight of Compound P1, 0.1 parts by weight of an isocyanate-based crosslinking agent (T39M, Soken Chemical & Engineering Co., Ltd.), 0.2 parts by weight of a silane-based coupling agent (T-789J, Soken Chemical & Engineering Co., Ltd.), 0.5 parts by weight of an antioxidant (Kinox-80, Hannong Chemicals Inc.), 1.5 parts by weight of an antistatic agent (FC-4400, 3M Corporation), 2 parts by weight of a hindered amine light stabilizer (Tinuvin 123, BASF Corporation) and 0.001 parts by weight a catalyst (dibutyltin dilaurate, Sigma-Aldrich) were added, and a solvent (methyl ethyl ketone, MEK) was added in 25 parts by weight based on the total parts by weight of the adhesive composition. The adhesive composition obtained by mixing these using a shaker (SKC 6100, JEIO Tech.) was coated on a release layer (PET) to a thickness of 22 μm to 23 μm using a knife bar coating apparatus (KP-3000, Kipae E&T Co., Ltd.) to prepare an adhesive film. After the coating, the release layer was removed, and the adhesive film and a binder resin film (TAC: cellulose triacetate) were consecutively laminated on glass through lamination to prepare a sample. The binder resin film (TAC: cellulose triacetate) was used to prepare the sample using the adhesive film including the adhesive composition including Compound P1, and does not affect measured properties of the adhesive film.

Maximum absorption wavelengths λmax (nm) of the adhesive composition and the adhesive film were each measured using a UV-vis apparatus (Shimazu UV-3600). In addition, in order to identify wavelength suitability as a light absorber, suitability as a light absorber was identified by subdividing the maximum absorption wavelength range, and the results are described in Table 1.

[Wavelength Suitability as Light Absorber]

Maximum absorption wavelength of 380 nm to 420 nm: o

Maximum absorption wavelength of 421 nm to 450 nm: Δ

Maximum absorption wavelength of greater than 450 nm: x

**Example 2**

An experiment was conducted under the same condition as in Example 1 except that Compound P5 was used instead of Compound P1, and the results are described in the following Table 1.

**Example 3**

An experiment was conducted under the same condition as in Example 1 except that Compound P6 was used instead of Compound P1, and the results are described in the following Table 1

**Example 4**

An experiment was conducted under the same condition as in Example 1 except that Compound P7 was used instead of Compound P1, and the results are described in the following Table 1.

**Example 5**

An experiment was conducted under the same condition as in Example 1 except that Compound P10 was used instead of Compound P1, and the results are described in the following Table 1.

**Example 6**

An experiment was conducted under the same condition as in Example 1 except that Compound P16 was used

instead of Compound P1, and the results are described in the following Table 1.

**Example 7**

An experiment was conducted under the same condition as in Example 1 except that Compound P17 was used instead of Compound P1, and the results are described in the following Table 1.

**Example 8**

An experiment was conducted under the same condition as in Example 1 except that Compound P18 was used instead of Compound P1, and the results are described in the following Table 1.

**Example 9**

An experiment was conducted under the same condition as in Example 1 except that Compound P19 was used instead of Compound P1, and the results are described in the following Table 1.

**Example 10**

An experiment was conducted under the same condition as in Example 1 except that Compound P20 was used instead of Compound P1, and the results are described in the following Table 1.

**Example 11**

An experiment was conducted under the same condition as in Example 1 except that Compound P21 was used instead of Compound P1, and the results are described in the following Table 1.

**Example 12**

An experiment was conducted under the same condition as in Example 1 except that Compound P22 was used instead of Compound P1, and the results are described in the following Table 1.

**Example 13**

An experiment was conducted under the same condition as in Example 1 except that Compound P24 was used instead of Compound P1, and the results are described in the following Table 1.

**Example 14**

An experiment was conducted under the same condition as in Example 1 except that Compound P25 was used instead of Compound P1, and the results are described in the following Table 1.

**Example 15**

An experiment was conducted under the same condition as in Example 1 except that Compound P26 was used instead of Compound P1, and the results are described in the following Table 1.

**Example 16**

An experiment was conducted under the same condition as in Example 1 except that Compound P27 was used instead of Compound P1, and the results are described in the following Table 1.

Example 17

An experiment was conducted under the same condition as in Example 1 except that Compound P28 was used instead of Compound P1, and the results are described in the following Table 1.

**Example 18**

An experiment was conducted under the same condition as in Example 1 except that Compound P29 was used instead of Compound P1, and the results are described in the following Table 1.

**Example 19**

An experiment was conducted under the same condition as in Example 1 except that Compound P30 was used instead of Compound P1, and the results are described in the following Table 1.

**Example 20**

An experiment was conducted under the same condition as in Example 1 except that Compound P31 was used instead of Compound P1, and the results are described in the following Table 1.

**Example 21**

An experiment was conducted under the same condition as in Example 1 except that Compound P32 was used instead of Compound P1, and the results are described in the following Table 1.

**Example 22**

An experiment was conducted under the same condition as in Example 1 except that Compound P33 was used instead of Compound P1, and the results are described in the following Table 1.

**Example 23**

An experiment was conducted under the same condition as in Example 1 except that Compound P34 was used instead of Compound P1, and the results are described in the following Table 1.

**Example 24**

An experiment was conducted under the same condition as in Example 1 except that Compound P35 was used instead of Compound P1, and the results are described in the following Table 1.

**Example 25**

An experiment was conducted under the same condition as in Example 1 except that Compound P36 was used instead of Compound P1, and the results are described in the following Table 1.

**Example 26**

An experiment was conducted under the same condition as in Example 1 except that Compound P37 was used instead of Compound P1, and the results are described in the following Table 1.

**Example 27**

An experiment was conducted under the same condition as in Example 1 except that Compound P38 was used instead of Compound P1, and the results are described in the following Table 1.

**Example 28**

An experiment was conducted under the same condition as in Example 1 except that Compound P41 was used instead of Compound P1, and the results are described in the following Table 1.

**Example 29**

An experiment was conducted under the same condition as in Example 1 except that Compound P43 was used

instead of Compound P1, and the results are described in the following Table 1.

**Example 30**

An experiment was conducted under the same condition as in Example 1 except that Compound P44 was used instead of Compound P1, and the results are described in the following Table 1.

**Example 31**

An experiment was conducted under the same condition as in Example 1 except that Compound P45 was used instead of Compound P1, and the results are described in the following Table 1.

**Example 32**

An experiment was conducted under the same condition as in Example 1 except that Compound P46 was used instead of Compound P1, and the results are described in the following Table 1.

**Example 33**

An experiment was conducted under the same condition as in Example 1 except that Compound P49 was used instead of Compound P1, and the results are described in the following Table 1.

**Example 34**

An experiment was conducted under the same condition as in Example 1 except that Compound P52 was used instead of Compound P1, and the results are described in the following Table 1.

**Example 35**

An experiment was conducted under the same condition as in Example 1 except that Compound P54 was used instead of Compound P1, and the results are described in the following Table 1.

**Example 36**

An experiment was conducted under the same condition as in Example 1 except that Compound P59 was used instead of Compound P1, and the results are described in the following Table 1.

**Example 37**

An experiment was conducted under the same condition as in Example 1 except that Compound P60 was used instead of Compound P1, and the results are described in the following Table 1.

**Example 38**

An experiment was conducted under the same condition as in Example 1 except that Compound P71 was used instead of Compound P1, and the results are described in the following Table 1.

**Comparative Experimental Example 1**

An experiment was conducted under the same condition as in Example 1 except that Comparative Compound P76 was used instead of Compound P1, and the results are described in the following Table 1.

**Comparative Experimental Example 2**

An experiment was conducted under the same condition as in Example 1 except that Comparative Compound P77 was used instead of Compound P1, and the results are described in the following Table 1.

**Comparative Experimental Example 3**

An experiment was conducted under the same condition as in Example 1 except that Comparative Compound P78 was used instead of Compound P1, and the results are described in the following Table 1.

**Comparative Experimental Example 4**

An experiment was conducted under the same condition as in Example 1 except that Comparative Compound P79 was used instead of Compound P1, and the results are described in the following Table 1.

[Table 1]

| Example | Preparation Example | Solution λmax (nm) | Film λmax (nm) | Wavelength Suitability |
|---|---|---|---|---|
| Example 1 | P1 | 382 | 382 | ○ |
| Example 2 | P5 | 380 | 381 | ○ |
| Example 3 | P6 | 383 | 383 | ○ |
| Example 4 | P7 | 383 | 382 | ○ |
| Example 5 | P10 | 384 | 386 | ○ |
| Example 6 | P16 | 416 | 415 | ○ |
| Example 7 | P17 | 417 | 418 | ○ |
| Example 8 | P18 | 411 | 412 | ○ |
| Example 9 | P19 | 411 | 411 | ○ |
| Example 10 | P20 | 409 | 408 | ○ |
| Example 11 | P21 | 427 | 429 | △ |
| Example 12 | P22 | 426 | 427 | △ |
| Example 13 | P24 | 408 | 409 | ○ |
| Example 14 | P25 | 429 | 428 | △ |
| Example 15 | P26 | 435 | 437 | △ |
| Example 16 | P27 | 407 | 410 | ○ |
| Example 17 | P28 | 408 | 409 | ○ |
| Example 18 | P29 | 409 | 409 | ○ |
| Example 19 | P30 | 436 | 438 | △ |
| Example 20 | P31 | 421 | 422 | △ |
| Example 21 | P32 | 418 | 418 | ○ |
| Example 22 | P33 | 418 | 419 | ○ |
| Example 23 | P34 | 406 | 405 | ○ |
| Example 24 | P35 | 404 | 404 | ○ |
| Example 25 | P36 | 409 | 409 | ○ |
| Example 26 | P37 | 415 | 416 | ○ |
| Example 27 | P38 | 413 | 412 | ○ |
| Example 28 | P41 | 446 | 446 | △ |
| Example 29 | P43 | 435 | 436 | △ |
| Example 30 | P44 | 428 | 429 | △ |

(continued)

| Example | Preparation Example | Solution λmax (nm) | Film λmax (nm) | Wavelength Suitability |
|---|---|---|---|---|
| Example 31 | P45 | 425 | 426 | △ |
| Example 32 | P46 | 433 | 432 | △ |
| Example 33 | P49 | 447 | 446 | △ |
| Example 34 | P52 | 401 | 402 | ○ |
| Example 35 | P54 | 397 | 399 | ○ |
| Example 36 | P59 | 394 | 393 | ○ |
| Example 37 | P60 | 396 | 397 | ○ |
| Example 38 | P71 | 384 | 384 | ○ |
| Comparative Experimental Example 1 | P76 | 492 | 495 | × |
| Comparative Experimental Example 2 | P77 | 473 | 471 | × |
| Comparative Experimental Example 3 | P78 | 504 | 504 | × |
| Comparative Experimental Example 4 | P79 | 471 | 472 | × |

From the experimental results of Table 1, it is identified that the adhesive films including the coordination compound according to one embodiment of the present disclosure have excellent wavelength suitability compared to the films that do not include the coordination compound. Specifically, when the adhesive films according to the examples of the present disclosure have a wavelength of at least one of 380 nm to 450 nm as a maximum absorption wavelength (λmax) unlike the adhesive films according to the comparative examples having a maximum absorption wavelength (λmax) of greater than 470 nm, a decrease in the blue luminance may be minimized by absorbing a longer wavelength region compared to an existing blue light blocking film, and efficiency may be maximized.

## Claims

1. A coordination compound, wherein, in a compound represented by $A^{n-}$, A is represented by the following Chemical Formula A, and n is 1 or 2:

[Chemical Formula A]

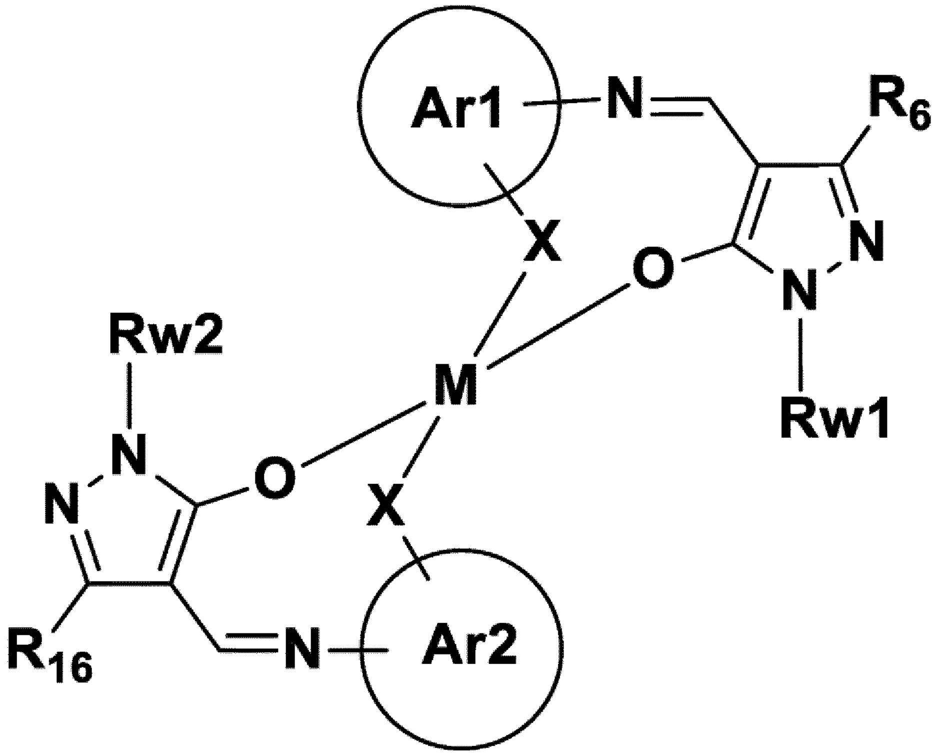

in Chemical Formula A,

M is a Cr ion or a Co ion;
X is O or O-C=O;
Rw1 and Rw2 are the same as or different from each other, and each independently a substituted or unsubstituted alkyl group; a substituted or unsubstituted alkenyl group; a substituted or unsubstituted cycloalkyl group; a substituted or unsubstituted aryl group; or a substituted or unsubstituted heteroaryl group;
Ar1 and Ar2 are the same as or different from each other, and each independently a substituted or unsubstituted arylene group; or a substituted or unsubstituted heteroarylene group;
$R_6$ and $R_{16}$ are the same as or different from each other, and each independently hydrogen; deuterium; a nitrile group; a nitro group; a hydroxyl group; -COOH; a halogen group; an imide group; a substituted or unsubstituted alkyl group; a substituted or unsubstituted cycloalkyl group; a substituted or unsubstituted heterocyclic group; a substituted or unsubstituted aryl group; a substituted or unsubstituted heteroaryl group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted arylphosphine group; a substituted or unsubstituted phosphine oxide group; a substituted or unsubstituted styryl group; $-OR_{100}$; $-CO_2R_{101}$; $-COR_{102}$; $-OCOR_{103}$; - $CONR_{104}R_{105}$; or $-SO_2R_{106}$; and
$R_{100}$ to $R_{106}$ are the same as or different from each other, and each independently hydrogen; a substituted or unsubstituted alkyl group; a substituted or unsubstituted cycloalkyl group; a substituted or unsubstituted aryl group; a substituted or unsubstituted heteroaryl group; or a substituted or unsubstituted amine group.

**2.** The coordination compound of Claim 1, wherein Chemical Formula A is represented by the following Chemical Formula A-1:

[Chemical Formula A-1]

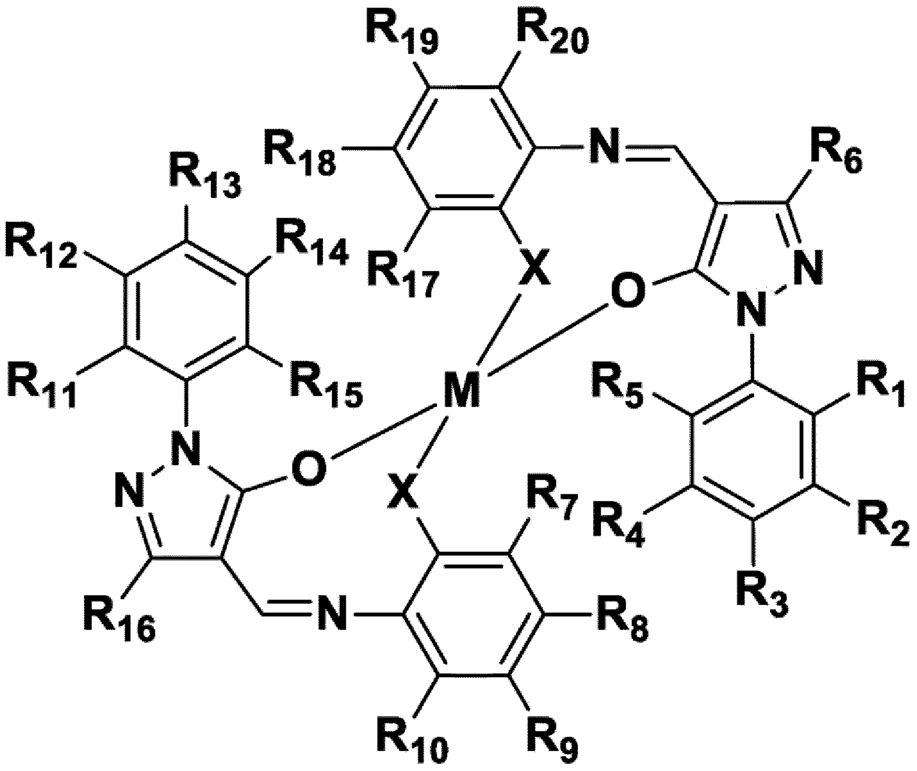

in Chemical Formula A-1,

M, X, $R_6$ and $R_{16}$ have the same definitions as in Chemical Formula A;
$R_1$ to $R_5$ $R_7$ to $R_{15}$ and $R_{17}$ to $R_{20}$ are the same as or different from each other, and each independently hydrogen; deuterium; a nitrile group; a nitro group; a hydroxyl group; - COOH; a halogen group; an imide group; a substituted or unsubstituted alkyl group; a substituted or unsubstituted cycloalkyl group; a substituted or unsubstituted heterocyclic group; a substituted or unsubstituted aryl group; a substituted or unsubstituted heteroaryl group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted arylphosphine group; a substituted or unsubstituted phosphine oxide group; $-OR_{100}$; - $CO_2R_{101}$; $-COR_{102}$; $-OCOR_{103}$; $-CONR_{104}R_{105}$; or $-SO_2R_{106}$, or adjacent substituents bond to each other to form a substituted or unsubstituted aromatic ring or a substituted or unsubstituted aliphatic ring; and
$R_{100}$ to $R_{106}$ are the same as or different from each other, and each independently hydrogen; a substituted or unsubstituted alkyl group; a substituted or unsubstituted cycloalkyl group; a substituted or unsubstituted aryl group; a substituted or unsubstituted heteroaryl group; or a substituted or unsubstituted amine group.

**3.** The coordination compound of Claim 2, wherein $R_1$ to $R_5$ and $R_{11}$ to $R_{15}$ are the same as or different from each other, and each independently hydrogen; deuterium; a halogen group; a nitrile group; a nitro group; -COOH; a substituted or unsubstituted alkyl group having 1 to 10 carbon atoms; a substituted or unsubstituted cycloalkyl group having 3 to 10 carbon atoms; an aryl group having 6 to 10 carbon atoms unsubstituted or substituted with an alkyl group; a substituted or unsubstituted heteroaryl group having 2 to 10 carbon atoms; $-OR_{100}$; or $-CO_2R_{101}$; and $R_{100}$ and $R_{101}$ are the same as or different from each other, and each independently a substituted or unsubstituted alkyl group having 1 to 10 carbon atoms.

**4.** The coordination compound of Claim 2, wherein $R_7$ to $R_{10}$ and $R_{17}$ to $R_{20}$ are the same as or different from each other, and each independently hydrogen; deuterium; a halogen group; a nitrile group; a nitro group; a substituted or unsubstituted alkyl group having 1 to 10 carbon atoms; a substituted or unsubstituted cycloalkyl group having 3 to 10 carbon atoms; an aryl group having 6 to 10 carbon atoms unsubstituted or substituted with $-OR_{100}$ or an alkyl group; a substituted or unsubstituted heteroaryl group having 2 to 10 carbon atoms; - $OR_{100}$; $-CO_2R_{101}$; $-COR_{102}$;

or $-SO_2R_{106}$, or adjacent substituents bond to each other to form a substituted or unsubstituted aromatic ring;

$R_{100}$, $R_{101}$ and $R_{102}$ are the same as or different from each other, and each independently a substituted or unsubstituted alkyl group having 1 to 10 carbon atoms; and
$R_{106}$ is a substituted or unsubstituted alkyl group having 1 to 10 carbon atoms; or $-NH_2$.

**5.** The coordination compound of Claim 1, wherein $R_6$ and $R_{16}$ are the same as or different from each other, and each independently a nitrile group; a methyl group unsubstituted or substituted with a fluoro group; a substituted or unsubstituted ethyl group; a substituted or unsubstituted isopropyl group; a phenyl group unsubstituted or substituted with one or more substituents selected from the group consisting of a halogen group, a nitrile group, a nitro group, -COOH, an alkyl group, $-OR_{100}$ and $-CO_2R_{101}$; a substituted or unsubstituted benzoxazole group; a substituted or unsubstituted pyridine group; a styryl group unsubstituted or substituted with an alkyl group or a nitro group; $-OR_{100}$; or $-CO_2R_{101}$; and
$R_{101}$ and $R_{102}$ are the same as or different from each other, and each independently a substituted or unsubstituted methyl group; or a substituted or unsubstituted ethyl group.

**6.** The coordination compound of Claim 1, wherein M is $Co^{2+}$; $Co^{3+}$; $Cr^{2+}$; or $Cr^{3+}$.

**7.** The coordination compound of Claim 1, wherein Chemical Formula A is any one selected from the group consisting of the following materials:

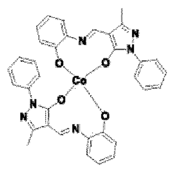
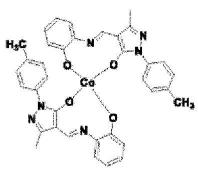
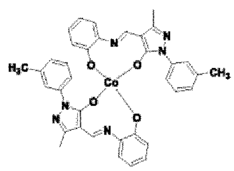
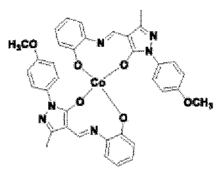
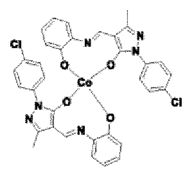

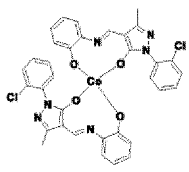
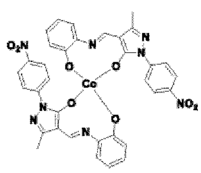
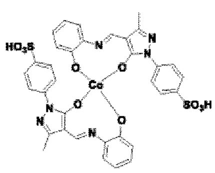
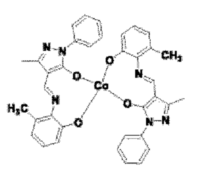
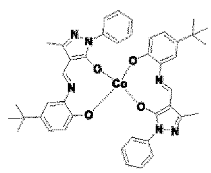

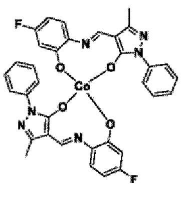
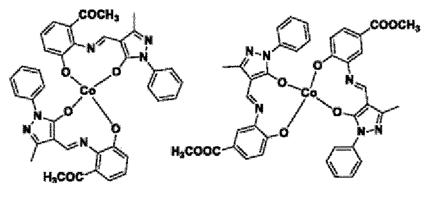
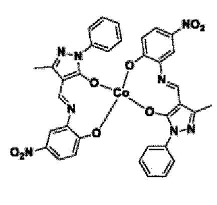
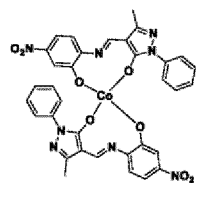

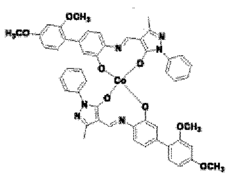
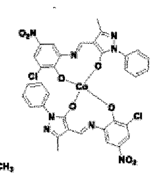
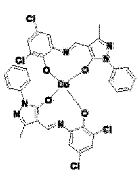
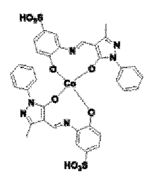
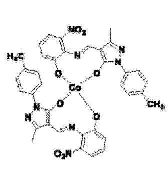
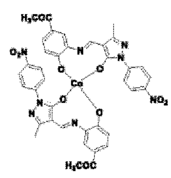

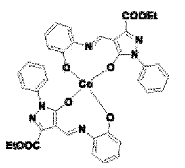
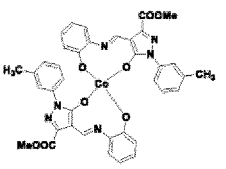
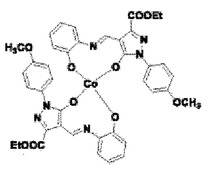
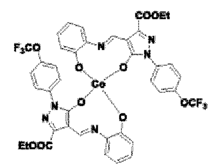
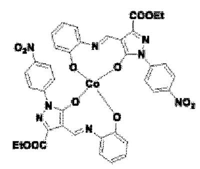

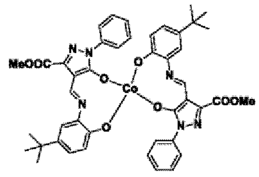
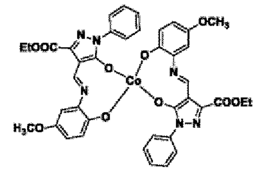
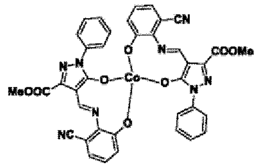
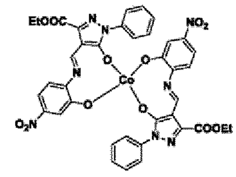

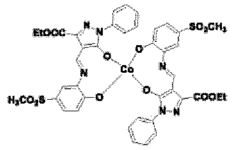
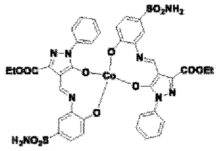
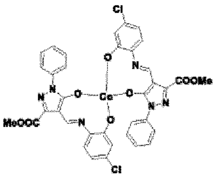
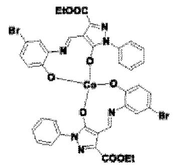
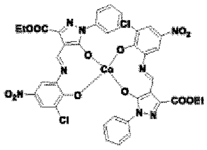

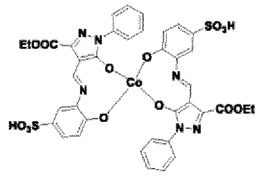
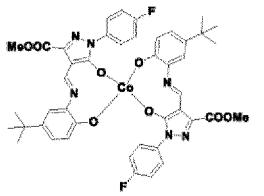
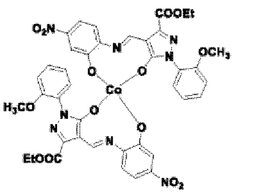
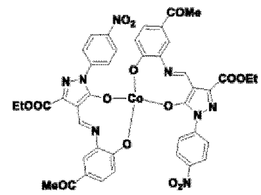

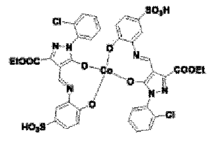
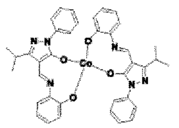
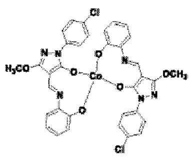
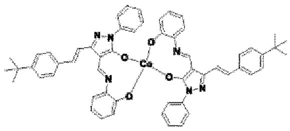
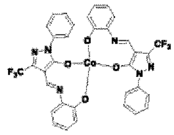

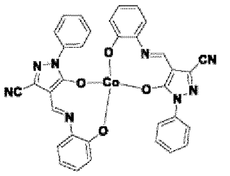
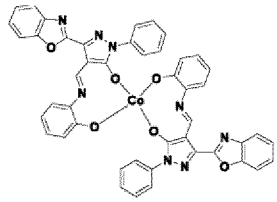
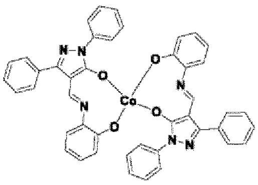
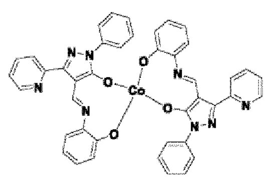

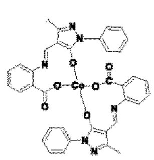
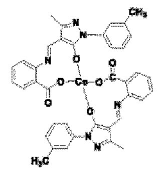
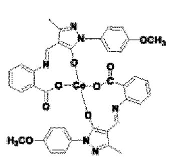
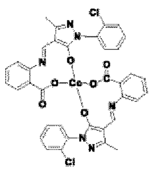
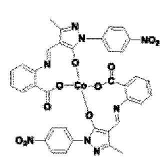
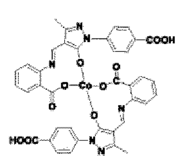

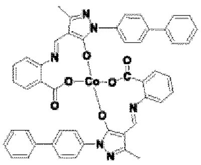
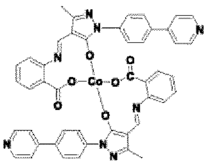
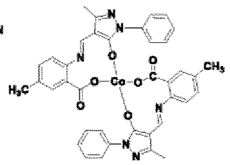
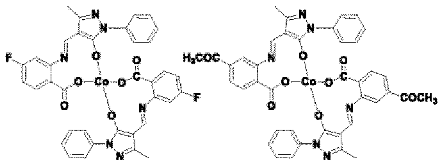

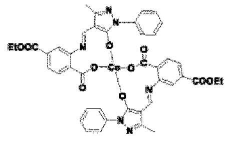
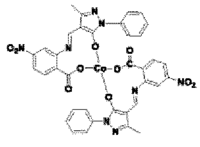
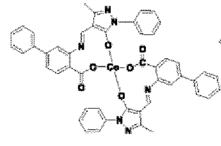
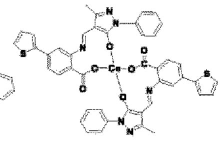
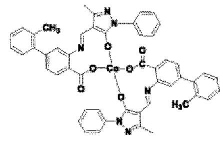

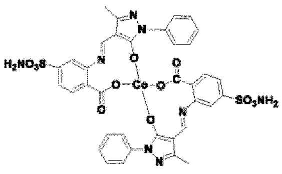
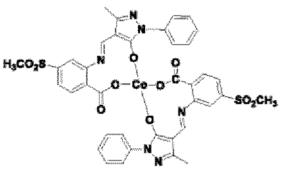
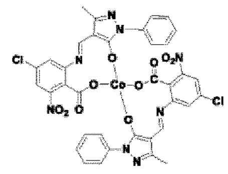
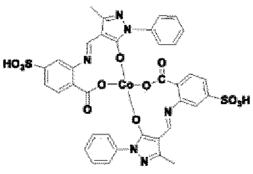

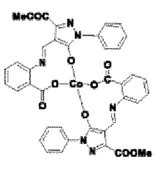
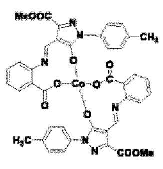
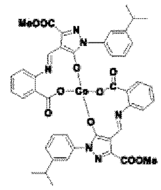
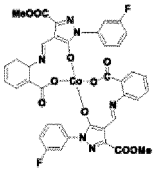
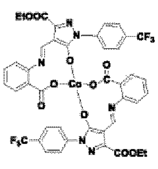
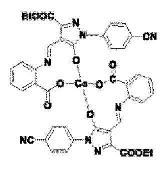

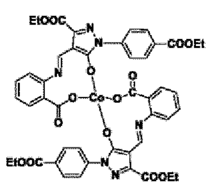
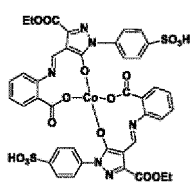
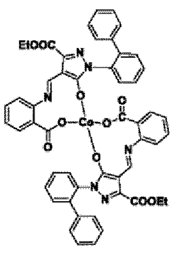
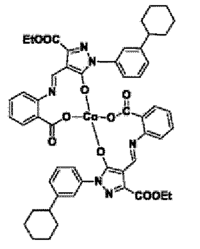
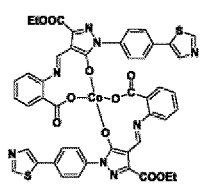

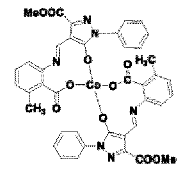
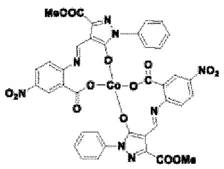
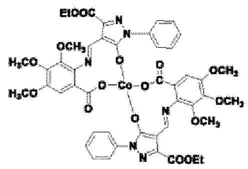
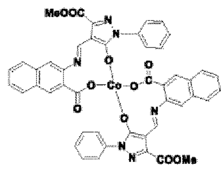
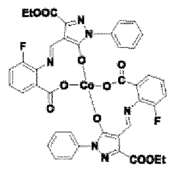

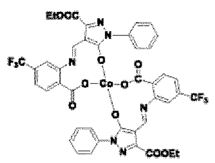
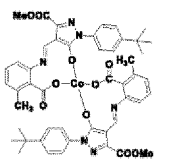
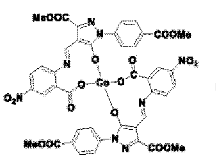
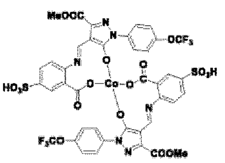
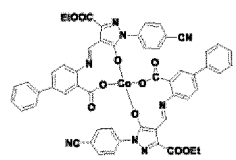

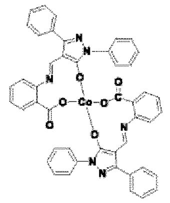
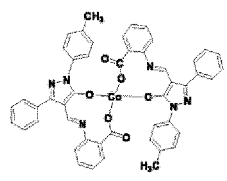
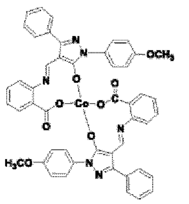
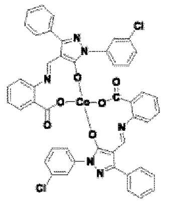
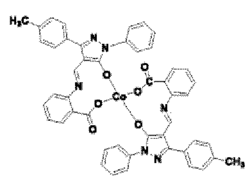

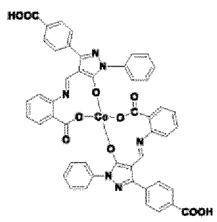
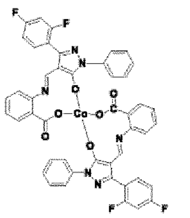
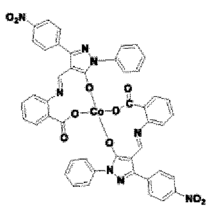
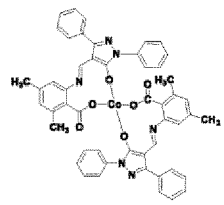
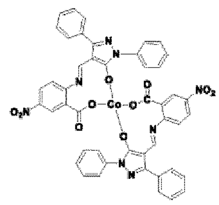

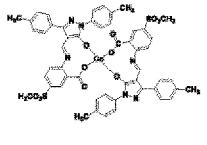
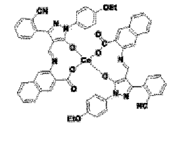
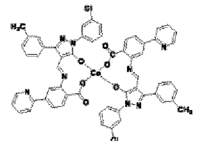
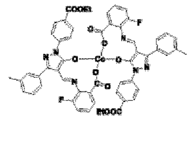
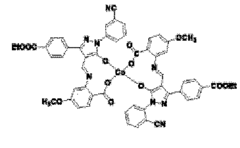

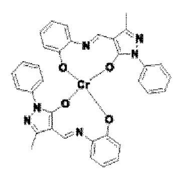 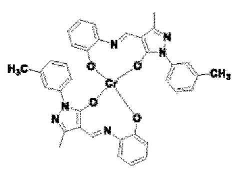 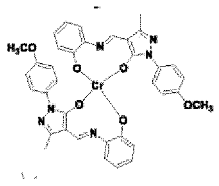 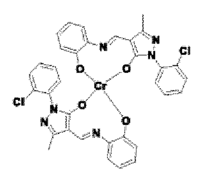 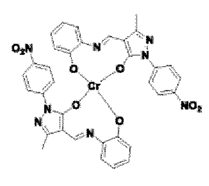

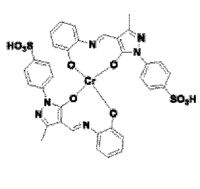 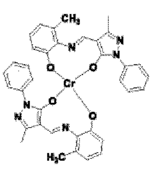 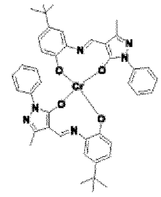 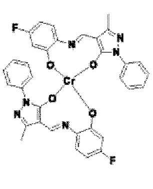 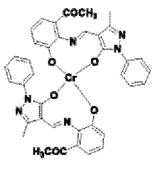 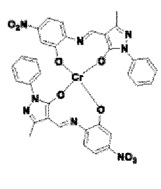

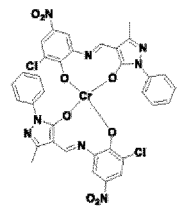 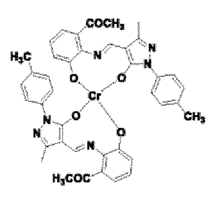 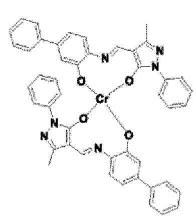 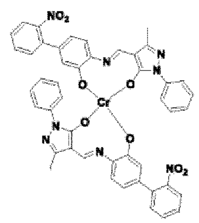 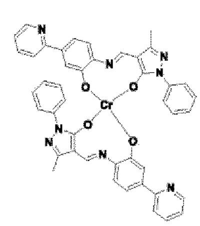

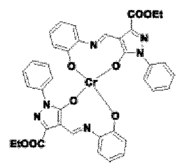 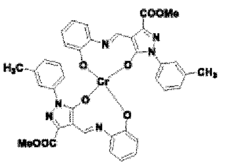 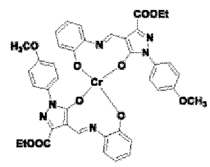 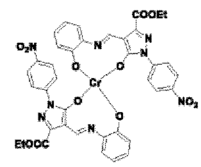 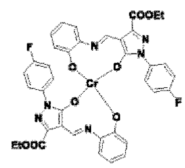

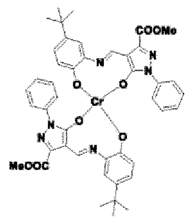 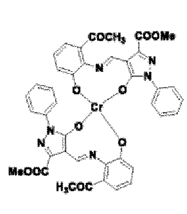 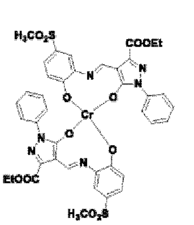 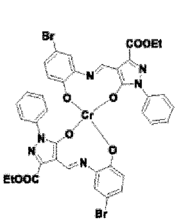 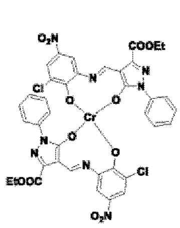

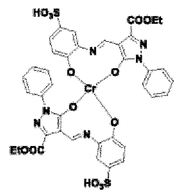 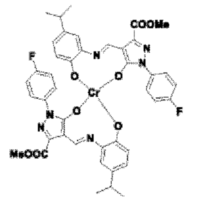 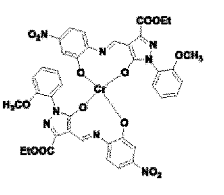 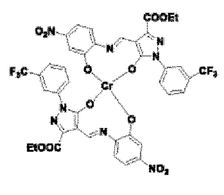 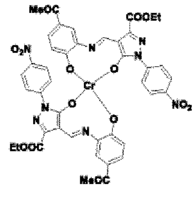

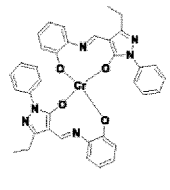 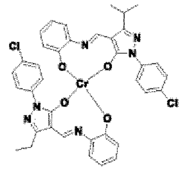 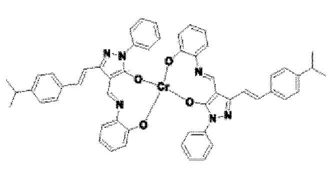 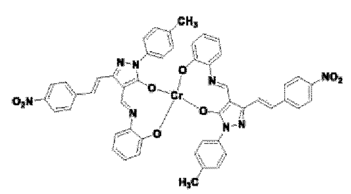

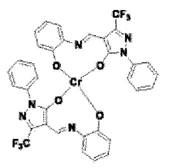
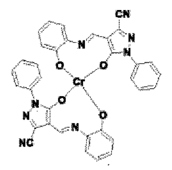
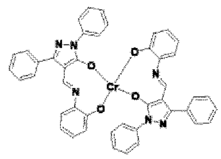
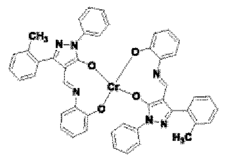
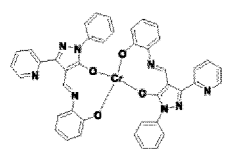

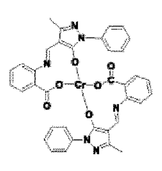
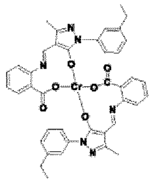
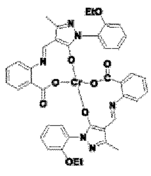
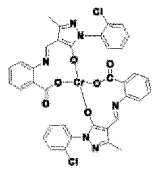
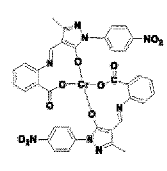
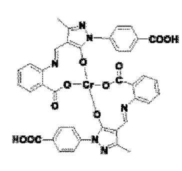

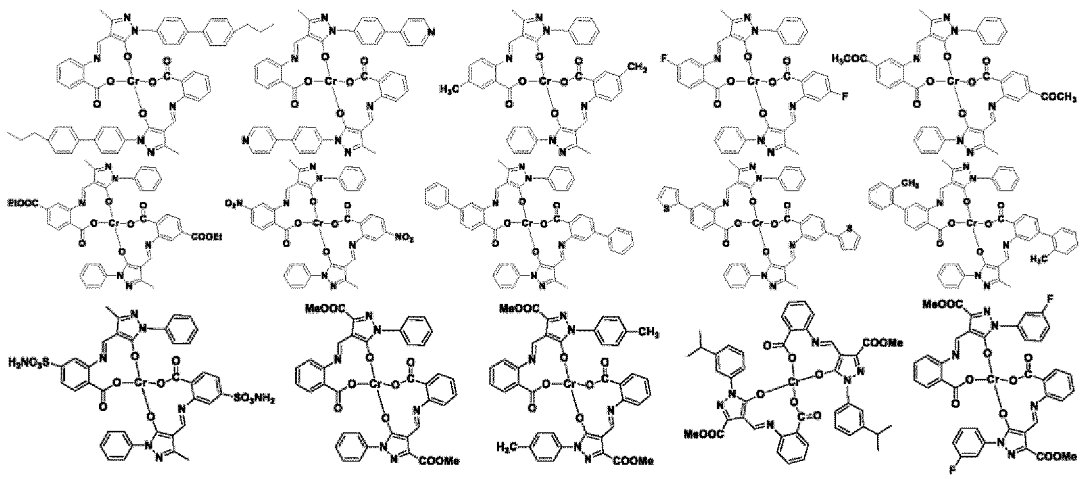

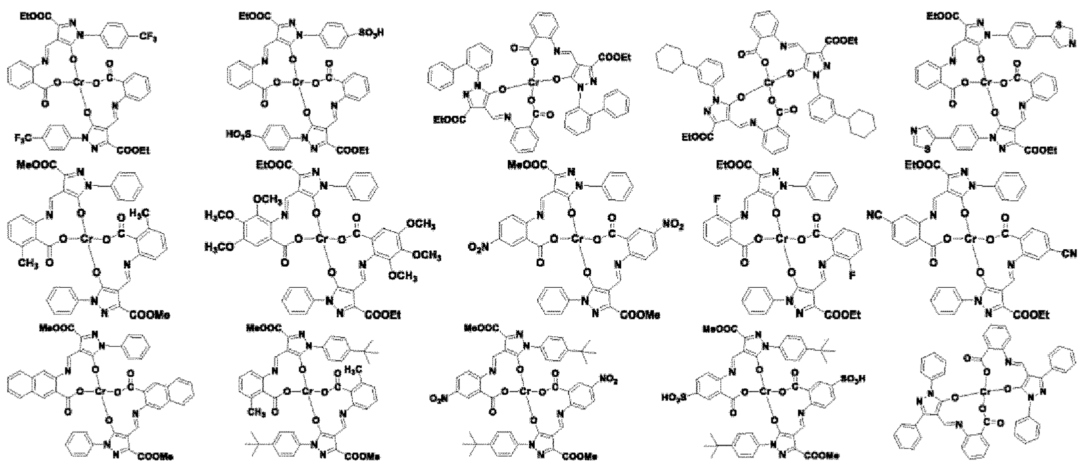

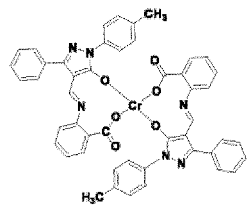
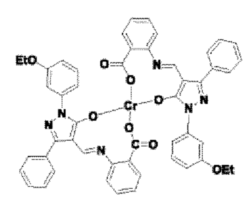
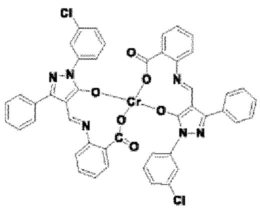
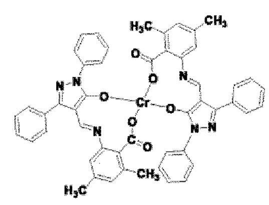

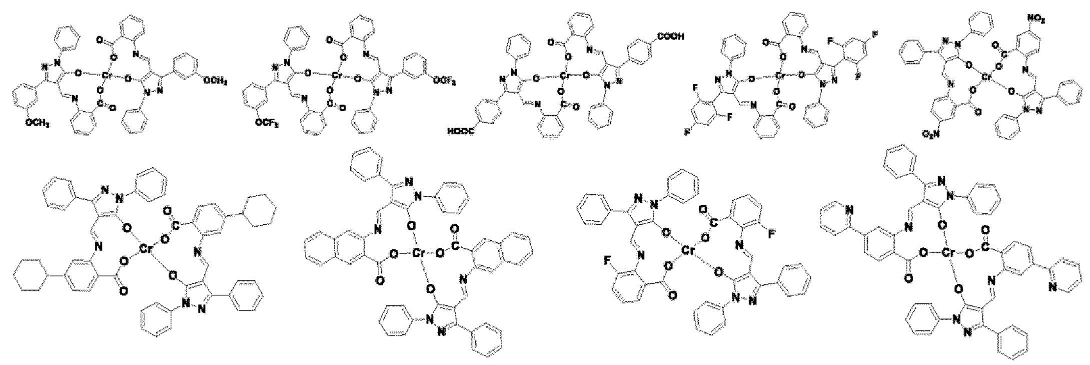

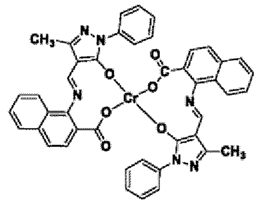

in the materials,
Me means a methyl group, and Et means an ethyl group.

**8.** The coordination compound of Claim 1, further comprising $(B^+)m$, wherein $B^+$ is $H^+$; $Na^+$; $K^+$; a phosphonium cation; an ammonium cation; a sulfonium cation; or a cationic heteroring, and m is an integer of 1 or 2.

**9.** The coordination compound of Claim 8, wherein $B^+$ is $H^+$; $Na^+$; $K^+$; $P^+ReRfRgRh$; $N^+RiRjRkRl$; $S^+RmRnRo$; or a cationic heteroring; and
Re to Ro are the same as or different from each other, and each independently hydrogen; a substituted or unsubstituted alkyl group; or a substituted or unsubstituted cycloalkyl group.

**10.** The coordination compound of Claim 8, wherein the cationic heteroring is any one selected from the group consisting of the following Structural Formulae B1 to B4:

[Structural Formula B1]

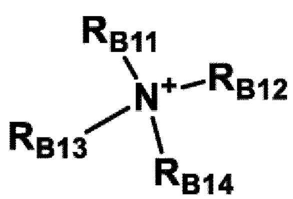

[Structural Formula B2]

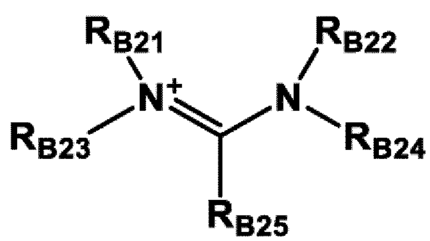

[Structural Formula B3]

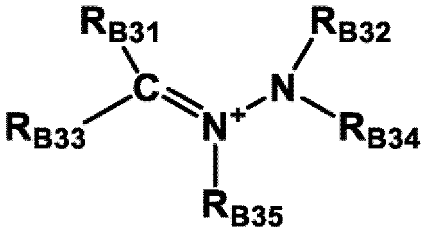

[Structural Formula B4]

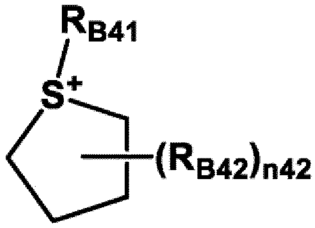

in Structural Formulae B1 to B4,

$R_{B11}$ and $R_{B12}$ bond to each other to form a substituted or unsubstituted heteroring having 4 to 30 carbon atoms, and $R_{B13}$ and $R_{B14}$ are the same as or different from each other and each independently hydrogen; a hydrocarbon group having 1 to 30 carbon atoms; or a substituent including one, two or more heteroatoms selected from the group consisting of N, O, P, S, Si and Se;
$R_{B21}$ and $R_{B22}$ bond to each other to form a substituted or unsubstituted heteroring having 2 to 30 carbon atoms, and $R_{B23}$ to $R_{B25}$ are the same as or different from each other and each independently hydrogen; a hydrocarbon group having 1 to 30 carbon atoms; or a substituent including one, two or more heteroatoms selected from the group consisting of N, O, P, S, Si and Se;
$R_{B31}$ and $R_{B32}$ bond to each other to form a substituted or unsubstituted heteroring having 2 to 30 carbon atoms, and $R_{B33}$ to $R_{B35}$ are the same as or different from each other and each independently hydrogen; a hydrocarbon group having 1 to 30 carbon atoms; or a substituent including one, two or more heteroatoms selected from the group consisting of N, O, P, S, Si and Se; and
$R_{B41}$ is hydrogen; a hydrocarbon group having 1 to 30 carbon atoms; or a substituent including one, two or more heteroatoms selected from the group consisting of N, O, P, S, Si and Se, $R_{B42}$ is hydrogen; a hydrocarbon group having 1 to 30 carbon atoms; or a substituent including one, two or more heteroatoms selected from the group consisting of N, O, P, S, Si and Se, and n42 is an integer of 1 to 8.

**11.** The coordination compound of Claim 8, wherein the compound further including $(B^+)m$ is any one selected from the group consisting of the following compounds:

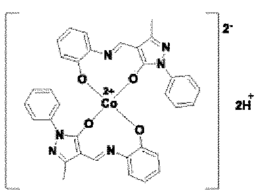 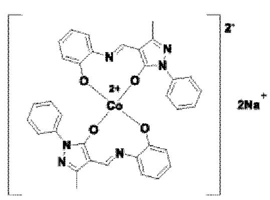 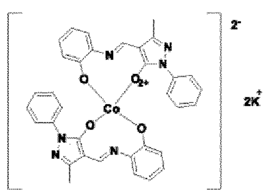 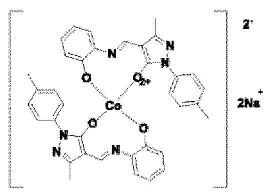

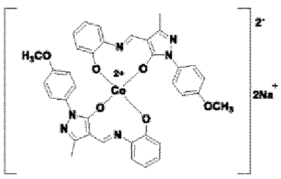
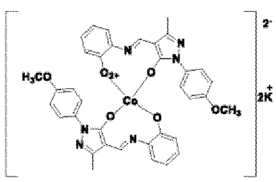
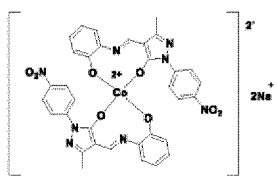
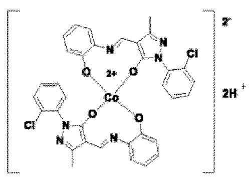

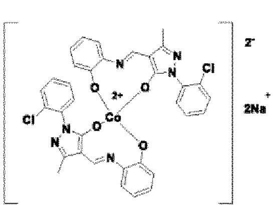
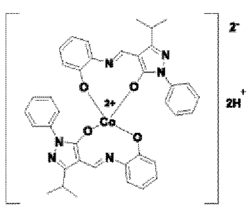
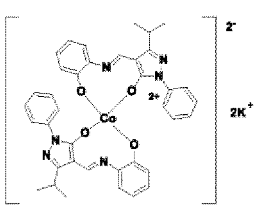
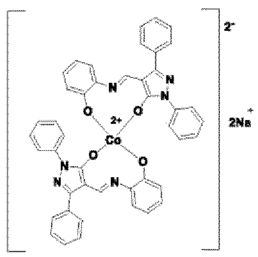

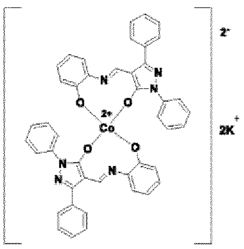
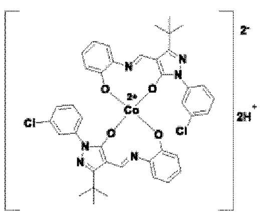
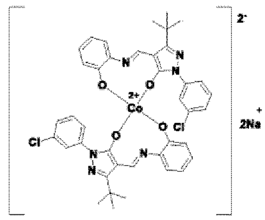
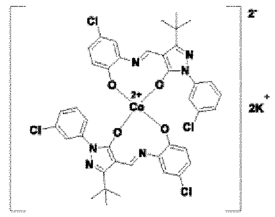

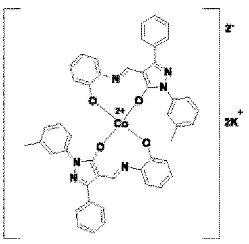
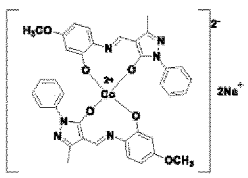
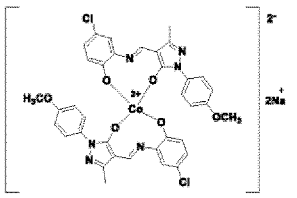
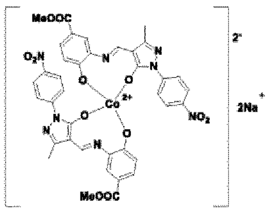

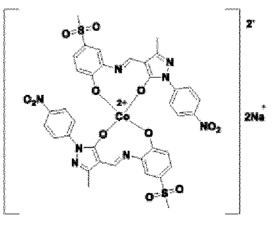
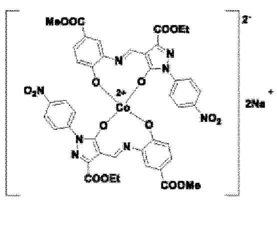
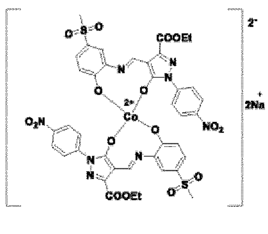
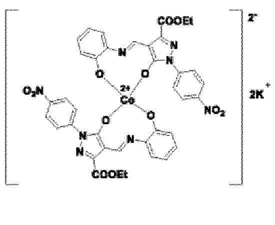

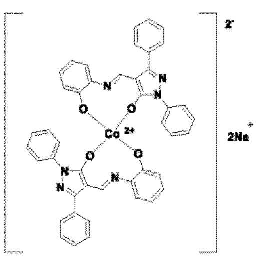
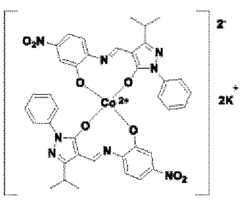
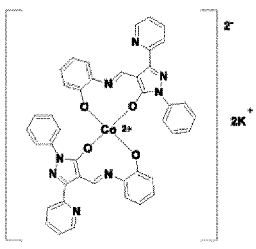
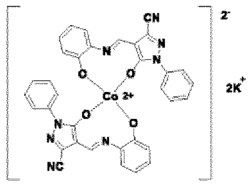

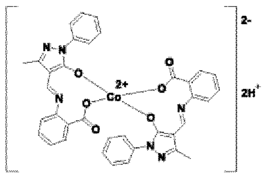

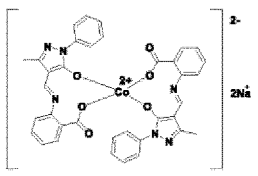

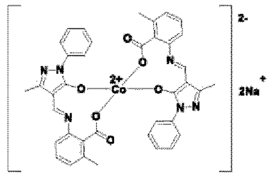

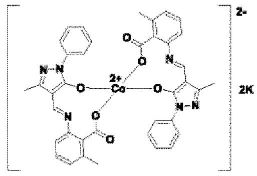

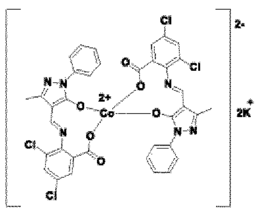

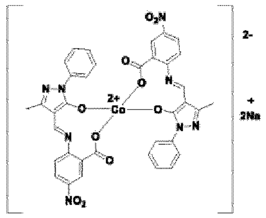

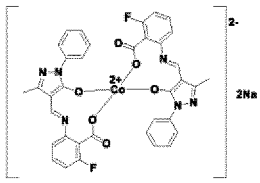

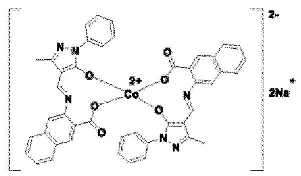

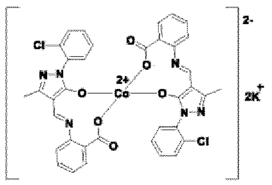

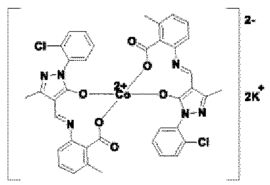

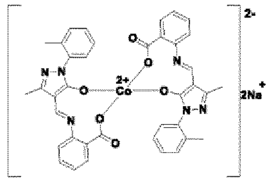

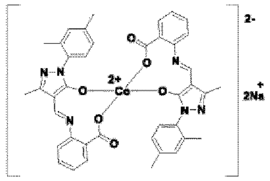

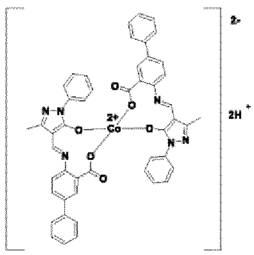

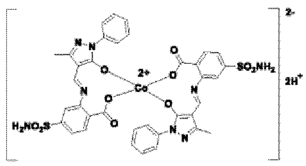

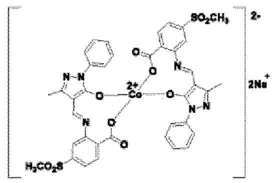

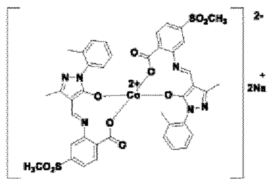

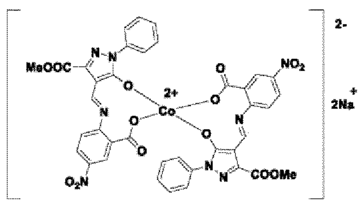

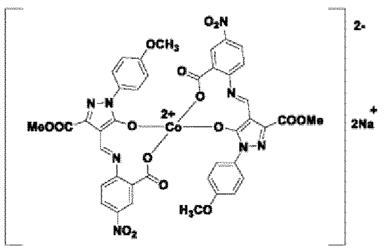

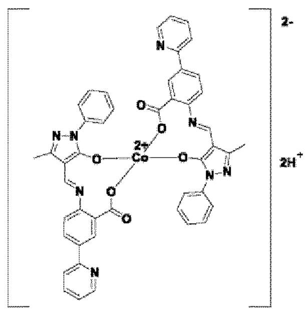

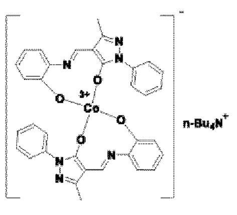

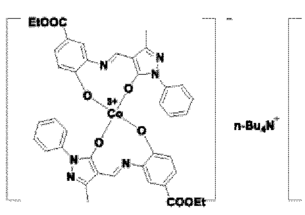

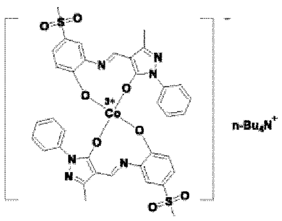

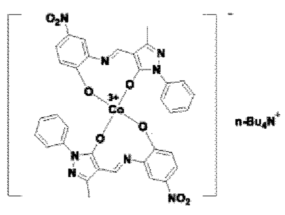

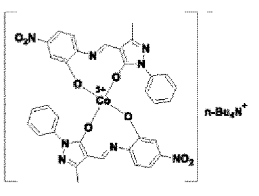
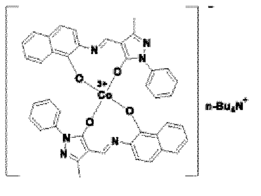
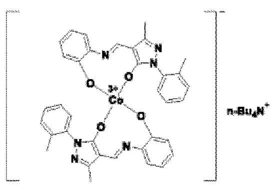
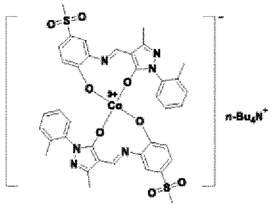

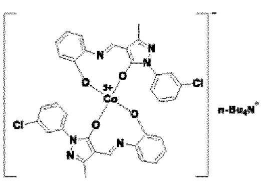
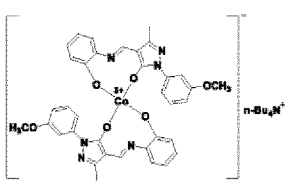
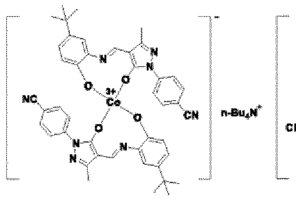
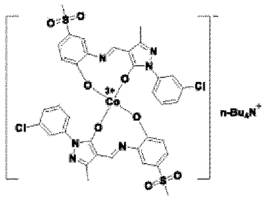

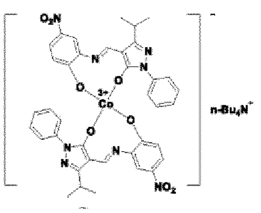
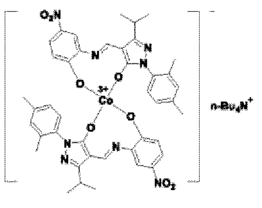
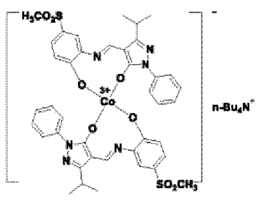
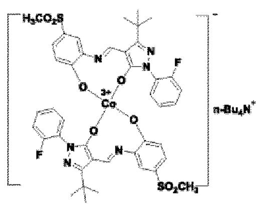

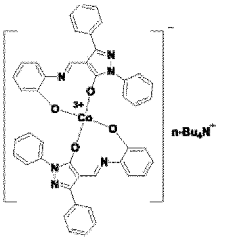
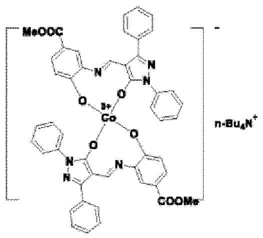
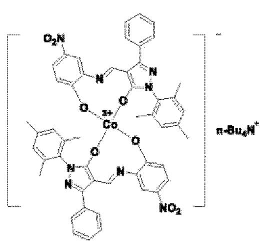
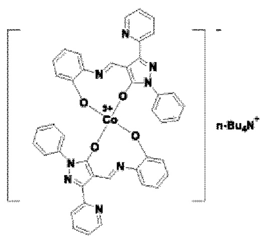

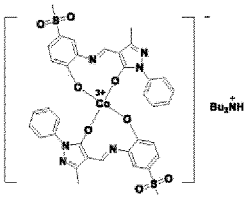
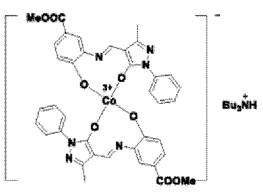
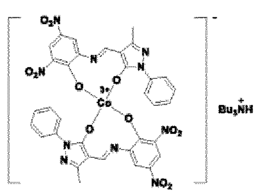
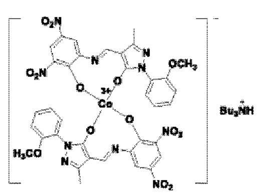

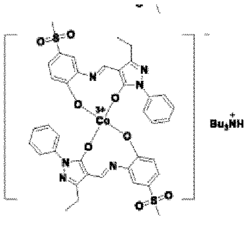
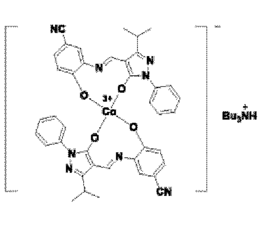
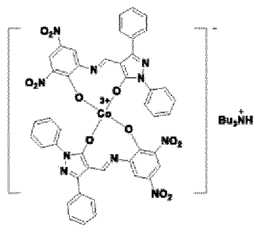
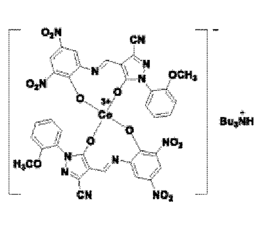

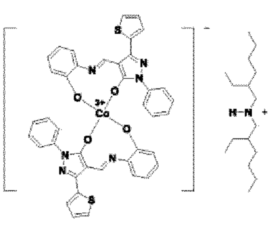
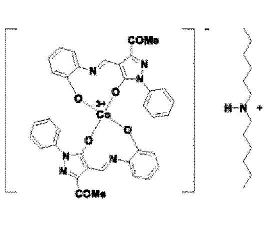
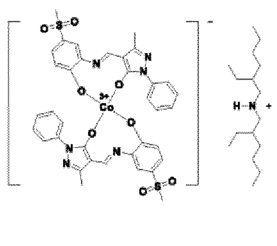
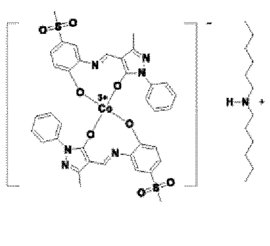

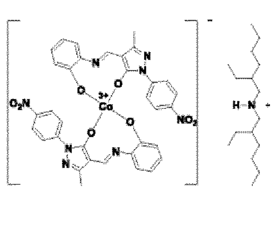
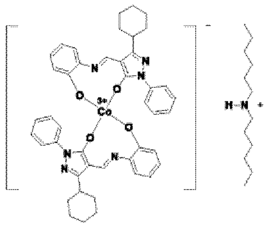
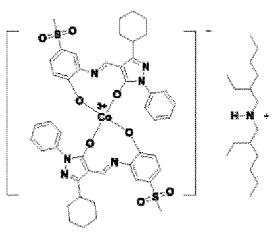
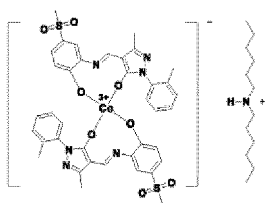
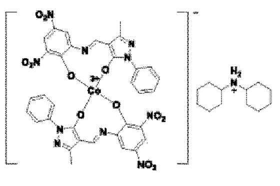
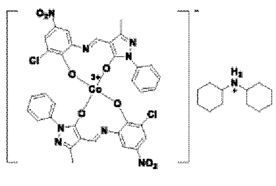
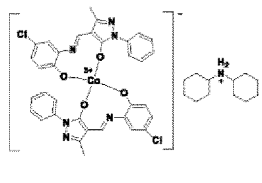
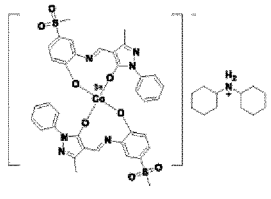
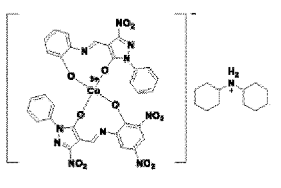
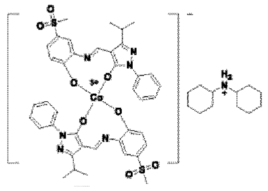
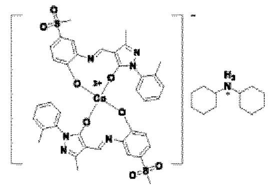
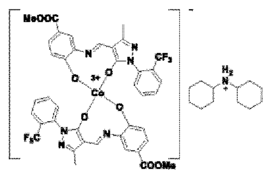
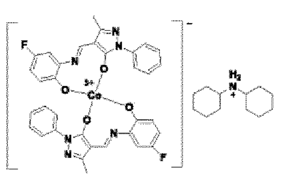
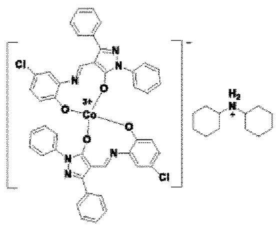
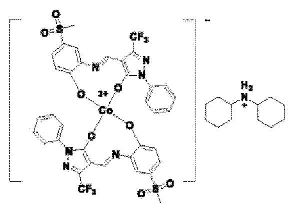
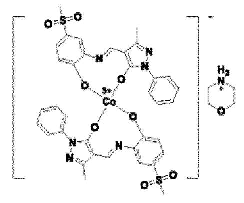
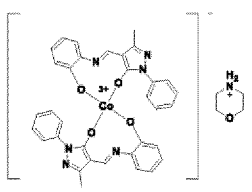
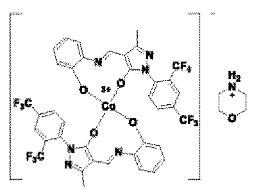
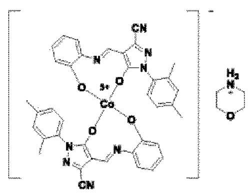
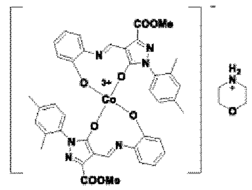
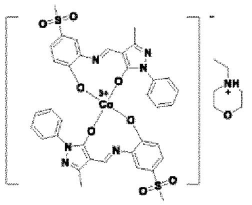
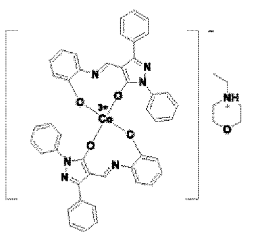
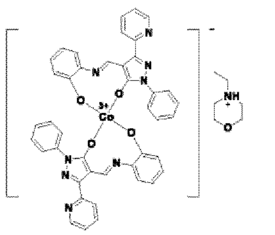
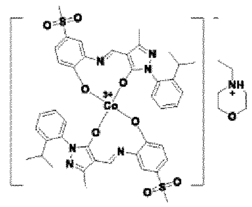

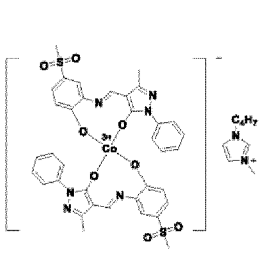
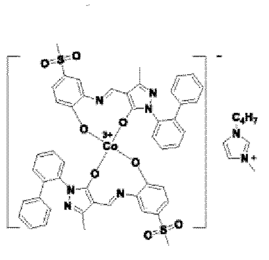
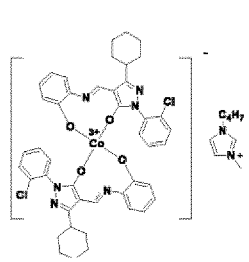
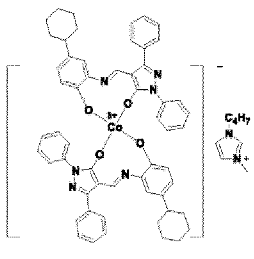

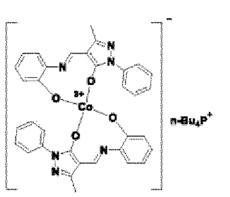
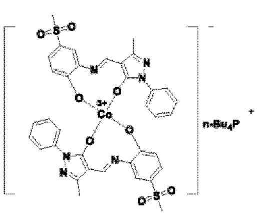
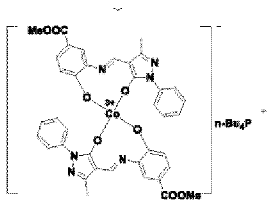
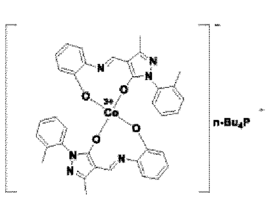

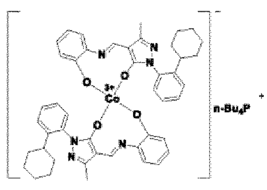
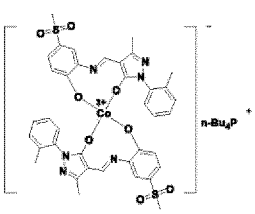
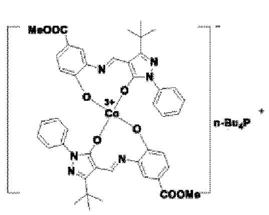
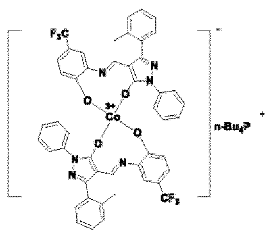

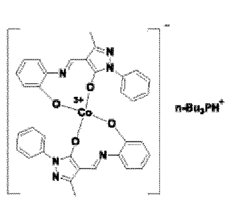
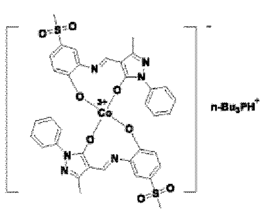
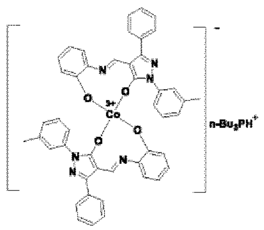
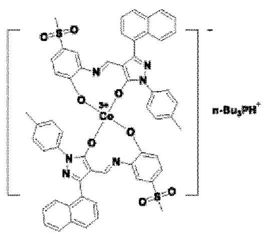

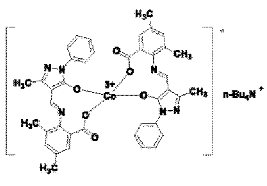
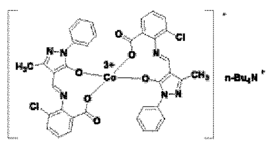
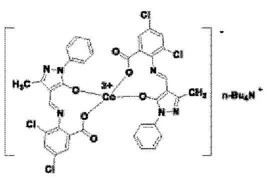
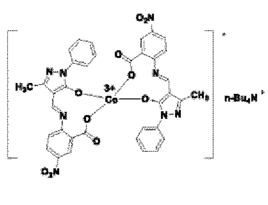

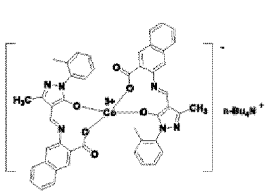
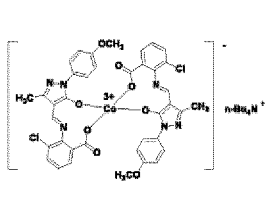
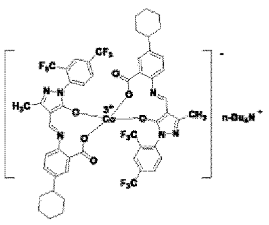
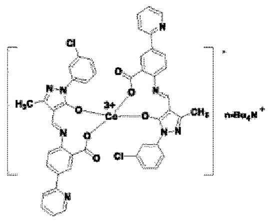

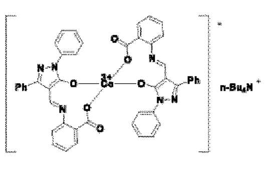
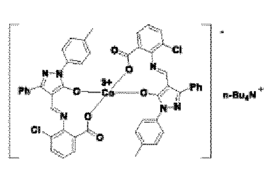
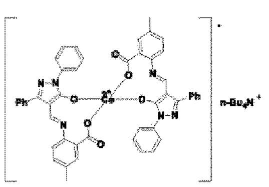
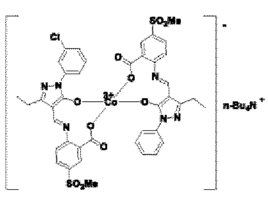

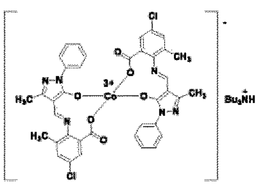
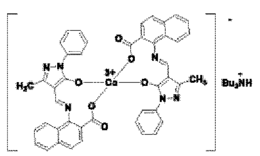
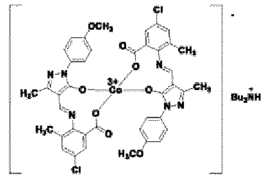
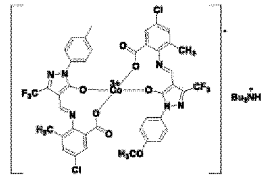

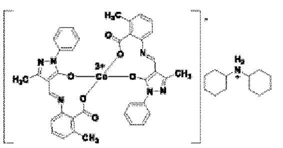
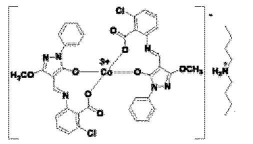
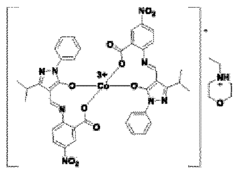
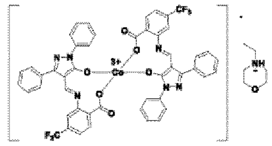

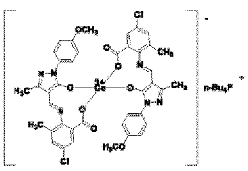
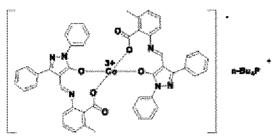
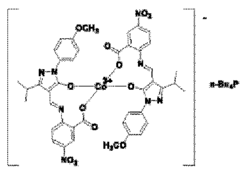
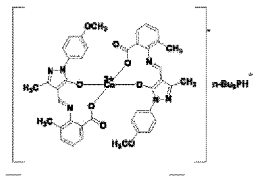

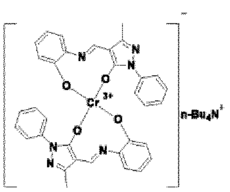
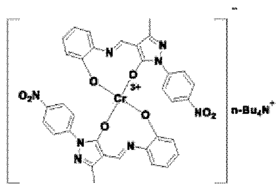
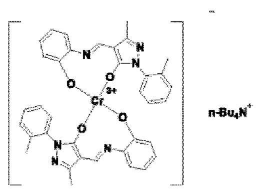
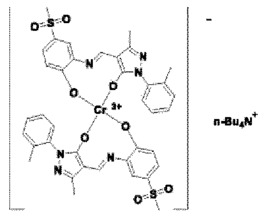

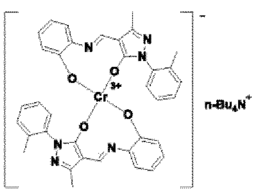
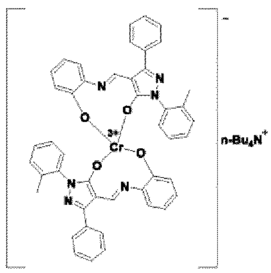
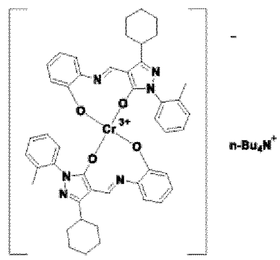
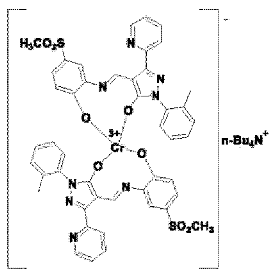

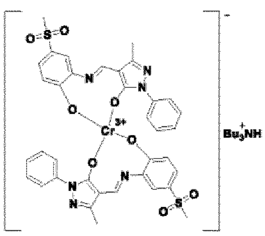
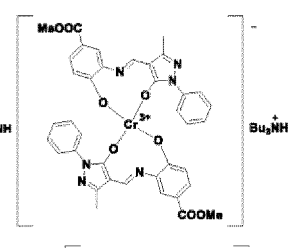
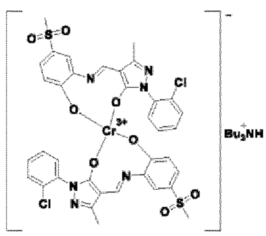
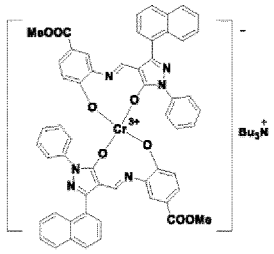

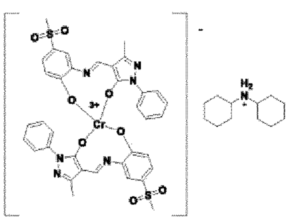
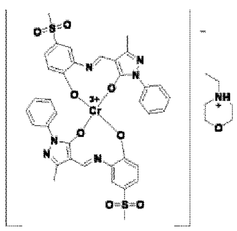
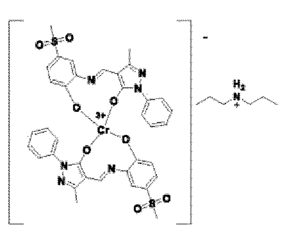
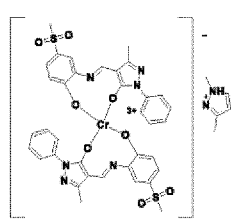

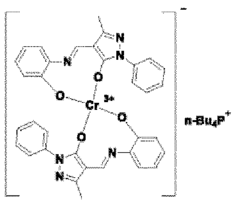
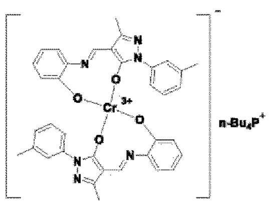
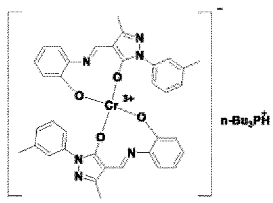
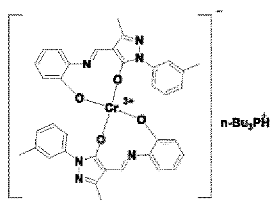

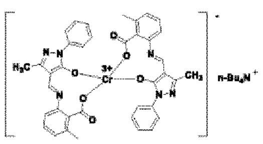
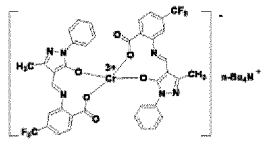
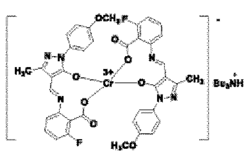
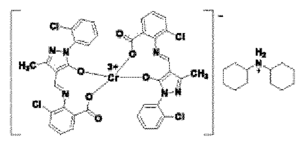

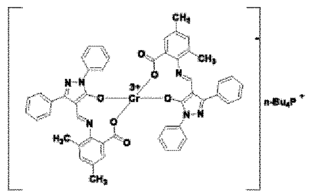
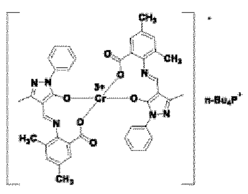
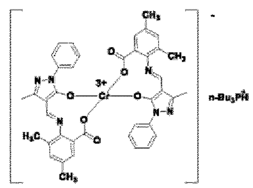
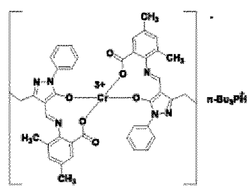

.

**12.** A light absorber comprising the coordination compound of any one of Claims 1 to 11.

**13.** The light absorber of Claim 12, which absorbs at least a portion of wavelengths selected from among 350 nm to 450 nm.

**14.** An adhesive film comprising the light absorber of Claim 12.

**15.** An optical film comprising the light absorber of Claim 12.

**16.** The optical film Claim 15, comprising:

an adhesive film; and
a binder resin film,
wherein the adhesive film includes the light absorber.

**17.** An electronic device comprising the light absorber of Claim 12.

**18.** The electronic device of Claim 17, comprising an adhesive film,
wherein the adhesive film includes the light absorber.

【FIG. 1】

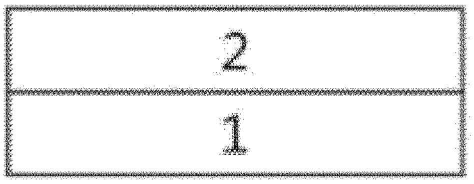


【FIG. 2】

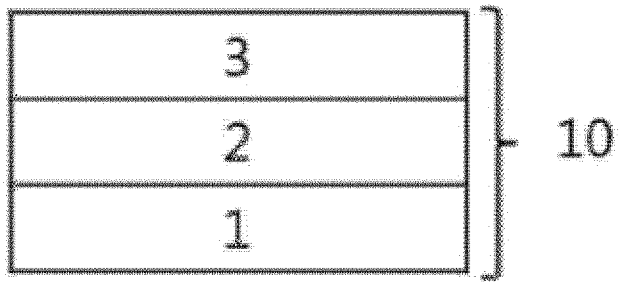

## INTERNATIONAL SEARCH REPORT

International application No.
**PCT/KR2021/014871**

### A. CLASSIFICATION OF SUBJECT MATTER

**C07F 15/06**(2006.01)i; **C09K 3/00**(2006.01)i

According to International Patent Classification (IPC) or to both national classification and IPC

### B. FIELDS SEARCHED

Minimum documentation searched (classification system followed by classification symbols)

C07F 15/06(2006.01); C07D 251/24(2006.01); C07D 403/12(2006.01); C09J 7/00(2006.01); C09J 7/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 광흡수제(light absorber), 디아졸(diazole), 크롬(chormium), 코발트(cobalt), 점착필름(adhesive film), 광학필름(optic film)

### C. DOCUMENTS CONSIDERED TO BE RELEVANT

| Category* | Citation of document, with indication, where appropriate, of the relevant passages | Relevant to claim No. |
|---|---|---|
| A | ALEXOPOULOU, K. I. et al. Mononuclear anionic octahedral cobalt (III) complexes based on N-salicylidene-o-aminophenol and its derivatives: Synthetic, structural and spectroscopic studies. Spectrochimica Acta Part A: Molecular and Biomolecular Spectroscopy. 2015, vol. 136, pp. 122-130. See abstract; and pages 123 and 124. | 1-18 |
| A | JINZHOU, L. et al. Synthesis, Characterization and Polarographic Behaviour of Complexes of Thenoylpyrazolone-β-alanine. Chinese Journal of Chemical Physics. 2004, vol. 17, no. 5, pp. 592-596. See abstract; table 3; and figure 1. | 1-18 |
| A | Chemical Abstract compound. STNext RN 757965-32-5 (Entered STN: 07 October 2004). | 1-18 |
| A | WO 2020-118933 A1 (EUTEC NEW MATERIALS TECHNOLOGY (SUZHOU) CO., LTD.) 18 June 2020 (2020-06-18) See claims 1-12. | 1-18 |
| DA | JP 2012-211305 A (MITSUBISHI PLASTICS INC.) 01 November 2012 (2012-11-01) See claims 1-8. | 1-18 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 February 2022** | **08 February 2022** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2021/014871**

| Patent document cited in search report | Publication date (day/month/year) | Patent family member(s) | Publication date (day/month/year) |
|---|---|---|---|
| WO 2020-118933 A1 | 18 June 2020 | CA 3124463 A1 | 18 June 2020 |
| | | CN 111285853 A | 16 June 2020 |
| | | EP 3896067 A1 | 20 October 2021 |
| | | KR 10-2021-0110829 A | 09 September 2021 |
| | | TW 202030308 A | 16 August 2020 |
| JP 2012-211305 A | 01 November 2012 | CN 202595022 U | 12 December 2012 |
| | | JP 5952013 B2 | 13 July 2016 |
| | | KR 10-2012-0006849 U | 05 October 2012 |
| | | KR 20-0484125 Y1 | 02 August 2017 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020200137608
- JP 2012211305 A